# EUROPEAN PATENT SPECIFICATION

(11) **EP 2 522 724 B1**
(45) Date of publication and mention of the grant of the patent: **05.02.2020**
(21) Application number: 10839561.7
(22) Date of filing: 24.12.2010
(51) Int. Cl.: C12N 15/00, A61K 39/395, C07K 1/22, C07K 16/46, C12N 1/15, C12N 1/19, C12N 1/21, C12N 5/10, C12N 15/09, C07K 19/00, C07K 16/28, C07K 16/30, C07K 16/36, C07K 16/00, C07K 14/735

(54) **POLYPEPTIDE MODIFICATION METHOD FOR PURIFYING POLYPEPTIDE MULTIMERS**
POLYPEPTIDMODIFIKATIONSVERFAHREN ZUR AUFREINIGUNG VON POLYPEPTIDMULTIMEREN
PROCÉDÉ DE MODIFICATION DE POLYPEPTIDE POUR PURIFIER UN MULTIMÈRE DE POLYPEPTIDE

(30) Priority: 25.12.2009 JP 2009294391
(43) Date of publication of application: 14.11.2012
(73) Proprietor: Chugai Seiyaku Kabushiki Kaisha, Kita-ku Tokyo 115-8543 (JP)
(72) Inventor: IGAWA, Tomoyuki, Gotemba-shi Shizuoka 412-8513 (JP); SAMPEI, Zenjiro, Gotemba-shi Shizuoka 412-8513 (JP); WAKABAYASHI, Tetsuya, Gotemba-shi Shizuoka 412-8513 (JP); ITO, Eriko, Gotemba-shi Shizuoka 412-8513 (JP)
(74) Representative: Bösl, Raphael Konrad
(86) International application number: PCT/JP2010/073361
(87) International publication number: WO 2011/078332

(56) References cited:
- EP-A1- 1 870 459
- EP-A1- 2 644 698
- WO-A1-2006/106905
- WO-A1-2007/114325
- WO-A1-2008/090960
- WO-A1-2010/151792
- WO-A1-2012/067176
- JP-A- 9 506 001
- US-A- 5 945 311
- VAN LOGHEM E ET AL: "Staphylococcal protein A and human IgG subclasses and allotypes", SCANDINAVIAN JOURNAL OF IMMUNOLOGY, BLACKWELL SCIENCE PUBL., OXFORD, GB, vol. 15, no. 3, 1 March 1982 (1982-03-01), pages 275-278, XP008112756, ISSN: 0300-9475
- JENDEBERG L ET AL: "Engineering of Fc1 and Fc3 from human immunoglobulin G to analyse subclass specificity for staphylococcal protein A", JOURNAL OF IMMUNOLOGICAL METHODS, ELSEVIER SCIENCE PUBLISHERS B.V.,AMSTERDAM, NL, vol. 201, no. 1, 14 February 1997 (1997-02-14), pages 25-34, XP004050039, ISSN: 0022-1759, DOI: 10.1016/S0022-1759(96)00215-3
- DUMONT JENNIFER A ET AL: "MONOMERIC FC FUSIONS: IMPACT ON PHARMACOKINETIC AND BIOLOGICAL ACTIVITY OF PROTEIN THERAPEUTICS", BIODRUGS: CLINICAL IMMUNOTHERAPEUTICS, BIOPHARMACEUTICALS AND GENE THERAPY, ADIS INTERNATIONAL, FR, vol. 20, no. 3, 1 January 2006 (2006-01-01), pages 151-160, XP009082835, ISSN: 1173-8804, DOI: 10.2165/00063030-200620030-00002
- LINDHOFER, HORST ET AL.: 'Preferential species-restricted heavy/light chain pairing in rat/mouse quadromas. Implications for a single- step purification of bispecific antibodies.' J. IMMUNOL. vol. 155, no. 1, 1995, pages 219 - 225, XP002190775
- KIM, JIN-KYOO ET AL.: 'Mapping the site on human IgG for binding of the MHC class I-related receptor, FcRn' EUR. J. IMMUNOL. vol. 29, 1999, pages 2819 - 2825, XP002300286

### SEQUENCE LISTING

<110> CHUGAI SEIYAKU KABUSHIKI KAISHA
<120> Polypeptide modification method for purifying multimeric polypeptides
<130> C1-A0916P
<150> JP 2009-294391
   <151> 2009-12-25
<160> 67
<170> PatentIn version 3.4
<210> 1
   <211> 123
   <212> PRT
   <213> Homo sapiens
<400> 1
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Asp Ile Asn Trp Val Arg Gln Ala Pro Gly Lys Gly Leu Glu Trp Val
           35                  40                  45
   Ala Ser Ile Ser Pro Ser Gly Gly Ser Thr Tyr Tyr Arg Arg Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Thr Arg Ala Gly His Asn Tyr Gly Ala Gly Trp Tyr Phe Asp Tyr
               100                 105                 110
   Trp Gly Gln Gly Thr Leu Val Thr Val Ser Ser
           115                 120
<210> 2
   <211> 123
   <212> PRT
   <213> Homo sapiens
<400> 2
   Gln Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Tyr Tyr
               20                  25                  30
   Asp Ile Asn Trp Val Arg Gln Ala Pro Gly Lys Gly Leu Glu Trp Val
           35                  40                  45
   Ser Ser Ile Ser Pro Ser Gly Gln Ser Thr Tyr Tyr Arg Arg Glu Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Arg Ala Gly His Asn Tyr Gly Ala Gly Trp Tyr Phe Asp Tyr
               100                 105                 110
   Trp Gly Gln Gly Thr Leu Val Thr Val Ser Ser
           115                 120
<210> 3
   <211> 119
   <212> PRT
   <213> Homo sapiens
<400> 3
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Thr Met Thr Ile Asp Lys Ser Thr Gly Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Arg Arg Ser Tyr Gly Tyr Tyr His Asp Glu Trp Gly Glu Gly
               100                 105                 110
   Thr Leu Val Thr Val Ser Ser
   115
<210> 4
   <211> 119
   <212> PRT
   <213> Homo sapiens
<400> 4
   Gln Val Gln Leu Val Gln Ser Gly Ser Glu Leu Lys Lys Pro Gly Ala
   1               5                   10                  15
   Ser Val Lys Val Ser Cys Lys Ala Ser Gly Tyr Thr Phe Thr Asp Asn
               20                  25                  30
   Asn Met Asp Trp Val Arg Gln Ala Pro Gly Gln Gly Leu Glu Trp Met
           35                  40                  45
   Gly Asp Ile Asn Thr Arg Ser Gly Gly Ser Ile Tyr Asn Glu Glu Phe
       50                  55                  60
   Gln Asp Arg Val Ile Met Thr Val Asp Lys Ser Thr Asp Thr Ala Tyr
   65                  70                  75                  80
   Met Glu Leu Ser Ser Leu Arg Ser Glu Asp Thr Ala Val Tyr His Cys
                   85                  90                  95
   Ala Arg Arg Lys Ser Tyr Gly Tyr Tyr Leu Asp Glu Trp Gly Glu Gly
               100                 105                 110
   Thr Leu Val Thr Val Ser Ser
           115
<210> 5
   <211> 119
   <212> PRT
   <213> Homo sapiens
<400> 5
<210> 6
   <211> 214
   <212> **PRT**
   <213> Homo sapiens
<400> 6
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Asn
               20                  25                  30
   Leu Ala Trp Tyr Gln Gln Lys Pro Gly Gln Ala Pro Glu Leu Leu Ile
           35                  40                  45
   Tyr Ser Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Leu Ala Thr Tyr Tyr Cys Gln Gln Tyr Tyr Ser Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 7
   <211> 214
   <212> PRT
   <213> Homo sapiens
<400> 7
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Asn
               20                  25                  30
   Leu Ala Trp Tyr Gln Gln Lys Pro Gly Gln Ala Pro Glu Leu Leu Ile
           35                  40                  45
   Tyr Ser Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Ser Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 8
   <211> 214
   <212> PRT
   <213> Homo sapiens
<400> 8
<210> 9
   <211> 325
   <212> PRT
   <213> Artificial
<220>
   <223> An artificially synthesized peptide sequence
<400> 9
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
                   180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu
                   325
<210> 10
   <211> 325
   <212> PRT
   <213> Artificial
<220>
   <223> An artificially synthesized peptide sequence
<400> 10
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn Arg Phe Thr Gln Lys Ser
   305                 310                 315                 320
   Leu Ser Leu Ser Pro
   325
<210> 11
   <211> 325
   <212> PRT
   <213> Artificial
<220>
   <223> An artificially synthesized peptide sequence
<400> 11
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Lys Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu
                   325
<210> 12
   <211> 325
   <212> PRT
   <213> Artificial
<220>
   <223> An artificially synthesized peptide sequence
<400> 12
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn Arg Phe Thr Gln Glu Ser
   305                 310                 315                 320
   Leu Ser Leu Ser Pro
                   325
<210> 13
   <211> 325
   <212> PRT
   <213> Artificial
<220>
   <223> An artificially synthesized peptide sequence
<400> 13
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Tyr
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Lys Glu Met Thr Lys
   225                 230                 235                 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Lys Leu Thr Val Asp Lys Ser Arg Trp Gln Glu Gly Asn Val Phe Ser
   290 295 300
   Cys Ser Val Met His Glu Ala Leu His Asn His Tyr Thr Gln Lys Ser
   305 310 315 320
   Leu Ser Leu Ser Leu
   325
<210> 14
   <211> 325
   <212> PRT
   <213> Artificial
<220>
   <223> An artificially synthesized peptide sequence
<400> 14
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
   130 135 140
   Asp Val Ser Gln Glu Asp Pro Glu Val Gln Phe Asn Trp Tyr Val Asp
   145 150 155 160
   Gly Val Glu Val His Asn Ala Lys Thr Lys Pro Arg Glu Glu Gln Tyr
   165 170 175
   Asn Ser Thr Tyr Arg Val Val Ser Val Leu Thr Val Leu His Gln Asp
   180 185 190
   Trp Leu Asn Gly Lys Glu Tyr Lys Cys Lys Val Ser Asn Lys Gly Leu
   195 200 205
   Pro Ser Ser Ile Glu Lys Thr Ile Ser Lys Ala Lys Gly Gln Pro Arg
   210 215 220
   Glu Pro Gln Val Tyr Thr Leu Pro Pro Ser Gln Glu Glu Met Thr Lys
   225 230 235 240
   Asn Gln Val Ser Leu Thr Cys Leu Val Lys Gly Phe Tyr Pro Ser Asp
   245 250 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Lys Leu Thr Val Asp Lys Ser Arg Trp Gln Glu Gly Asn Val Phe Ser
   290 295 300
   Cys Ser Val Met His Glu Ala Leu His Asn Arg Tyr Thr Gln Glu Ser
   305 310 315 320
   Leu Ser Leu Ser Pro
   325
<210> 15
   <211> 328
   <212> PRT
   <213> Artificial
<220>
   <223> An artificially synthesized peptide sequence
<400> 15
   Ala Ser Thr Lys Gly Pro Ser Val Phe Pro Leu Ala Pro Ser Ser Lys
   1 5 10 15
   Ser Thr Ser Gly Gly Thr Ala Ala Leu Gly Cys Leu Val Lys Asp Tyr
   20 25 30
   Phe Pro Glu Pro Val Thr Val Ser Trp Asn Ser Gly Ala Leu Thr Ser
   35 40 45
   Gly Val His Thr Phe Pro Ala Val Leu Gln Ser Ser Gly Leu Tyr Ser
   50 55 60
   Leu Ser Ser Val Val Thr Val Pro Ser Ser Ser Leu Gly Thr Gln Thr
   65 70 75 80
   Tyr Ile Cys Asn Val Asn His Lys Pro Ser Asn Thr Lys Val Asp Lys
   85 90 95
   Lys Val Glu Pro Lys Ser Cys Asp Lys Thr His Thr Cys Pro Pro Cys
   100 105 110
   Pro Ala Pro Glu Leu Leu Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Asn Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Asp Glu
   225 230 235 240
   Leu Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Lys Ser Leu Ser Leu Ser Pro
   325
<210> 16
   <211> 123
   <212> PRT
   <213> Homo sapiens
<400> 16
   Gln Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Tyr Tyr
   20 25 30
   Asp Ile Gln Trp Val Arg Gln Ala Pro Gly Lys Gly Leu Glu Trp Val
   35 40 45
   Ser Ser Ile Ser Pro Ser Gly Gln Ser Thr Tyr Tyr Arg Arg Glu Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Arg Thr Gly Arg Glu Tyr Gly Gly Gly Trp Tyr Phe Asp Tyr
   100 105 110
   Trp Gly Gln Gly Thr Leu Val Thr Val Ser Ser
   115 120
<210> 17
   <211> 119
   <212> PRT
   <213> Homo sapiens
<400> 17
   Gln Val Gln Leu Val Gln Ser Gly Ser Glu Leu Lys Lys Pro Gly Ala
   1 5 10 15
   Ser Val Lys Val Ser Cys Lys Ala Ser Gly Tyr Thr Phe Thr Asp Asn
   20 25 30
   Asn Met Asp Trp Val Arg Gln Ala Pro Gly Gln Gly Leu Glu Trp Met
   35 40 45
   Gly Asp Ile Asn Thr Arg Ser Gly Gly Ser Ile Tyr Asn Glu Glu Phe
   50 55 60
   Gln Asp Arg Val Ile Met Thr Val Asp Thr Ser Thr Asp Thr Ala Tyr
   65 70 75 80
   Met Glu Leu Ser Ser Leu Arg Ser Glu Asp Thr Ala Thr Tyr His Cys
   85 90 95
   Ala Arg Arg Lys Ser Tyr Gly Tyr His Leu Asp Glu Trp Gly Glu Gly
   100 105 110
   Thr Leu Val Thr Val Ser Ser
   115
<210> 18
   <211> 214
   <212> PRT
   <213> Homo sapiens
<400> 18
   Asp Ile Gln Met Thr Gln Ser Pro Ser Ser Leu Ser Ala Ser Val Gly
   1               5                   10                  15
   Asp Arg Val Thr Ile Thr Cys Lys Ala Ser Arg Asn Ile Glu Arg Gln
               20                  25                  30
   Leu Ala Trp Tyr Gln Gln Lys Pro Gly Gln Ala Pro Glu Leu Leu Ile
           35                  40                  45
   Tyr Gln Ala Ser Arg Lys Glu Ser Gly Val Pro Asp Arg Phe Ser Gly
       50                  55                  60
   Ser Arg Tyr Gly Thr Asp Phe Thr Leu Thr Ile Ser Ser Leu Gln Pro
   65                  70                  75                  80
   Glu Asp Ile Ala Thr Tyr Tyr Cys Gln Gln Tyr Ser Ser Pro Pro Leu
                   85                  90                  95
   Thr Phe Gly Gly Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 19
   <211> 325
   <212> PRT
   <213> Artificial
<220>
   <223> An artificially synthesized peptide sequence
<400> 19
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Tyr
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Lys Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Lys Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Pro
                   325
<210> 20
   <211> 325
   <212> PRT
   <213> Artificial
<220>
   <223> An artificially synthesized peptide sequence
<400> 20
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Tyr
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Lys Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
   260 265 270
   Thr Thr Pro Pro Val Leu Asp Ser Asp Gly Ser Phe Phe Leu Tyr Ser
   275 280 285
   Lys Leu Thr Val Asp Lys Ser Arg Trp Gln Gln Gly Asn Val Phe Ser
   290 295 300
   Cys Ser Val Met His Glu Ala Leu His Asn Arg Tyr Thr Gln Lys Ser
   305 310 315 320
   Leu Ser Leu Ser Pro
   325
<210> 21
   <211> 325
   <212> PRT
   <213> Artificial
<220>
   <223> An artificially synthesized peptide sequence
<400> 21
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Pro Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
           130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Tyr
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Lys Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Glu Ser
   305                 310                 315                 320
   Leu Ser Leu Ser Pro
                   325
<210> 22
   <211> 115
   <212> PRT
   <213> Homo sapiens
<400> 22
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Glu Met His Trp Ile Arg Gln Pro Pro Gly Gln Gly Leu Glu Trp Ile
           35                  40                  45
   Gly Ala Ile Asp Pro Lys Thr Gly Asp Thr Ala Tyr Ser Gln Lys Phe
       50                  55                  60
   Lys Gly Arg Val Thr Leu Thr Ala Asp Lys Ser Thr Ser Thr Ala Tyr
   65                  70                  75                      80
   Met Glu Leu Ser Ser Leu Thr Ser Glu Asp Thr Ala Val Tyr Tyr Cys
                   85                  90                  95
   Thr Arg Phe Tyr Ser Tyr Thr Tyr Trp Gly Gln Gly Thr Leu Val Thr
               100                 105                 110
   Val Ser Ser
           115
<210> 23
   <211> 219
   <212> PRT
   <213> Homo sapiens
<400> 23
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Val His Ser
               20                  25                  30
   Asn Arg Asn Thr Tyr Leu His Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Asn
                   85                  90                  95
   Thr His Val Pro Pro Thr Phe Gly Gln Gly Thr Lys Leu Glu Ile Lys
               100                 105                 110
   Arg Thr Val Ala Ala Pro Ser Val Phe Ile Phe Pro Pro Ser Asp Glu
           115                 120                 125
   Gln Leu Lys Ser Gly Thr Ala Ser Val Val Cys Leu Leu Asn Asn Phe
       130                 135                 140
   Tyr Pro Arg Glu Ala Lys Val Gln Trp Lys Val Asp Asn Ala Leu Gln
   145                 150                 155                 160
   Ser Gly Asn Ser Gln Glu Ser Val Thr Glu Gln Asp Ser Lys Asp Ser
                   165                 170                 175
   Thr Tyr Ser Leu Ser Ser Thr Leu Thr Leu Ser Lys Ala Asp Tyr Glu
               180                 185                 190
   Lys His Lys Val Tyr Ala Cys Glu Val Thr His Gln Gly Leu Ser Ser
           195                 200                 205
   Pro Val Thr Lys Ser Phe Asn Arg Gly Glu Cys
       210                 215
<210> 24
   <211> 328
   <212> PRT
   <213> Artificial
<220>
   <223> An artificially synthesized peptide sequence
<400> 24
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro
                   325
<210> 25
   <211> 587
   <212> PRT
   <213> Artificial
<220>
   <223> An artificially synthesized peptide sequence
<400> 25
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Gly Gly Gly Ser Gly Gly Gly
                   325                 330                 335
   Gly Ser Gln Ala Val Val Thr Gln Glu Ser Ala Leu Thr Thr Ser Pro
               340                 345                 350
   Gly Glu Thr Val Thr Leu Thr Cys Arg Ser Ser Thr Gly Ala Val Thr
           355                 360                 365
   Thr Ser Asn Tyr Ala Asn Trp Val Gln Glu Lys Pro Asp His Leu Phe
       370                 375                 380
   Thr Gly Leu Ile Gly Gly Thr Asn Lys Arg Ala Pro Gly Val Pro Ala
   385                 390                 395                 400
   Arg Phe Ser Gly Ser Leu Ile Gly Asp Lys Ala Ala Leu Thr Ile Thr
                   405                 410                 415
   Gly Ala Gln Thr Glu Asp Glu Ala Ile Tyr Phe Cys Ala Leu Trp Tyr
               420                 425                 430
   Ser Asn Leu Trp Val Phe Gly Gly Gly Thr Lys Leu Thr Val Leu Gly
           435                 440                 445
   Gly Gly Gly Ser Gly Gly Gly Gly Ser Gly Gly Gly Gly Ser Glu Val
       450                 455                 460
   Lys Leu Leu Glu Ser Gly Gly Gly Leu Val Gln Pro Lys Gly Ser Leu
   465                 470                 475                 480
   Lys Leu Ser Cys Ala Ala Ser Gly Phe Thr Phe Asn Thr Tyr Ala Met
                   485                 490                 495
   Asn Trp Val Arg Gln Ala Pro Gly Lys Gly Leu Glu Trp Val Ala Arg
               500                 505                 510
   Ile Arg Ser Lys Tyr Asn Asn Tyr Ala Thr Tyr Tyr Ala Asp Ser Val
           515                 520                 525
   Lys Asp Arg Phe Thr Ile Ser Arg Asp Asp Ser Gln Ser Ile Leu Tyr
   530 535 540
   Leu Gln Met Asn Asn Leu Lys Thr Glu Asp Thr Ala Met Tyr Tyr Cys
   545 550 555 560
   Val Arg His Gly Asn Phe Gly Asn Ser Tyr Val Ser Trp Phe Ala Tyr
   565 570 575
   Trp Gly Gln Gly Thr Leu Val Thr Val Ser Ala
   580 585
<210> 26
   <211> 328
   <212> PRT
   <213> Artificial
<220>
   <223> An artificially synthesized peptide sequence
<400> 26
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn Arg Tyr Thr
   305                 310                 315                 320
   Gln Glu Ser Leu Ser Leu Ser Pro
                   325
<210> 27
   <211> 587
   <212> PRT
   <213> Artificial
<220>
   <223> An artificially synthesized peptide sequence
<400> 27
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Lys Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Gly Gly Gly Ser Gly Gly Gly
                   325                 330                 335
   Gly Ser Gln Ala Val Val Thr Gln Glu Ser Ala Leu Thr Thr Ser Pro
               340                 345                 350
   Gly Glu Thr Val Thr Leu Thr Cys Arg Ser Ser Thr Gly Ala Val Thr
           355                 360                 365
   Thr Ser Asn Tyr Ala Asn Trp Val Gln Glu Lys Pro Asp His Leu Phe
       370                 375                 380
   Thr Gly Leu Ile Gly Gly Thr Asn Lys Arg Ala Pro Gly Val Pro Ala
   385                 390                 395                 400
   Arg Phe Ser Gly Ser Leu Ile Gly Asp Lys Ala Ala Leu Thr Ile Thr
                   405                 410                 415
   Gly Ala Gln Thr Glu Asp Glu Ala Ile Tyr Phe Cys Ala Leu Trp Tyr
               420                 425                 430
   Ser Asn Leu Trp Val Phe Gly Gly Gly Thr Lys Leu Thr Val Leu Gly
           435                 440                 445
   Gly Gly Gly Ser Gly Gly Gly Gly Ser Gly Gly Gly Gly Ser Glu Val
       450                 455                 460
   Lys Leu Leu Glu Ser Gly Gly Gly Leu Val Gln Pro Lys Gly Ser Leu
   465                 470                 475                 480
   Lys Leu Ser Cys Ala Ala Ser Gly Phe Thr Phe Asn Thr Tyr Ala Met
                   485                 490                 495
   Asn Trp Val Arg Gln Ala Pro Gly Lys Gly Leu Glu Trp Val Ala Arg
               500                 505                 510
   Ile Arg Ser Lys Tyr Asn Asn Tyr Ala Thr Tyr Tyr Ala Asp Ser Val
           515                 520                 525
   Lys Asp Arg Phe Thr Ile Ser Arg Asp Asp Ser Gln Ser Ile Leu Tyr
       530                 535                 540
   Leu Gln Met Asn Asn Leu Lys Thr Glu Asp Thr Ala Met Tyr Tyr Cys
   545                 550                 555                 560
   Val Arg His Gly Asn Phe Gly Asn Ser Tyr Val Ser Trp Phe Ala Tyr
   565 570 575
   Trp Gly Gln Gly Thr Leu Val Thr Val Ser Ala
   580 585
<210> 28
   <211> 328
   <212> PRT
   <213> Artificial
<220>
   <223> An artificially synthesized peptide sequence
<400> 28
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn Arg Tyr Thr
   305                 310                 315                 320
   Gln Lys Ser Leu Ser Leu Ser Pro
                   325
<210> 29
   <211> 230
   <212> PRT
   <213> Artificial
<220>
   <223> An artificially synthesized peptide sequence
<400> 29
   Glu Pro Lys Ser Cys Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65                  70                  75                  80
   Tyr Ala Ser Thr Tyr Arg Val Val Ser Val Leu Thr Val Leu His Gln
                   85                  90                  95
   Asp Trp Leu Asn Gly Lys Glu Tyr Lys Cys Lys Val Ser Asn Lys Ala
               100                 105                 110
   Leu Pro Ala Pro Ile Glu Lys Thr Ile Ser Lys Ala Lys Gly Gln Pro
           115                 120                 125
   Arg Glu Pro Gln Val Tyr Thr Leu Pro Pro Ser Arg Asp Glu Leu Thr
       130                 135                 140
   Lys Asn Gln Val Ser Leu Thr Cys Leu Val Lys Gly Phe Tyr Pro Ser
   145                 150                 155                 160
   Asp Ile Ala Val Glu Trp Glu Ser Asn Gly Gln Pro Glu Asn Asn Tyr
                   165                 170                 175
   Lys Thr Thr Pro Pro Val Leu Asp Ser Asp Gly Ser Phe Phe Leu Tyr
               180                 185                 190
   Ser Lys Leu Thr Val Asp Lys Ser Arg Trp Gln Gln Gly Asn Val Phe
           195                 200                 205
   Ser Cys Ser Val Met His Glu Ala Leu His Asn His Tyr Thr Gln Lys
       210                 215                 220
   Ser Leu Ser Leu Ser Pro
   225                 230
<210> 30
   <211> 230
   <212> PRT
   <213> Artificial
<220>
   <223> An artificially synthesized peptide sequence
<400> 30
   Glu Pro Lys Ser Cys Asp Lys Thr His Thr Cys Pro Pro Cys Pro Ala
   1               5                   10                  15
   Pro Glu Ala Ala Gly Gly Pro Ser Val Phe Leu Phe Pro Pro Lys Pro
               20                  25                  30
   Lys Asp Thr Leu Met Ile Ser Arg Thr Pro Glu Val Thr Cys Val Val
           35                  40                  45
   Val Asp Val Ser His Glu Asp Pro Glu Val Lys Phe Asn Trp Tyr Val
       50                  55                  60
   Asp Gly Val Glu Val His Asn Ala Lys Thr Lys Pro Arg Glu Glu Gln
   65 70 75 80
   Tyr Ala Ser Thr Tyr Arg Val Val Ser Val Leu Thr Val Leu His Gln
   85 90 95
   Asp Trp Leu Asn Gly Lys Glu Tyr Lys Cys Lys Val Ser Asn Lys Ala
   100 105 110
   Leu Pro Ala Pro Ile Glu Lys Thr Ile Ser Lys Ala Lys Gly Gln Pro
   115 120 125
   Arg Glu Pro Gln Val Tyr Thr Leu Pro Pro Ser Arg Lys Glu Leu Thr
   130 135 140
   Lys Asn Gln Val Ser Leu Thr Cys Leu Val Lys Gly Phe Tyr Pro Ser
   145 150 155 160
   Asp Ile Ala Val Glu Trp Glu Ser Asn Gly Gln Pro Glu Asn Asn Tyr
   165 170 175
   Lys Thr Thr Pro Pro Val Leu Asp Ser Asp Gly Ser Phe Phe Leu Tyr
   180 185 190
   Ser Lys Leu Thr Val Asp Lys Ser Arg Trp Gln Gln Gly Asn Val Phe
   195 200 205
   Ser Cys Ser Val Met His Glu Ala Leu His Asn His Tyr Thr Gln Lys
   210 215 220
   Ser Leu Ser Leu Ser Pro
   225 230
<210> 31
   <211> 190
   <212> PRT
   <213> Homo sapiens
<400> 31
   Pro Met Pro Phe Ile Ser Ala Lys Ser Ser Pro Val Ile Pro Leu Asp
   1 5 10 15
   Gly Ser Val Lys Ile Gln Cys Gln Ala Ile Arg Glu Ala Tyr Leu Thr
   20 25 30
   Gln Leu Met Ile Ile Lys Asn Ser Thr Tyr Arg Glu Ile Gly Arg Arg
   35 40 45
   Leu Lys Phe Trp Asn Glu Thr Asp Pro Glu Phe Val Ile Asp His Met
       50                  55                  60
   Asp Ala Asn Lys Ala Gly Arg Tyr Gln Cys Gln Tyr Arg Ile Gly His
   65                  70                  75                  80
   Tyr Arg Phe Arg Tyr Ser Asp Thr Leu Glu Leu Val Val Thr Gly Leu
                   85                  90                  95
   Tyr Gly Lys Pro Phe Leu Ser Ala Asp Arg Gly Leu Val Leu Met Pro
               100                 105                 110
   Gly Glu Asn Ile Ser Leu Thr Cys Ser Ser Ala His Ile Pro Phe Asp
           115                 120                 125
   Arg Phe Ser Leu Ala Lys Glu Gly Glu Leu Ser Leu Pro Gln His Gln
       130                 135                 140
   Ser Gly Glu His Pro Ala Asn Phe Ser Leu Gly Pro Val Asp Leu Asn
   145                 150                 155                 160
   Val Ser Gly Ile Tyr Arg Cys Tyr Gly Trp Tyr Asn Arg Ser Pro Tyr
                   165                 170                 175
   Leu Trp Ser Phe Pro Ser Asn Ala Leu Glu Leu Val Val Thr
               180                 185                 190
<210> 32
   <211> 221
   <212> PRT
   <213> Artificial
<220>
   <223> An artificially synthesized peptide sequence
<400> 32
   His Thr Cys Pro Pro Cys Pro Ala Pro Glu Leu Leu Gly Gly Pro Ser
   1               5                   10                  15
   Val Phe Leu Phe Pro Pro Lys Pro Lys Asp Thr Leu Met Ile Ser Arg
               20                  25                  30
   Thr Pro Glu Val Thr Cys Val Val Val Asp Val Ser His Glu Asp Pro
           35                  40                  45
   Glu Val Lys Phe Asn Trp Tyr Val Asp Gly Val Glu Val His Asn Ala
       50                  55                  60
   Lys Thr Lys Pro Arg Glu Glu Gln Tyr Asn Ser Thr Tyr Arg Val Val
   65                  70                  75                  80
   Ser Val Leu Thr Val Leu His Gln Asp Trp Leu Asn Gly Lys Glu Tyr
                   85                  90                  95
   Lys Cys Lys Val Ser Asn Lys Ala Leu Pro Ala Pro Ile Glu Lys Thr
               100                 105                 110
   Ile Ser Lys Ala Lys Gly Gln Pro Arg Glu Pro Gln Val Tyr Thr Leu
           115                 120                 125
   Pro Pro Ser Arg Asp Glu Leu Thr Lys Asn Gln Val Ser Leu Thr Cys
       130                 135                 140
   Leu Val Lys Gly Phe Tyr Pro Ser Asp Ile Ala Val Glu Trp Glu Ser
   145                 150                 155                 160
   Asn Gly Gln Pro Glu Asn Asn Tyr Lys Thr Thr Pro Pro Val Leu Asp
                   165                 170                 175
   Ser Asp Gly Ser Phe Phe Leu Tyr Ser Lys Leu Thr Val Asp Lys Ser
               180                 185                 190
   Arg Trp Gln Gln Gly Asn Val Phe Ser Cys Ser Val Met His Glu Ala
           195                 200                 205
   Leu His Asn His Tyr Thr Gln Lys Ser Leu Ser Leu Ser
       210                 215                 220
<210> 33
   <211> 221
   <212> PRT
   <213> Artificial
<220>
   <223> An artificially synthesized peptide sequence
<400> 33
   His Thr Cys Pro Pro Cys Pro Ala Pro Glu Leu Leu Gly Gly Pro Ser
   1               5                   10                  15
   Val Phe Leu Phe Pro Pro Lys Pro Lys Asp Thr Leu Met Ile Ser Arg
               20                  25                  30
   Thr Pro Glu Val Thr Cys Val Val Val Asp Val Ser His Glu Asp Pro
           35                  40                  45
   Glu Val Lys Phe Asn Trp Tyr Val Asp Gly Val Glu Val His Asn Ala
       50                  55                  60
   Lys Thr Lys Pro Arg Glu Glu Gln Tyr Asn Ser Thr Tyr Arg Val Val
   65                  70                  75                  80
   Ser Val Leu Thr Val Leu His Gln Asp Trp Leu Asn Gly Lys Glu Tyr
                   85                  90                  95
   Lys Cys Lys Val Ser Asn Lys Ala Leu Pro Ala Pro Ile Glu Lys Thr
               100                 105                 110
   Ile Ser Lys Ala Lys Gly Gln Pro Arg Glu Pro Gln Val Tyr Thr Leu
           115                 120                 125
   Pro Pro Ser Arg Lys Glu Leu Thr Lys Asn Gln Val Ser Leu Thr Cys
       130                 135                 140
   Leu Val Lys Gly Phe Tyr Pro Ser Asp Ile Ala Val Glu Trp Glu Ser
   145                 150                 155                 160
   Asn Gly Gln Pro Glu Asn Asn Tyr Lys Thr Thr Pro Pro Val Leu Asp
                   165                 170                 175
   Ser Asp Gly Ser Phe Phe Leu Tyr Ser Lys Leu Thr Val Asp Lys Ser
               180                 185                 190
   Arg Trp Gln Gln Gly Asn Val Phe Ser Cys Ser Val Met His Glu Ala
           195                 200                 205
   Leu His Asn Arg Tyr Thr Gln Lys Ser Leu Ser Leu Ser
       210                 215                 220
<210> 34
   <211> 221
   <212> PRT
   <213> Artificial
<220>
   <223> An artificially synthesized peptide sequence
<400> 34
   His Thr Cys Pro Pro Cys Pro Ala Pro Glu Leu Leu Gly Gly Pro Ser
   1               5                   10                  15
   Val Phe Leu Phe Pro Pro Lys Pro Lys Asp Thr Leu Met Ile Ser Arg
               20                  25                  30
   Thr Pro Glu Val Thr Cys Val Val Val Asp Val Ser His Glu Asp Pro
           35                  40                  45
   Glu Val Lys Phe Asn Trp Tyr Val Asp Gly Val Glu Val His Asn Ala
       50                  55                  60
   Lys Thr Lys Pro Arg Glu Glu Gln Tyr Asn Ser Thr Tyr Arg Val Val
   65                  70                  75                  80
   Ser Val Leu Thr Val Leu His Gln Asp Trp Leu Asn Gly Lys Glu Tyr
                   85                  90                  95
   Lys Cys Lys Val Ser Asn Lys Ala Leu Pro Ala Pro Ile Glu Lys Thr
               100                 105                 110
   Ile Ser Lys Ala Lys Gly Gln Pro Arg Glu Pro Gln Val Tyr Thr Leu
           115                 120                 125
   Pro Pro Ser Arg Asp Glu Leu Thr Lys Asn Gln Val Ser Leu Thr Cys
       130                 135                 140
   Leu Val Lys Gly Phe Tyr Pro Ser Asp Ile Ala Val Glu Trp Glu Ser
   145                 150                 155                 160
   Asn Gly Gln Pro Glu Asn Asn Tyr Lys Thr Thr Pro Pro Val Leu Asp
                   165                 170                 175
   Ser Asp Gly Ser Phe Phe Leu Tyr Ser Lys Leu Thr Val Asp Lys Ser
               180                 185                 190
   Arg Trp Gln Gln Gly Asn Val Phe Ser Cys Ser Val Met His Glu Ala
           195                 200                 205
   Leu His Asn His Tyr Thr Gln Glu Ser Leu Ser Leu Ser
       210                 215                 220
<210> 35
   <211> 20
   <212> PRT
   <213> Artificial
<220>
   <223> An artificially synthesized peptide sequence
<400> 35
   Gly Gly Gly Gly Ser Gly Gly Gly Gly Ser Gly Gly Gly Gly Ser Gly
   1               5                   10                  15
   Gly Gly Gly Ser
               20
<210> 36
   <211> 119
   <212> PRT
   <213> Homo sapiens
<400> 36
<210> 37
   <211> 115
   <212> PRT
   <213> Homo sapiens
<400> 37
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Glu Met His Trp Val Arg Gln Ala Pro Gly Gln Gly Leu Glu Trp Met
           35                  40                  45
   Gly Ala Leu Asp Pro Lys Thr Gly Asp Thr Ala Tyr Ser Gln Lys Phe
       50                  55                  60
   Lys Gly Arg Val Thr Leu Thr Ala Asp Lys Ser Thr Ser Thr Ala Tyr
   65                  70                  75                  80
   Met Glu Leu Ser Ser Leu Thr Ser Glu Asp Thr Ala Val Tyr Tyr Cys
                   85                  90                  95
   Thr Arg Phe Tyr Ser Tyr Thr Tyr Trp Gly Gln Gly Thr Leu Val Thr
               100                 105                 110
   Val Ser Ser
           115
<210> 38
   <211> 119
   <212> PRT
   <213> Homo sapiens
<400> 38
   Gln Val Gln Leu Gln Glu Ser Gly Pro Gly Leu Val Lys Pro Ser Glu
   1               5                   10                  15
   Thr Leu Ser Leu Thr Cys Ala Val Ser Gly Tyr Ser Ile Ser Asp Asp
               20                  25                  30
   Gln Ala Trp Ser Trp Val Arg Gln Pro Pro Gly Glu Gly Leu Glu Trp
           35                  40                  45
   Ile Gly Tyr Ile Ser Tyr Ser Gly Ile Thr Asn Tyr Asn Pro Ser Leu
       50                  55                  60
   Lys Gly Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Ala Tyr Tyr Cys
                   85                  90                  95
   Ala Arg Ser Leu Ala Arg Thr Thr Ala Met Asp Tyr Trp Gly Glu Gly
               100                 105                 110
   Thr Leu Val Thr Val Ser Ser
           115
<210> 39
   <211> 115
   <212> PRT
   <213> Homo sapiens
<400> 39
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Glu Met His Trp Ile Arg Gln Pro Pro Gly Lys Gly Leu Glu Trp Ile
           35                  40                  45
   Gly Ala Ile Asn Pro Lys Thr Gly Asp Thr Ala Tyr Ser Gln Lys Phe
       50                  55                  60
   Lys Gly Arg Val Thr Leu Thr Ala Asp Lys Ser Thr Ser Thr Ala Tyr
   65                  70                  75                  80
   Met Glu Leu Ser Ser Leu Thr Ser Glu Asp Thr Ala Val Tyr Tyr Cys
                   85                  90                  95
   Thr Arg Phe Tyr Ser Tyr Thr Tyr Trp Gly Arg Gly Thr Leu Val Thr
               100                 105                 110
   Val Ser Ser
           115
<210> 40
   <211> 119
   <212> PRT
   <213> Artificial
<220>
   <223> An artificially synthesized peptide sequence
<400> 40
   Gln Val Gln Leu Gln Glu Ser Gly Pro Gly Leu Val Lys Pro Ser Glu
   1               5                   10                  15
   Thr Leu Ser Leu Thr Cys Ala Val Ser Gly Tyr Ser Ile Ser Asp Asp
               20                  25                  30
   Gln Ala Trp Ser Trp Val Arg Lys Pro Pro Gly Glu Gly Leu Glu Trp
           35                  40                  45
   Ile Gly Tyr Ile Ser Tyr Ser Gly Ile Thr Asn Tyr Asn Pro Ser Leu
       50                  55                  60
   Lys Gly Arg Val Thr Ile Ser Arg Asp Thr Ser Lys Asn Gln Phe Ser
   65                  70                  75                  80
   Leu Lys Leu Ser Ser Val Thr Ala Ala Asp Thr Ala Ala Tyr Tyr Cys
                   85                  90                  95
   Ala Arg Ser Leu Ala Arg Thr Thr Ala Met Asp Tyr Trp Gly Glu Gly
               100                 105                 110
   Thr Leu Val Thr Val Ser Ser
           115
<210> 41
   <211> 115
   <212> PRT
   <213> Artificial
<220>
   <223> An artificially synthesized peptide sequence
<400> 41
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Asp Tyr
               20                  25                  30
   Glu Met His Trp Ile Arg Lys Pro Pro Gly Lys Gly Leu Glu Trp Ile
           35                  40                  45
   Gly Ala Ile Asn Pro Lys Thr Gly Asp Thr Ala Tyr Ser Gln Lys Phe
       50                  55                  60
   Lys Gly Arg Val Thr Leu Thr Ala Asp Lys Ser Thr Ser Thr Ala Tyr
   65                  70                  75                  80
   Met Glu Leu Ser Ser Leu Thr Ser Glu Asp Thr Ala Val Tyr Tyr Cys
                   85                  90                  95
   Thr Arg Phe Tyr Ser Tyr Thr Tyr Trp Gly Arg Gly Thr Leu Val Thr
               100                 105                 110
   Val Ser Ser
           115
<210> 42
   <211> 328
   <212> **PRT**
   <213> Artificial
<220>
   <223> An artificially synthesized peptide sequence
<400> 42
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Ala Ala Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Ala Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro
                   325
<210> 43
   <211> 330
   <212> PRT
   <213> Artificial
<220>
   <223> An artificially synthesized peptide sequence
<400> 43
   Ser Ser Ala Ser Thr Lys Gly Pro Ser Val Phe Pro Leu Ala Pro Ser
   1               5                   10                  15
   Ser Lys Ser Thr Ser Gly Gly Thr Ala Ala Leu Gly Cys Leu Val Lys
               20                  25                  30
   Asp Tyr Phe Pro Glu Pro Val Thr Val Ser Trp Asn Ser Gly Ala Leu
           35                  40                  45
   Thr Ser Gly Val His Thr Phe Pro Ala Val Leu Gln Ser Ser Gly Leu
       50                  55                  60
   Tyr Ser Leu Ser Ser Val Val Thr Val Pro Ser Ser Ser Leu Gly Thr
   65                  70                  75                  80
   Gln Thr Tyr Ile Cys Asn Val Asn His Lys Pro Ser Asn Thr Lys Val
                   85                  90                  95
   Asp Lys Lys Val Glu Pro Lys Ser Cys Asp Lys Thr His Thr Cys Pro
               100                 105                 110
   Pro Cys Pro Ala Pro Glu Ala Ala Gly Gly Pro Ser Val Phe Leu Phe
           115                 120                 125
   Pro Pro Lys Pro Lys Asp Thr Leu Met Ile Ser Arg Thr Pro Glu Val
       130                 135                 140
   Thr Cys Val Val Val Asp Val Ser His Glu Asp Pro Glu Val Lys Phe
   145                 150                 155                 160
   Asn Trp Tyr Val Asp Gly Val Glu Val His Asn Ala Lys Thr Lys Pro
                   165                 170                 175
   Arg Glu Glu Gln Tyr Ala Ser Thr Tyr Arg Val Val Ser Val Leu Thr
               180                 185                 190
   Val Leu His Gln Asp Trp Leu Asn Gly Lys Glu Tyr Lys Cys Lys Val
           195                 200                 205
   Ser Asn Lys Ala Leu Pro Ala Pro Ile Glu Lys Thr Ile Ser Lys Ala
       210                 215                 220
   Lys Gly Gln Pro Arg Glu Pro Gln Val Tyr Thr Leu Pro Pro Ser Arg
   225                 230                 235                 240
   Asp Glu Leu Thr Lys Asn Gln Val Ser Leu Thr Cys Leu Val Lys Gly
                   245                 250                 255
   Phe Tyr Pro Ser Asp Ile Ala Val Glu Trp Glu Ser Asn Gly Gln Pro
               260                 265                 270
   Glu Asn Asn Tyr Lys Thr Thr Pro Pro Val Leu Asp Ser Asp Gly Ser
           275                 280                 285
   Phe Phe Leu Tyr Ser Lys Leu Thr Val Asp Lys Ser Arg Trp Gln Gln
       290                 295                 300
   Gly Asn Val Phe Ser Cys Ser Val Met His Glu Ala Leu His Asn His
   305                 310                 315                 320
   Tyr Thr Gln Lys Ser Leu Ser Leu Ser Pro
                   325                 330
<210> 44
   <211> 328
   <212> PRT
   <213> Artificial
<220>
   <223> An artificially synthesized peptide sequence
<400> 44
   Ala Ser Thr Lys Gly Pro Ser Val Phe Pro Leu Ala Pro Ser Ser Lys
   1               5                   10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
   100 105 110
   Pro Ala Pro Glu Ala Ala Gly Gly Pro Ser Val Phe Leu Phe Pro Pro
   115 120 125
   Lys Pro Lys Asp Thr Leu Met Ile Ser Arg Thr Pro Glu Val Thr Cys
   130 135 140
   Val Val Val Asp Val Ser His Glu Asp Pro Glu Val Lys Phe Asn Trp
   145 150 155 160
   Tyr Val Asp Gly Val Glu Val His Asn Ala Lys Thr Lys Pro Arg Glu
   165 170 175
   Glu Gln Tyr Ala Ser Thr Tyr Arg Val Val Ser Val Leu Thr Val Leu
   180 185 190
   His Gln Asp Trp Leu Asn Gly Lys Glu Tyr Lys Cys Lys Val Ser Asn
   195 200 205
   Lys Ala Leu Pro Ala Pro Ile Glu Lys Thr Ile Ser Lys Ala Lys Gly
   210 215 220
   Gln Pro Arg Glu Pro Gln Val Tyr Thr Leu Pro Pro Ser Arg Asp Glu
   225 230 235 240
   Leu Thr Lys Asn Gln Val Ser Leu Thr Cys Leu Val Lys Gly Phe Tyr
   245 250 255
   Pro Ser Asp Ile Ala Val Glu Trp Glu Ser Asn Gly Gln Pro Glu Asn
   260 265 270
   Asn Tyr Lys Thr Thr Pro Pro Val Leu Asp Ser Asp Gly Ser Phe Phe
   275 280 285
   Leu Tyr Ser Lys Leu Thr Val Asp Lys Ser Arg Trp Gln Gln Gly Asn
   290 295 300
   Val Phe Ser Cys Ser Val Met His Glu Ala Leu His Asn His Tyr Thr
   305 310 315 320
   Gln Glu Ser Leu Ser Leu Ser Pro
   325
<210> 45
   <211> 330
   <212> PRT
   <213> Artificial
<220>
   <223> An artificially synthesized peptide sequence
<400> 45
   Ser Ser Ala Ser Thr Lys Gly Pro Ser Val Phe Pro Leu Ala Pro Ser
   1               5                   10                  15
   Ser Lys Ser Thr Ser Gly Gly Thr Ala Ala Leu Gly Cys Leu Val Lys
               20                  25                  30
   Asp Tyr Phe Pro Glu Pro Val Thr Val Ser Trp Asn Ser Gly Ala Leu
           35                  40                  45
   Thr Ser Gly Val His Thr Phe Pro Ala Val Leu Gln Ser Ser Gly Leu
       50                  55                  60
   Tyr Ser Leu Ser Ser Val Val Thr Val Pro Ser Ser Ser Leu Gly Thr
   65                  70                  75                  80
   Gln Thr Tyr Ile Cys Asn Val Asn His Lys Pro Ser Asn Thr Lys Val
                   85                  90                  95
   Asp Lys Lys Val Glu Pro Lys Ser Cys Asp Lys Thr His Thr Cys Pro
               100                 105                 110
   Pro Cys Pro Ala Pro Glu Ala Ala Gly Gly Pro Ser Val Phe Leu Phe
           115                 120                 125
   Pro Pro Lys Pro Lys Asp Thr Leu Met Ile Ser Arg Thr Pro Glu Val
       130                 135                 140
   Thr Cys Val Val Val Asp Val Ser His Glu Asp Pro Glu Val Lys Phe
   145                 150                 155                 160
   Asn Trp Tyr Val Asp Gly Val Glu Val His Asn Ala Lys Thr Lys Pro
                   165                 170                 175
   Arg Glu Glu Gln Tyr Ala Ser Thr Tyr Arg Val Val Ser Val Leu Thr
               180                 185                 190
   Val Leu His Gln Asp Trp Leu Asn Gly Lys Glu Tyr Lys Cys Lys Val
           195                 200                 205
   Ser Asn Lys Ala Leu Pro Ala Pro Ile Glu Lys Thr Ile Ser Lys Ala
       210                 215                 220
   Lys Gly Gln Pro Arg Glu Pro Gln Val Tyr Thr Leu Pro Pro Ser Arg
   225                 230                 235                 240
   Lys Glu Leu Thr Lys Asn Gln Val Ser Leu Thr Cys Leu Val Lys Gly
                   245                 250                 255
   Phe Tyr Pro Ser Asp Ile Ala Val Glu Trp Glu Ser Asn Gly Gln Pro
               260                 265                 270
   Glu Asn Asn Tyr Lys Thr Thr Pro Pro Val Leu Asp Ser Asp Gly Ser
           275                 280                 285
   Phe Phe Leu Tyr Ser Lys Leu Thr Val Asp Lys Ser Arg Trp Gln Gln
       290                 295                 300
   Gly Asn Val Phe Ser Cys Ser Val Met His Glu Ala Leu His Asn Arg
   305                 310                 315                 320
   Tyr Thr Gln Lys Ser Leu Ser Leu Ser Pro
                   325                 330
<210> 46
   <211> 107
   <212> PRT
   <213> Homo sapiens
<400> 46
<210> 47
   <211> 112
   <212> PRT
   <213> Homo sapiens
<400> 47
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Val His Ser
               20                  25                  30
   Asn Arg Asn Thr Tyr Leu His Trp Tyr Leu Gln Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Asn
                   85                  90                  95
   Thr His Val Pro Pro Thr Phe Gly Gln Gly Thr Lys Leu Glu Ile Lys
               100                 105                 110
<210> 48
   <211> 107
   <212> PRT
   <213> Homo sapiens
<400> 48
   Asp Ile Gln Met Thr Gln Ser Pro Ser Ser Leu Ser Ala Ser Val Gly
   1               5                   10                  15
   Asp Ser Val Thr Ile Thr Cys Gln Ala Ser Gln Asp Ile Ser Ser Tyr
               20                  25                  30
   Leu Asn Trp Tyr Gln Gln Lys Pro Gly Lys Ala Pro Glu Leu Leu Ile
           35                  40                  45
   Tyr Tyr Gly Ser Glu Leu His Ser Gly Val Pro Ser Arg Phe Ser Gly
       50                  55                  60
   Ser Gly Ser Gly Thr Asp Phe Thr Phe Thr Ile Ser Ser Leu Glu Ala
   65                  70                  75                  80
   Glu Asp Ala Ala Thr Tyr Tyr Cys Gln Gln Gly Asn Ser Leu Pro Tyr
                   85                  90                  95
   Thr Phe Gly Gln Gly Thr Lys Val Glu Ile Glu
               100                 105
<210> 49
   <211> 107
   <212> PRT
   <213> Artificial
<220>
   <223> An artificially synthesized peptide sequence
<400> 49
   Asp Ile Gln Met Thr Gln Ser Pro Ser Ser Leu Ser Ala Ser Val Gly
   1               5                   10                  15
   Asp Ser Val Thr Ile Thr Cys Gln Ala Ser Gln Asp Ile Ser Ser Tyr
               20                  25                  30
   Leu Asn Trp Tyr Gln Glu Lys Pro Gly Lys Ala Pro Glu Leu Leu Ile
           35                  40                  45
   Tyr Tyr Gly Ser Glu Leu His Ser Gly Val Pro Ser Arg Phe Ser Gly
       50                  55                  60
   Ser Gly Ser Gly Thr Asp Phe Thr Phe Thr Ile Ser Ser Leu Glu Ala
   65                  70                  75                  80
   Glu Asp Ala Ala Thr Tyr Tyr Cys Gln Gln Gly Asn Ser Leu Pro Tyr
                   85                  90                  95
   Thr Phe Gly Gln Gly Thr Lys Val Glu Ile Glu
               100                 105
<210> 50
   <211> 112
   <212> PRT
   <213> Artificial
<220>
   <223> An artificially synthesized peptide sequence
<400> 50
   Asp Val Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Val His Ser
               20                  25                  30
   Asn Arg Asn Thr Tyr Leu His Trp Tyr Leu Glu Lys Pro Gly Gln Ser
           35                  40                  45
   Pro Gln Leu Leu Ile Tyr Lys Val Ser Asn Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Ser Gln Asn
   85 90 95
   Thr His Val Pro Pro Thr Phe Gly Gln Gly Thr Lys Leu Glu Ile Lys
   100 105 110
<210> 51
   <211> 107
   <212> PRT
   <213> Homo sapiens
<400> 51
<210> 52
   <211> 107
   <212> PRT
   <213> Artificial
<220>
   <223> An artificially synthesized peptide sequence
<400> 52
   Ala Ser Val Ala Ala Pro Ser Val Phe Ile Phe Pro Pro Ser Asp Glu
   1               5                   10                  15
   Gln Leu Lys Ser Gly Thr Ala Ser Val Val Cys Leu Leu Asn Asn Phe
               20                  25                  30
   Tyr Pro Arg Glu Ala Lys Val Gln Trp Lys Val Asp Asn Ala Leu Gln
           35                  40                  45
   Ser Gly Asn Ser Gln Glu Ser Val Thr Glu Gln Asp Ser Lys Asp Ser
       50                  55                  60
   Thr Tyr Ser Leu Ser Ser Thr Leu Thr Leu Ser Lys Ala Asp Tyr Glu
   65                  70                  75                  80
   Lys His Lys Val Tyr Ala Cys Glu Val Thr His Gln Gly Leu Ser Ser
                   85                  90                  95
   Pro Val Thr Lys Ser Phe Asn Arg Gly Glu Cys
               100                 105
<210> 53
   <211> 466
   <212> PRT
   <213> Artificial
<220>
   <223> An artificially synthesized peptide sequence
<400> 53
   Met Gly Trp Ser Cys Ile Ile Leu Phe Leu Val Ala Thr Ala Thr Gly
   1               5                   10                  15
   Val His Ser Gln Val Gln Leu Gln Glu Ser Gly Pro Gly Leu Val Arg
               20                  25                  30
   Pro Ser Gln Thr Leu Ser Leu Thr Cys Thr Val Ser Gly Tyr Ser Ile
           35                  40                  45
   Thr Ser Asp His Ala Trp Ser Trp Val Arg Gln Pro Pro Gly Arg Gly
       50                  55                  60
   Leu Glu Trp Ile Gly Tyr Ile Ser Tyr Ser Gly Ile Thr Thr Tyr Asn
   65                  70                  75                  80
   Pro Ser Leu Lys Ser Arg Val Thr Met Leu Arg Asp Thr Ser Lys Asn
                   85                  90                  95
   Gln Phe Ser Leu Arg Leu Ser Ser Val Thr Ala Ala Asp Thr Ala Val
               100                 105                 110
   Tyr Tyr Cys Ala Arg Ser Leu Ala Arg Thr Thr Ala Met Asp Tyr Trp
           115                 120                 125
   Gly Gln Gly Ser Leu Val Thr Val Ser Ser Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
   165 170 175
   Val Ser Trp Asn Ser Gly Ala Leu Thr Ser Gly Val His Thr Phe Pro
   180 185 190
   Ala Val Leu Gln Ser Ser Gly Leu Tyr Ser Leu Ser Ser Val Val Thr
   195 200 205
   Val Pro Ser Ser Ser Leu Gly Thr Gln Thr Tyr Ile Cys Asn Val Asn
   210 215 220
   His Lys Pro Ser Asn Thr Lys Val Asp Lys Lys Val Glu Pro Lys Ser
   225 230 235 240
   Cys Asp Lys Thr His Thr Cys Pro Pro Cys Pro Ala Pro Glu Ala Ala
   245 250 255
   Gly Gly Pro Ser Val Phe Leu Phe Pro Pro Lys Pro Lys Asp Thr Leu
   260 265 270
   Met Ile Ser Arg Thr Pro Glu Val Thr Cys Val Val Val Asp Val Ser
   275 280 285
   His Glu Asp Pro Glu Val Lys Phe Asn Trp Tyr Val Asp Gly Val Glu
   290 295 300
   Val His Asn Ala Lys Thr Lys Pro Arg Glu Glu Gln Tyr Ala Ser Thr
   305 310 315 320
   Tyr Arg Val Val Ser Val Leu Thr Val Leu His Gln Asp Trp Leu Asn
   325 330 335
   Gly Lys Glu Tyr Lys Cys Lys Val Ser Asn Lys Ala Leu Pro Ala Pro
   340 345 350
   Ile Glu Lys Thr Ile Ser Lys Ala Lys Gly Gln Pro Arg Glu Pro Gln
   355 360 365
   Val Tyr Thr Leu Pro Pro Ser Arg Asp Glu Leu Thr Lys Asn Gln Val
   370 375 380
   Ser Leu Thr Cys Leu Val Lys Gly Phe Tyr Pro Ser Asp Ile Ala Val
   385 390 395 400
   Glu Trp Glu Ser Asn Gly Gln Pro Glu Asn Asn Tyr Lys Thr Thr Pro
   405 410 415
   Pro Val Leu Asp Ser Asp Gly Ser Phe Phe Leu Tyr Ser Lys Leu Thr
               420                 425                 430
   Val Asp Lys Ser Arg Trp Gln Gln Gly Asn Val Phe Ser Cys Ser Val
           435                 440                 445
   Met His Glu Ala Leu His Asn His Tyr Thr Gln Lys Ser Leu Ser Leu
       450                 455                 460
   Ser Pro
   465
<210> 54
   <211> 233
   <212> PRT
   <213> Artificial
<220>
   <223> An artificially synthesized peptide sequence
<400> 54
   Met Gly Trp Ser Cys Ile Ile Leu Phe Leu Val Ala Thr Ala Thr Gly
   1               5                   10                  15
   Val His Ser Asp Ile Gln Met Thr Gln Ser Pro Ser Ser Leu Ser Ala
               20                  25                  30
   Ser Val Gly Asp Arg Val Thr Ile Thr Cys Arg Ala Ser Gln Asp Ile
           35                  40                  45
   Ser Ser Tyr Leu Asn Trp Tyr Gln Gln Lys Pro Gly Lys Ala Pro Lys
       50                  55                  60
   Leu Leu Ile Tyr Tyr Thr Ser Arg Leu His Ser Gly Val Pro Ser Arg
   65                  70                  75                  80
   Phe Ser Gly Ser Gly Ser Gly Thr Asp Phe Thr Phe Thr Ile Ser Ser
                   85                  90                  95
   Leu Gln Pro Glu Asp Ile Ala Thr Tyr Tyr Cys Gln Gln Gly Asn Thr
               100                 105                 110
   Leu Pro Tyr Thr Phe Gly Gln Gly Thr Lys Val Glu Ile Lys Arg Thr
           115                 120                 125
   Val Ala Ala Pro Ser Val Phe Ile Phe Pro Pro Ser Asp Glu Gln Leu
       130                 135                 140
   Lys Ser Gly Thr Ala Ser Val Val Cys Leu Leu Asn Asn Phe Tyr Pro
   145                 150                 155                 160
   Arg Glu Ala Lys Val Gln Trp Lys Val Asp Asn Ala Leu Gln Ser Gly
                   165                 170                 175
   Asn Ser Gln Glu Ser Val Thr Glu Gln Asp Ser Lys Asp Ser Thr Tyr
               180                 185                 190
   Ser Leu Ser Ser Thr Leu Thr Leu Ser Lys Ala Asp Tyr Glu Lys His
           195                 200                 205
   Lys Val Tyr Ala Cys Glu Val Thr His Gln Gly Leu Ser Ser Pro Val
       210                 215                 220
   Thr Lys Ser Phe Asn Arg Gly Glu Cys
   225                 230
<210> 55
   <211> 462
   <212> PRT
   <213> Artificial
<220>
   <223> An artificially synthesized peptide sequence
<400> 55
   Met Asp Trp Thr Trp Arg Phe Leu Phe Val Val Ala Ala Ala Thr Gly
   1               5                   10                  15
   Val Gln Ser Gln Val Gln Leu Val Gln Ser Gly Ala Glu Val Lys Lys
               20                  25                  30
   Pro Gly Ala Ser Val Lys Val Ser Cys Lys Ala Ser Gly Tyr Thr Phe
           35                  40                  45
   Thr Asp Tyr Glu Met His Trp Val Arg Gln Ala Pro Gly Gln Gly Leu
       50                  55                  60
   Glu Trp Met Gly Ala Leu Asp Pro Lys Thr Gly Asp Thr Ala Tyr Ser
   65                  70                  75                  80
   Gln Lys Phe Lys Gly Arg Val Thr Leu Thr Ala Asp Lys Ser Thr Ser
                   85                  90                  95
   Thr Ala Tyr Met Glu Leu Ser Ser Leu Thr Ser Glu Asp Thr Ala Val
               100                 105                 110
   Tyr Tyr Cys Thr Arg Phe Tyr Ser Tyr Thr Tyr Trp Gly Gln Gly Thr
           115                 120                 125
   Leu Val Thr Val Ser Ser Ala Ser Thr Lys Gly Pro Ser Val Phe Pro
       130                 135                 140
   Leu Ala Pro Ser Ser Lys Ser Thr Ser Gly Gly Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Ser Leu Gly Thr Gln Thr Tyr Ile Cys Asn Val Asn His Lys Pro Ser
       210                 215                 220
   Asn Thr Lys Val Asp Lys Lys Val Glu Pro Lys Ser Cys Asp Lys Thr
   225                 230                 235                 240
   His Thr Cys Pro Pro Cys Pro Ala Pro Glu Ala Ala Gly Gly Pro Ser
                   245                 250                 255
   Val Phe Leu Phe Pro Pro Lys Pro Lys Asp Thr Leu Met Ile Ser Arg
               260                 265                 270
   Thr Pro Glu Val Thr Cys Val Val Val Asp Val Ser His Glu Asp Pro
           275                 280                 285
   Glu Val Lys Phe Asn Trp Tyr Val Asp Gly Val Glu Val His Asn Ala
       290                 295                 300
   Lys Thr Lys Pro Arg Glu Glu Gln Tyr Ala Ser Thr Tyr Arg Val Val
   305                 310                 315                 320
   Ser Val Leu Thr Val Leu His Gln Asp Trp Leu Asn Gly Lys Glu Tyr
                   325                 330                 335
   Lys Cys Lys Val Ser Asn Lys Ala Leu Pro Ala Pro Ile Glu Lys Thr
               340                 345                 350
   Ile Ser Lys Ala Lys Gly Gln Pro Arg Glu Pro Gln Val Tyr Thr Leu
           355                 360                 365
   Pro Pro Ser Arg Asp Glu Leu Thr Lys Asn Gln Val Ser Leu Thr Cys
       370                 375                 380
   Leu Val Lys Gly Phe Tyr Pro Ser Asp Ile Ala Val Glu Trp Glu Ser
   385                 390                 395                 400
   Asn Gly Gln Pro Glu Asn Asn Tyr Lys Thr Thr Pro Pro Val Leu Asp
                   405                 410                 415
   Ser Asp Gly Ser Phe Phe Leu Tyr Ser Lys Leu Thr Val Asp Lys Ser
               420                 425                 430
   Arg Trp Gln Gln Gly Asn Val Phe Ser Cys Ser Val Met His Glu Ala
           435                 440                 445
   Leu His Asn His Tyr Thr Gln Lys Ser Leu Ser Leu Ser Pro
       450                 455                 460
<210> 56
   <211> 239
   <212> PRT
   <213> Artificial
<220>
   <223> An artificially synthesized peptide sequence
<400> 56
   Met Arg Leu Pro Ala Gln Leu Leu Gly Leu Leu Met Leu Trp Val Ser
   1               5                   10                  15
   Gly Ser Ser Gly Asp Val Val Met Thr Gln Ser Pro Leu Ser Leu Pro
               20                  25                  30
   Val Thr Pro Gly Glu Pro Ala Ser Ile Ser Cys Arg Ser Ser Gln Ser
           35                  40                  45
   Leu Val His Ser Asn Arg Asn Thr Tyr Leu His Trp Tyr Leu Gln Lys
       50                  55                  60
   Pro Gly Gln Ser Pro Gln Leu Leu Ile Tyr Lys Val Ser Asn Arg Phe
   65                  70                  75                  80
   Ser Gly Val Pro Asp Arg Phe Ser Gly Ser Gly Ser Gly Thr Asp Phe
                   85                  90                  95
   Thr Leu Lys Ile Ser Arg Val Glu Ala Glu Asp Val Gly Val Tyr Tyr
               100                 105                 110
   Cys Ser Gln Asn Thr His Val Pro Pro Thr Phe Gly Gln Gly Thr Lys
           115                 120                 125
   Leu Glu Ile Lys Arg Thr Val Ala Ala Pro Ser Val Phe Ile Phe Pro
       130                 135                 140
   Pro Ser Asp Glu Gln Leu Lys Ser Gly Thr Ala Ser Val Val Cys Leu
   145                 150                 155                 160
   Leu Asn Asn Phe Tyr Pro Arg Glu Ala Lys Val Gln Trp Lys Val Asp
                   165                 170                 175
   Asn Ala Leu Gln Ser Gly Asn Ser Gln Glu Ser Val Thr Glu Gln Asp
               180                 185                 190
   Ser Lys Asp Ser Thr Tyr Ser Leu Ser Ser Thr Leu Thr Leu Ser Lys
           195                 200                 205
   Ala Asp Tyr Glu Lys His Lys Val Tyr Ala Cys Glu Val Thr His Gln
       210                 215                 220
   Gly Leu Ser Ser Pro Val Thr Lys Ser Phe Asn Arg Gly Glu Cys
   225                 230                 235
<210> 57
   <211> 462
   <212> PRT
   <213> Artificial
<220>
   <223> An artificially synthesized peptide sequence
<400> 57
   Met Arg Leu Pro Ala Gln Leu Leu Gly Leu Leu Met Leu Trp Val Ser
   1               5                   10                  15
   Gly Ser Ser Gly Asp Val Val Met Thr Gln Ser Pro Leu Ser Leu Pro
               20                  25                  30
   Val Thr Pro Gly Glu Pro Ala Ser Ile Ser Cys Arg Ser Ser Gln Ser
           35                  40                  45
   Leu Val His Ser Asn Arg Asn Thr Tyr Leu His Trp Tyr Leu Gln Lys
       50                  55                  60
   Pro Gly Gln Ser Pro Gln Leu Leu Ile Tyr Lys Val Ser Asn Arg Phe
   65                  70                  75                  80
   Ser Gly Val Pro Asp Arg Phe Ser Gly Ser Gly Ser Gly Thr Asp Phe
                   85                  90                  95
   Thr Leu Lys Ile Ser Arg Val Glu Ala Glu Asp Val Gly Val Tyr Tyr
               100                 105                 110
   Cys Ser Gln Asn Thr His Val Pro Pro Thr Phe Gly Gln Gly Thr Lys
           115                 120                 125
   Leu Glu Ile Lys Ser Ser Ala Ser Thr Lys Gly Pro Ser Val Phe Pro
       130                 135                 140
   Leu Ala Pro Ser Ser Lys Ser Thr Ser Gly Gly Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Ser Leu Gly Thr Gln Thr Tyr Ile Cys Asn Val Asn His Lys Pro Ser
       210                 215                 220
   Asn Thr Lys Val Asp Lys Lys Val Glu Pro Lys Ser Cys Asp Lys Thr
   225                 230                 235                 240
   His Thr Cys Pro Pro Cys Pro Ala Pro Glu Ala Ala Gly Gly Pro Ser
                   245                 250                 255
   Val Phe Leu Phe Pro Pro Lys Pro Lys Asp Thr Leu Met Ile Ser Arg
               260                 265                 270
   Thr Pro Glu Val Thr Cys Val Val Val Asp Val Ser His Glu Asp Pro
           275                 280                 285
   Glu Val Lys Phe Asn Trp Tyr Val Asp Gly Val Glu Val His Asn Ala
       290                 295                 300
   Lys Thr Lys Pro Arg Glu Glu Gln Tyr Ala Ser Thr Tyr Arg Val Val
   305                 310                 315                 320
   Ser Val Leu Thr Val Leu His Gln Asp Trp Leu Asn Gly Lys Glu Tyr
                   325                 330                 335
   Lys Cys Lys Val Ser Asn Lys Ala Leu Pro Ala Pro Ile Glu Lys Thr
               340                 345                 350
   Ile Ser Lys Ala Lys Gly Gln Pro Arg Glu Pro Gln Val Tyr Thr Leu
           355                 360                 365
   Pro Pro Ser Arg Asp Glu Leu Thr Lys Asn Gln Val Ser Leu Thr Cys
       370                 375                 380
   Leu Val Lys Gly Phe Tyr Pro Ser Asp Ile Ala Val Glu Trp Glu Ser
   385                 390                 395                 400
   Asn Gly Gln Pro Glu Asn Asn Tyr Lys Thr Thr Pro Pro Val Leu Asp
                   405                 410                 415
   Ser Asp Gly Ser Phe Phe Leu Tyr Ser Lys Leu Thr Val Asp Lys Ser
               420                 425                 430
   Arg Trp Gln Gln Gly Asn Val Phe Ser Cys Ser Val Met His Glu Ala
           435                 440                 445
   Leu His Asn His Tyr Thr Gln Lys Ser Leu Ser Leu Ser Pro
       450                 455                 460
<210> 58
   <211> 241
   <212> PRT
   <213> Artificial
<220>
   <223> An artificially synthesized peptide sequence
<400> 58
   Met Asp Trp Thr Trp Arg Phe Leu Phe Val Val Ala Ala Ala Thr Gly
   1               5                   10                  15
   Val Gln Ser Gln Val Gln Leu Val Gln Ser Gly Ala Glu Val Lys Lys
               20                  25                  30
   Pro Gly Ala Ser Val Lys Val Ser Cys Lys Ala Ser Gly Tyr Thr Phe
           35                  40                  45
   Thr Asp Tyr Glu Met His Trp Val Arg Gln Ala Pro Gly Gln Gly Leu
       50                  55                  60
   Glu Trp Met Gly Ala Leu Asp Pro Lys Thr Gly Asp Thr Ala Tyr Ser
   65                  70                  75                  80
   Gln Lys Phe Lys Gly Arg Val Thr Leu Thr Ala Asp Lys Ser Thr Ser
                   85                  90                  95
   Thr Ala Tyr Met Glu Leu Ser Ser Leu Thr Ser Glu Asp Thr Ala Val
               100                 105                 110
   Tyr Tyr Cys Thr Arg Phe Tyr Ser Tyr Thr Tyr Trp Gly Gln Gly Thr
           115                 120                 125
   Leu Val Thr Val Ser Ser Ala Ser Val Ala Ala Pro Ser Val Phe Ile
       130                 135                 140
   Phe Pro Pro Ser Asp Glu Gln Leu Lys Ser Gly Thr Ala Ser Val Val
   145                 150                 155                 160
   Cys Leu Leu Asn Asn Phe Tyr Pro Arg Glu Ala Lys Val Gln Trp Lys
                   165                 170                 175
   Val Asp Asn Ala Leu Gln Ser Gly Asn Ser Gln Glu Ser Val Thr Glu
               180                 185                 190
   Gln Asp Ser Lys Asp Ser Thr Tyr Ser Leu Ser Ser Thr Leu Thr Leu
           195                 200                 205
   Ser Lys Ala Asp Tyr Glu Lys His Lys Val Tyr Ala Cys Glu Val Thr
       210                 215                 220
   His Gln Gly Leu Ser Ser Pro Val Thr Lys Ser Phe Asn Arg Gly Glu
   225                 230                 235                 240
   Cys
<210> 59
   <211> 466
   <212> PRT
   <213> Artificial
<220>
   <223> An artificially synthesized peptide sequence
<400> 59
<210> 60
   <211> 233
   <212> PRT
   <213> Artificial
<220>
   <223> An artificially synthesized peptide sequence
<400> 60
   Met Gly Trp Ser Cys Ile Ile Leu Phe Leu Val Ala Thr Ala Thr Gly
   1               5                   10                  15
   Val His Ser Asp Ile Gln Met Thr Gln Ser Pro Ser Ser Leu Ser Ala
               20                  25                  30
   Ser Val Gly Asp Ser Val Thr Ile Thr Cys Gln Ala Ser Gln Asp Ile
           35                  40                  45
   Ser Ser Tyr Leu Asn Trp Tyr Gln Gln Lys Pro Gly Lys Ala Pro Glu
       50                  55                  60
   Leu Leu Ile Tyr Tyr Gly Ser Glu Leu His Ser Gly Val Pro Ser Arg
   65                  70                  75                  80
   Phe Ser Gly Ser Gly Ser Gly Thr Asp Phe Thr Phe Thr Ile Ser Ser
                   85                  90                  95
   Leu Glu Ala Glu Asp Ala Ala Thr Tyr Tyr Cys Gln Gln Gly Asn Ser
               100                 105                 110
   Leu Pro Tyr Thr Phe Gly Gln Gly Thr Lys Val Glu Ile Glu Arg Thr
           115                 120                 125
   Val Ala Ala Pro Ser Val Phe Ile Phe Pro Pro Ser Asp Glu Gln Leu
       130                 135                 140
   Lys Ser Gly Thr Ala Ser Val Val Cys Leu Leu Asn Asn Phe Tyr Pro
   145                 150                 155                 160
   Arg Glu Ala Lys Val Gln Trp Lys Val Asp Asn Ala Leu Gln Ser Gly
                   165                 170                 175
   Asn Ser Gln Glu Ser Val Thr Glu Gln Asp Ser Lys Asp Ser Thr Tyr
               180                 185                 190
   Ser Leu Ser Ser Thr Leu Thr Leu Ser Lys Ala Asp Tyr Glu Lys His
           195                 200                 205
   Lys Val Tyr Ala Cys Glu Val Thr His Gln Gly Leu Ser Ser Pro Val
       210                 215                 220
   Thr Lys Ser Phe Asn Arg Gly Glu Cys
   225                 230
<210> 61
   <211> 241
   <212> PRT
   <213> Artificial
<220>
   <223> An artificially synthesized peptide sequence
<400> 61
   Met Asp Trp Thr Trp Arg Phe Leu Phe Val Val Ala Ala Ala Thr Gly
   1               5                   10                  15
   Val Gln Ser Gln Val Gln Leu Val Gln Ser Gly Ala Glu Val Lys Lys
               20                  25                  30
   Pro Gly Ala Ser Val Lys Val Ser Cys Lys Ala Ser Gly Tyr Thr Phe
           35                  40                  45
   Thr Asp Tyr Glu Met His Trp Ile Arg Gln Pro Pro Gly Lys Gly Leu
       50                  55                  60
   Glu Trp Ile Gly Ala Ile Asn Pro Lys Thr Gly Asp Thr Ala Tyr Ser
   65                  70                  75                  80
   Gln Lys Phe Lys Gly Arg Val Thr Leu Thr Ala Asp Lys Ser Thr Ser
                   85                  90                  95
   Thr Ala Tyr Met Glu Leu Ser Ser Leu Thr Ser Glu Asp Thr Ala Val
               100                 105                 110
   Tyr Tyr Cys Thr Arg Phe Tyr Ser Tyr Thr Tyr Trp Gly Arg Gly Thr
           115                 120                 125
   Leu Val Thr Val Ser Ser Ala Ser Val Ala Ala Pro Ser Val Phe Ile
       130                 135                 140
   Phe Pro Pro Ser Asp Glu Gln Leu Lys Ser Gly Thr Ala Ser Val Val
   145                 150                 155                 160
   Cys Leu Leu Asn Asn Phe Tyr Pro Arg Glu Ala Lys Val Gln Trp Lys
                   165                 170                 175
   Val Asp Asn Ala Leu Gln Ser Gly Asn Ser Gln Glu Ser Val Thr Glu
               180                 185                 190
   Gln Asp Ser Lys Asp Ser Thr Tyr Ser Leu Ser Ser Thr Leu Thr Leu
           195                 200                 205
   Ser Lys Ala Asp Tyr Glu Lys His Lys Val Tyr Ala Cys Glu Val Thr
       210                 215                 220
   His Gln Gly Leu Ser Ser Pro Val Thr Lys Ser Phe Asn Arg Gly Glu
   225                 230                 235                 240
   Cys
<210> 62
   <211> 466
   <212> PRT
   <213> Artificial
<220>
   <223> An artificially synthesized peptide sequence
<400> 62
   Met Gly Trp Ser Cys Ile Ile Leu Phe Leu Val Ala Thr Ala Thr Gly
   1               5                   10                  15
   Val His Ser Gln Val Gln Leu Gln Glu Ser Gly Pro Gly Leu Val Lys
               20                  25                  30
   Pro Ser Glu Thr Leu Ser Leu Thr Cys Ala Val Ser Gly Tyr Ser Ile
           35                  40                  45
   Ser Asp Asp Gln Ala Trp Ser Trp Val Arg Gln Pro Pro Gly Glu Gly
   50 55 60
   Leu Glu Trp Ile Gly Tyr Ile Ser Tyr Ser Gly Ile Thr Asn Tyr Asn
   65 70 75 80
   Pro Ser Leu Lys Gly Arg Val Thr Ile Ser Arg Asp Thr Ser Lys Asn
   85 90 95
   Gln Phe Ser Leu Lys Leu Ser Ser Val Thr Ala Ala Asp Thr Ala Ala
   100 105 110
   Tyr Tyr Cys Ala Arg Ser Leu Ala Arg Thr Thr Ala Met Asp Tyr Trp
   115 120 125
   Gly Glu Gly Thr Leu Val Thr Val Ser Ser Ala Ser Thr Lys Gly Pro
   130 135 140
   Ser Val Phe Pro Leu Ala Pro Ser Ser Lys Ser Thr Ser Gly Gly Thr
   145 150 155 160
   Ala Ala Leu Gly Cys Leu Val Lys Asp Tyr Phe Pro Glu Pro Val Thr
   165 170 175
   Val Ser Trp Asn Ser Gly Ala Leu Thr Ser Gly Val His Thr Phe Pro
   180 185 190
   Ala Val Leu Gln Ser Ser Gly Leu Tyr Ser Leu Ser Ser Val Val Thr
   195 200 205
   Val Pro Ser Ser Ser Leu Gly Thr Gln Thr Tyr Ile Cys Asn Val Asn
   210 215 220
   His Lys Pro Ser Asn Thr Lys Val Asp Lys Lys Val Glu Pro Lys Ser
   225 230 235 240
   Cys Asp Lys Thr His Thr Cys Pro Pro Cys Pro Ala Pro Glu Ala Ala
   245 250 255
   Gly Gly Pro Ser Val Phe Leu Phe Pro Pro Lys Pro Lys Asp Thr Leu
   260 265 270
   Met Ile Ser Arg Thr Pro Glu Val Thr Cys Val Val Val Asp Val Ser
   275 280 285
   His Glu Asp Pro Glu Val Lys Phe Asn Trp Tyr Val Asp Gly Val Glu
   290 295 300
   Val His Asn Ala Lys Thr Lys Pro Arg Glu Glu Gln Tyr Ala Ser Thr
   305 310 315 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Arg Asp Glu Leu Thr Lys Asn Gln Val
       370                 375                 380
   Ser Leu Thr Cys Leu Val Lys Gly Phe Tyr Pro Ser Asp Ile Ala Val
   385                 390                 395                 400
   Glu Trp Glu Ser Asn Gly Gln Pro Glu Asn Asn Tyr Lys Thr Thr Pro
                   405                 410                 415
   Pro Val Leu Asp Ser Asp Gly Ser Phe Phe Leu Tyr Ser Lys Leu Thr
               420                 425                 430
   Val Asp Lys Ser Arg Trp Gln Gln Gly Asn Val Phe Ser Cys Ser Val
           435                 440                 445
   Met His Glu Ala Leu His Asn His Tyr Thr Gln Glu Ser Leu Ser Leu
       450                 455                 460
   Ser Pro
   465
<210> 63
   <211> 462
   <212> PRT
   <213> Artificial
<220>
   <223> An artificially synthesized peptide sequence
<400> 63
<210> 64
   <211> 466
   <212> PRT
   <213> Artificial
<220>
   <223> An artificially synthesized peptide sequence
<400> 64
   Met Gly Trp Ser Cys Ile Ile Leu Phe Leu Val Ala Thr Ala Thr Gly
   1               5                   10                  15
   Val His Ser Gln Val Gln Leu Gln Glu Ser Gly Pro Gly Leu Val Lys
               20                  25                  30
   Pro Ser Glu Thr Leu Ser Leu Thr Cys Ala Val Ser Gly Tyr Ser Ile
           35                  40                  45
   Ser Asp Asp Gln Ala Trp Ser Trp Val Arg Lys Pro Pro Gly Glu Gly
       50                  55                  60
   Leu Glu Trp Ile Gly Tyr Ile Ser Tyr Ser Gly Ile Thr Asn Tyr Asn
   65                  70                  75                  80
   Pro Ser Leu Lys Gly Arg Val Thr Ile Ser Arg Asp Thr Ser Lys Asn
                   85                  90                  95
   Gln Phe Ser Leu Lys Leu Ser Ser Val Thr Ala Ala Asp Thr Ala Ala
               100                 105                 110
   Tyr Tyr Cys Ala Arg Ser Leu Ala Arg Thr Thr Ala Met Asp Tyr Trp
           115                 120                 125
   Gly Glu Gly Thr Leu Val Thr Val Ser Ser Ala Ser Thr Lys Gly Pro
       130                 135                 140
   Ser Val Phe Pro Leu Ala Pro Ser Ser Lys Ser Thr Ser Gly Gly Thr
   145                 150                 155                 160
   Ala Ala Leu Gly Cys Leu Val Lys Asp Tyr Phe Pro Glu Pro Val Thr
                   165                 170                 175
   Val Ser Trp Asn Ser Gly Ala Leu Thr Ser Gly Val His Thr Phe Pro
               180                 185                 190
   Ala Val Leu Gln Ser Ser Gly Leu Tyr Ser Leu Ser Ser Val Val Thr
           195                 200                 205
   Val Pro Ser Ser Ser Leu Gly Thr Gln Thr Tyr Ile Cys Asn Val Asn
       210                 215                 220
   His Lys Pro Ser Asn Thr Lys Val Asp Lys Lys Val Glu Pro Lys Ser
   225                 230                 235                 240
   Cys Asp Lys Thr His Thr Cys Pro Pro Cys Pro Ala Pro Glu Ala Ala
                   245                 250                 255
   Gly Gly Pro Ser Val Phe Leu Phe Pro Pro Lys Pro Lys Asp Thr Leu
               260                 265                 270
   Met Ile Ser Arg Thr Pro Glu Val Thr Cys Val Val Val Asp Val Ser
           275                 280                 285
   His Glu Asp Pro Glu Val Lys Phe Asn Trp Tyr Val Asp Gly Val Glu
       290                 295                 300
   Val His Asn Ala Lys Thr Lys Pro Arg Glu Glu Gln Tyr Ala Ser Thr
   305                 310                 315                 320
   Tyr Arg Val Val Ser Val Leu Thr Val Leu His Gln Asp Trp Leu Asn
                   325                 330                 335
   Gly Lys Glu Tyr Lys Cys Lys Val Ser Asn Lys Ala Leu Pro Ala Pro
               340                 345                 350
   Ile Glu Lys Thr Ile Ser Lys Ala Lys Gly Gln Pro Arg Glu Pro Gln
           355                 360                 365
   Val Tyr Thr Leu Pro Pro Ser Arg Asp Glu Leu Thr Lys Asn Gln Val
       370                 375                 380
   Ser Leu Thr Cys Leu Val Lys Gly Phe Tyr Pro Ser Asp Ile Ala Val
   385                 390                 395                 400
   Glu Trp Glu Ser Asn Gly Gln Pro Glu Asn Asn Tyr Lys Thr Thr Pro
                   405                 410                 415
   Pro Val Leu Asp Ser Asp Gly Ser Phe Phe Leu Tyr Ser Lys Leu Thr
               420                 425                 430
   Val Asp Lys Ser Arg Trp Gln Gln Gly Asn Val Phe Ser Cys Ser Val
           435                 440                 445
   Met His Glu Ala Leu His Asn His Tyr Thr Gln Glu Ser Leu Ser Leu
       450                 455                 460
   Ser Pro
   465
<210> 65
   <211> 233
   <212> PRT
   <213> Artificial
<220>
   <223> An artificially synthesized peptide sequence
<400> 65
   Met Gly Trp Ser Cys Ile Ile Leu Phe Leu Val Ala Thr Ala Thr Gly
   1               5                   10                  15
   Val His Ser Asp Ile Gln Met Thr Gln Ser Pro Ser Ser Leu Ser Ala
               20                  25                  30
   Ser Val Gly Asp Ser Val Thr Ile Thr Cys Gln Ala Ser Gln Asp Ile
           35                  40                  45
   Ser Ser Tyr Leu Asn Trp Tyr Gln Glu Lys Pro Gly Lys Ala Pro Glu
       50                  55                  60
   Leu Leu Ile Tyr Tyr Gly Ser Glu Leu His Ser Gly Val Pro Ser Arg
   65                  70                  75                  80
   Phe Ser Gly Ser Gly Ser Gly Thr Asp Phe Thr Phe Thr Ile Ser Ser
                   85                  90                  95
   Leu Glu Ala Glu Asp Ala Ala Thr Tyr Tyr Cys Gln Gln Gly Asn Ser
               100                 105                 110
   Leu Pro Tyr Thr Phe Gly Gln Gly Thr Lys Val Glu Ile Glu Arg Thr
           115                 120                 125
   Val Ala Ala Pro Ser Val Phe Ile Phe Pro Pro Ser Asp Glu Gln Leu
       130                 135                 140
   Lys Ser Gly Thr Ala Ser Val Val Cys Leu Leu Asn Asn Phe Tyr Pro
   145                 150                 155                 160
   Arg Glu Ala Lys Val Gln Trp Lys Val Asp Asn Ala Leu Gln Ser Gly
                   165                 170                 175
   Asn Ser Gln Glu Ser Val Thr Glu Gln Asp Ser Lys Asp Ser Thr Tyr
               180                 185                 190
   Ser Leu Ser Ser Thr Leu Thr Leu Ser Lys Ala Asp Tyr Glu Lys His
           195                 200                 205
   Lys Val Tyr Ala Cys Glu Val Thr His Gln Gly Leu Ser Ser Pro Val
       210                 215                 220
   Thr Lys Ser Phe Asn Arg Gly Glu Cys
   225                 230
<210> 66
   <211> 462
   <212> PRT
   <213> Artificial
<220>
   <223> An artificially synthesized peptide sequence
<400> 66
   Met Arg Leu Pro Ala Gln Leu Leu Gly Leu Leu Met Leu Trp Val Ser
   1               5                   10                  15
   Gly Ser Ser Gly Asp Val Val Met Thr Gln Ser Pro Leu Ser Leu Pro
               20                  25                  30
   Val Thr Pro Gly Glu Pro Ala Ser Ile Ser Cys Arg Ser Ser Gln Ser
           35                  40                  45
   Leu Val His Ser Asn Arg Asn Thr Tyr Leu His Trp Tyr Leu Glu Lys
       50                  55                  60
   Pro Gly Gln Ser Pro Gln Leu Leu Ile Tyr Lys Val Ser Asn Arg Phe
   65                  70                  75                  80
   Ser Gly Val Pro Asp Arg Phe Ser Gly Ser Gly Ser Gly Thr Asp Phe
                   85                  90                  95
   Thr Leu Lys Ile Ser Arg Val Glu Ala Glu Asp Val Gly Val Tyr Tyr
               100                 105                 110
   Cys Ser Gln Asn Thr His Val Pro Pro Thr Phe Gly Gln Gly Thr Lys
           115                 120                 125
   Leu Glu Ile Lys Ser Ser Ala Ser Thr Lys Gly Pro Ser Val Phe Pro
       130                 135                 140
   Leu Ala Pro Ser Ser Lys Ser Thr Ser Gly Gly Thr Ala Ala Leu Gly
   145                 150                 155                 160
   Cys Leu Val Lys Asp Tyr Phe Pro Glu Pro Val Thr Val Ser Trp Asn
                   165                 170                 175
   Ser Gly Ala Leu Thr Ser Gly Val His Thr Phe Pro Ala Val Leu Gln
               180                 185                 190
   Ser Ser Gly Leu Tyr Ser Leu Ser Ser Val Val Thr Val Pro Ser Ser
           195                 200                 205
   Ser Leu Gly Thr Gln Thr Tyr Ile Cys Asn Val Asn His Lys Pro Ser
       210                 215                 220
   Asn Thr Lys Val Asp Lys Lys Val Glu Pro Lys Ser Cys Asp Lys Thr
   225                 230                 235                 240
   His Thr Cys Pro Pro Cys Pro Ala Pro Glu Ala Ala Gly Gly Pro Ser
                   245                 250                 255
   Val Phe Leu Phe Pro Pro Lys Pro Lys Asp Thr Leu Met Ile Ser Arg
               260                 265                 270
   Thr Pro Glu Val Thr Cys Val Val Val Asp Val Ser His Glu Asp Pro
           275                 280                 285
   Glu Val Lys Phe Asn Trp Tyr Val Asp Gly Val Glu Val His Asn Ala
       290                 295                 300
   Lys Thr Lys Pro Arg Glu Glu Gln Tyr Ala Ser Thr Tyr Arg Val Val
   305 310 315 320
   Ser Val Leu Thr Val Leu His Gln Asp Trp Leu Asn Gly Lys Glu Tyr
   325 330 335
   Lys Cys Lys Val Ser Asn Lys Ala Leu Pro Ala Pro Ile Glu Lys Thr
   340 345 350
   Ile Ser Lys Ala Lys Gly Gln Pro Arg Glu Pro Gln Val Tyr Thr Leu
   355 360 365
   Pro Pro Ser Arg Lys Glu Leu Thr Lys Asn Gln Val Ser Leu Thr Cys
   370 375 380
   Leu Val Lys Gly Phe Tyr Pro Ser Asp Ile Ala Val Glu Trp Glu Ser
   385 390 395 400
   Asn Gly Gln Pro Glu Asn Asn Tyr Lys Thr Thr Pro Pro Val Leu Asp
   405 410 415
   Ser Asp Gly Ser Phe Phe Leu Tyr Ser Lys Leu Thr Val Asp Lys Ser
   420 425 430
   Arg Trp Gln Gln Gly Asn Val Phe Ser Cys Ser Val Met His Glu Ala
   435 440 445
   Leu His Asn Arg Tyr Thr Gln Lys Ser Leu Ser Leu Ser Pro
   450 455 460
<210> 67
   <211> 241
   <212> PRT
   <213> Artificial
<220>
   <223> An artificially synthesized peptide sequence
<400> 67

## Description

### Technical Field

The present invention relates to methods for producing or purifying polypeptide multimers, polypeptide multimers with an altered protein A-binding ability, and such.

### Background Art

There are some previously reported methods for producing an IgG-type bispecific antibody having a human constant region (IgG-type antibody which has a human constant region and in which one of the arms has a specific binding activity to antigen A and the other has a specific binding activity to antigen B). In general, an IgG-type bispecific antibody is composed of two types of H chains (i.e., H chain against antigen A and H chain against antigen B) and two types of L chains (i.e., L chain against antigen A and L chain against antigen B). When such an IgG-type bispecific antibody is expressed, two types of H chains and two types of L chains are expressed, and there are ten possible combinations for the H2L2 combination. Of these, only one combination has the specificity of interest (one arm has binding activity specific to antigen A and the other has binding activity specific to antigen B). Thus, to obtain a bispecific antibody of interest, it is necessary to purify a single antibody of interest from the ten types of antibodies. This is an extremely inefficient and difficult process.

There are reported methods for solving this problem which use a common L chain so that the L chain against antigen A and the L chain against antigen B have an identical amino acid sequence (Patent Documents 1 and 2). When an IgG-type bispecific antibody having such a common L chain is expressed, two types of H chains and one type of common L chain are expressed, and there are three possible combinations for the H2L2 combination. One of these combinations is a bispecific antibody of interest. These three combinations are: monospecific antibody against antigen A (homomeric H chain antibody against antigen A), bispecific antibody against both antigen A and antigen B (heteromeric antibody with an H chain against antigen A and an H chain against antigen B), and monospecific antibody against antigen B (homomeric H chain antibody against antigen B). Since their ratio is in general 1:2:1, the expression efficiency of the desired bispecific antibody is about 50%. A method for further improving this efficiency has been reported which allows two types of H chains heteromerically associate (Patent Document 3). This can increase the expression efficiency of the desired bispecific antibody up to about 90-95%. Meanwhile, a method has been reported for efficiently removing the two types of homomeric antibodies which are impurities, in which amino acid substitutions are introduced into the variable regions of the two types of H chains to give them different isoelectric points so that the two types of homomeric antibodies and the bispecific antibody of interest (heteromeric antibody) can be purified by ion exchange chromatography (Patent Document 4). A combination of the above-mentioned methods has made it possible to efficiently produce a bispecific antibody (heteromeric antibody) having an IgG-type human constant region.

On the other hand, in the industrial production of IgG-type antibodies, a purification step by protein A chromatography must be used, but ion exchange chromatography is not necessarily used in the purification step. Therefore, the use of ion exchange chromatography for producing a highly pure bispecific antibody leads to an increase of production costs. In addition, since ion exchange chromatography alone may not ensure a robust purification method for pharmaceuticals, it is preferable to perform more than one chromatographic step to remove impurities.

In any case, it is preferable that bispecific antibodies can also be highly purified by a chromatographic step that has a separation mode different from that of ion exchange chromatography. It is desirable that as one of such separation modes, protein A chromatography, which must be used in the industrial production of IgG-type antibodies, could purify bispecific antibodies to high purity.

A previously reported method for purifying a bispecific antibody (heteromeric antibody) using protein A is to use a bispecific antibody having a mouse IgG2a H chain that binds to protein A and a rat IgG2b H chain that does not bind to protein A. It has been reported that this method allows a bispecific antibody of interest to be purified to a purity of 95% by the protein A-based purification step alone (Non-patent Document 1 and Patent Document 5). However, this method also uses ion exchange chromatography to improve the purity of the bispecific antibody. In other words, purification of a highly pure bispecific antibody cannot be achieved by the purification step using protein A chromatography alone. Moreover, catumaxomab, a bispecific antibody produced by the above-described method and having a mouse IgG2a H chain and a rat IgG2b H chain, has a half-life of about 2.1 days in human, which is extremely shorter than that of normal human IgG1 (2 to 3 weeks) (Non-patent Document 2). In addition to having a short half-life, catumaxomab is highly immunogenic because of its mouse and rat constant regions (Non-patent Document 3). Thus, a bispecific antibody obtained by such methods is considered inappropriate as a pharmaceutical.

On the other hand, it has been suggested that from the viewpoint of immunogenicity, a human IgG3 constant region may be used as a protein A-nonbinding constant region (Non-patent Document 1). However, as it is known that the H chains of human IgG1 and human IgG3 hardly associate with each other (Non-patent Document 1), it is impossible to produce a bispecific antibody of interest using a human IgG1 H chain and a human IgG3 H chain by the same method used for the bispecific antibody having a mouse IgG2a H chain and a rat IgG2b H chain. Furthermore, the half-life of human IgG3 in human has been reported to be generally shorter than that of human IgG1, human IgG2, and human IgG4 (Non-patent Documents 4 and 5). Accordingly, like the bispecific antibody using a mouse IgG2a and a rat IgG2b, a bispecific antibody using human IgG3 might also have a short half-life in human. The reason that H chain association rarely occurs between human IgG1 and human IgG3 is suggested to be the hinge sequence of human IgG3 (Non-patent Document 1). Meanwhile, the reason for the short half-life of the human IgG3 constant region has not been fully elucidated yet. Thus, there has been no report so far with regard to bispecific antibodies that use a human IgG3 constant region as a protein A-nonbinding constant region. Moreover, there is also no report regarding methods for efficiently producing or purifying highly pure bispecific antibodies that have a human constant region and show a similarly long half-life as human IgG1.
Furthermore Patent Document 6 e.g. discloses heterodimeric bispecific antigen-binding proteins, Patent Document 7 e.g. discloses methods for making heterologous bi-specific antibodies, Patent Document 8 e.g. discloses methods for producing a polypeptide comprising a mutation in an amino acid residue forming a polypeptide interface such that polypeptide association will be regulated, and Non-Patent Document 6 e.g. postulates that all IgG proteins that bind protein A have His at position 435, whereas those that do not, have Arg at that position.

### Prior Art Documents

### Patent Documents

Patent Document 1: WO98050431
Patent Document 2: WO2006109592
Patent Document 3: WO2006106905
Patent Document 4: WO2007114325
Patent Document 5: WO95033844
Patent Document 6: WO2010151792
Patent Document 7: US5945311
Patent Document 8: EP1870459

### Non-patent Documents

Non-patent Document 1: The Journal of Immunology, 1995, 155:219-225
Non-patent Document 2: J Clin Oncol 26: 2008 (May 20 suppl; abstr 14006)
Non-patent Document 3: Clin Cancer Res 2007 13:3899-3905
Non-patent Document 4: Nat Biotechnol. 2007 Dec; 25(12):1369-72
Non-patent Document 5: J. Clin Invest 1970; 49:673-80
Non-patent Document 6: Scandinavian Journal of Immunology 1982; 15(3):275-78

### Disclosure of the Invention

### [Problems to be Solved by the Invention]

In general, an ordinary IgG-type antibody can be efficiently produced as a highly pure IgG through a protein A-based purification step. However, the production of a highly pure bispecific antibody requires an additional purification step using ion exchange chromatography. The addition of such a purification step by ion exchange chromatography can complicate the production and increase production cost. Thus, it is preferable to produce a highly pure bispecific antibody by a protein A-based purification step alone. An objective of the present invention is to provide methods that use only a protein A-based purification step for efficiently producing or purifying a highly pure IgG-type bispecific antibody having a human antibody heavy chain constant region.

Meanwhile, since the protein A binding site in the Fc domain is identical to the FcRn-binding site in the Fc domain, it is expected to be difficult to adjust the protein A-binding activity while retaining the binding to human FcRn. Retaining the human FcRn-binding ability is very important for the long plasma retention (long half-life) in human which is characteristic of IgG-type antibodies. The present invention provides methods that use only a protein A-based purification step to efficiently produce or purify a highly pure bispecific antibody that maintains a plasma retention time comparable to or longer than that of human IgG1.

### [Means for Solving the Problems]

The present inventors discovered methods that use only a protein A-based purification step for efficiently purifying or producing a highly pure polypeptide multimer capable of binding to two or more antigens, in particular, a multispecific IgG-type antibody having a human constant region, by altering its protein A-binding ability.

Furthermore, these methods were combined with methods for regulating the association between a first polypeptide having an antigen-binding activity and a second polypeptide having an antigen-binding activity by modifying amino acids that constitute the interface formed upon association of the polypeptides. By this combination, the present invention enables efficient production or purification of a highly pure polypeptide multimer of interest.

The present inventors also discovered that by modifying the amino acid residue at position 435 (EU numbering) in the heavy chain constant region, the protein A-binding ability could be adjusted while keeping its plasma retention comparable to or longer than that of human IgG1. Based on this finding, a highly pure bispecific antibody with plasma retention time comparable to or longer than that of human IgG1 can be produced or purified.

The present invention is based on the findings described above, and provides the subject-matter as defined in the claims.

Specifically, the present invention provides [1] to [5] below:
[1] A method for producing a polypeptide multimer that comprises a first polypeptide having an antigen-binding activity and a second polypeptide having an antigen-binding activity or no antigen-binding activity, which comprises the steps of:
   (a) expressing a DNA that encodes the first polypeptide having an antigen-binding activity and a DNA that encodes the second polypeptide having an antigen-binding activity or no antigen-binding activity; and
   (b) collecting the expression product of step (a) by protein A affinity chromatography, wherein the first polypeptide having an antigen-binding activity and the second polypeptide having an antigen-binding activity or no antigen-binding activity comprise an amino acid sequence of an antibody Fc domain or an amino acid sequence of an antibody heavy-chain constant region;
   wherein the antibody Fc domain or antibody heavy-chain constant region is derived from human IgG;
   wherein the amino acid residue at position 435 (EU numbering) in the amino acid sequence of the Fc domain or heavy-chain constant region is histidine or arginine in the first polypeptide having an antigen-binding activity and arginine or histidine respectively in the second polypeptide having or not having an antigen-binding activity;
   wherein the amino acid combination at positions 356 and 439 in the amino acid sequence of the Fc region or heavy chain constant region of the first polypeptide having an antigen-binding activity have been modified to amino acids with the same electric charge; and
   wherein the amino acid combination at positions 356 and 439 in the amino acid sequence of the Fc region or heavy chain constant region of the second polypeptide having or not having an antigen binding activity have been modified to amino acids with an electric charge opposite to that of the first polypeptide having an antigen binding activity.
[2] The method of [1],
   i) wherein the purity of the collected polypeptide multimer is 95% or more;
      and/or
   ii) wherein the first polypeptide having an antigen-binding activity and the second polypeptide having an antigen-binding activity comprise an amino acid sequence of an antibody heavy-chain variable region,
      particularly wherein at least one amino acid residue has been modified in the amino acid sequences of FR1, CDR2, and FR3 of the antibody heavy-chain variable region; and/or
   iii) wherein the polypeptide multimer comprises one or two third polypeptides having an antigen-binding activity, and step (a) comprises expressing a DNA that encodes the third polypeptide having an antigen-binding activity,
      particularly
      a) wherein the third polypeptide having an antigen-binding activity comprises an amino acid sequence of an antibody light chain,
         and/or
      b) wherein the polypeptide multimer additionally comprises a fourth polypeptide having an antigen-binding activity, and step (a) comprises expressing a DNA that encodes the fourth polypeptide having an antigen-binding activity,
         especially
         b1) wherein at least one of the third and fourth polypeptides having an antigen-binding activity comprises an amino acid sequence of an antibody light chain,
            or
         b2) wherein the first polypeptide having an antigen-binding activity comprises amino acid sequences of an antibody light-chain variable region and an antibody heavy-chain constant region; the second polypeptide having an antigen-binding activity comprises an amino acid sequence of an antibody heavy chain; the third polypeptide having an antigen-binding activity comprises amino acid sequences of an antibody heavy-chain variable region and an antibody light-chain constant region; and the fourth polypeptide having an antigen-binding activity comprises an amino acid sequence of an antibody light chain;
         and/or
   iv) wherein the polypeptide multimer is a multispecific antibody,
      particularly wherein the multispecific antibody is a bispecific antibody.
[3] The method of any one of [1] and [2] i) to [2] iv), which comprises the first polypeptide having an antigen-binding activity and the second polypeptide having no antigen-binding activity, and wherein the first polypeptide having an antigen-binding activity comprises an amino acid sequence of an antigen-binding domain of a receptor and an amino acid sequence of an antibody Fc domain, and the second polypeptide having no antigen-binding activity comprises an amino acid sequence of an antibody Fc domain.
[4] A method for purifying a polypeptide multimer that comprises a first polypeptide having an antigen-binding activity and a second polypeptide having an antigen-binding activity or no antigen-binding activity, which comprises the steps of:
   (a) expressing a DNA that encodes the first polypeptide having an antigen-binding activity and a DNA that encodes the second polypeptide having an antigen-binding activity or no antigen-binding activity; and
   (b) collecting the expression product of step (a) by protein A affinity chromatography, wherein the first polypeptide having an antigen-binding activity and the second polypeptide having an antigen-binding activity or no antigen-binding activity comprise an amino acid sequence of an antibody Fc domain or an amino acid sequence of an antibody heavy-chain constant region;
   wherein the antibody Fc domain or antibody heavy-chain constant region is derived from human IgG;
   wherein the amino acid residue at position 435 (EU numbering) in the amino acid sequence of the Fc domain or heavy-chain constant region is histidine or arginine in the first polypeptide having an antigen-binding activity and arginine or histidine respectively in the second polypeptide having or not having an antigen-binding activity;
   wherein the amino acid combination at positions 356 and 439 in the amino acid sequence of the Fc region or heavy chain constant region of the first polypeptide having an antigen-binding activity have been modified to amino acids with the same electric charge; and
   wherein the amino acid combination at positions 356 and 439 in the amino acid sequence of the Fc region or heavy chain constant region of the second polypeptide having or not having an antigen binding activity have been modified to amino acids with an electric charge opposite to that of the first polypeptide having an antigen binding activity.
[5] The method of [4],
   i) wherein the purity of the collected polypeptide multimer is 95% or more;
      and/or
   ii) wherein the first polypeptide having an antigen-binding activity and the second polypeptide having an antigen-binding activity comprise an amino acid sequence of an antibody heavy-chain variable region,
      particularly wherein at least one amino acid residue has been modified in the amino acid sequences of FR1, CDR2, and FR3 of the antibody heavy-chain variable region; and/or
   iii) wherein the polypeptide multimer comprises one or two third polypeptides having an antigen-binding activity, and step (a) comprises expressing a DNA that encodes the third polypeptide having an antigen-binding activity,
      particularly
      a) wherein the third polypeptide having an antigen-binding activity comprises an amino acid sequence of an antibody light chain,
         and/or
      b) wherein the polypeptide multimer additionally comprises a fourth polypeptide having an antigen-binding activity, and step (a) comprises expressing a DNA that encodes the fourth polypeptide having an antigen-binding activity,
         especially
         b1) wherein at least one of the third and fourth polypeptides having an antigen-binding activity comprises an amino acid sequence of an antibody light chain,
            or
         b2) wherein the first polypeptide having an antigen-binding activity comprises amino acid sequences of an antibody light-chain variable region and an antibody heavy-chain constant region; the second polypeptide having an antigen-binding activity comprises an amino acid sequence of an antibody heavy chain; the third polypeptide having an antigen-binding activity comprises amino acid sequences of an antibody heavy-chain variable region and an antibody light-chain constant region; and the fourth polypeptide having an antigen-binding activity comprises an amino acid sequence of an antibody light chain;
         and/or
   iv) wherein the polypeptide multimer is a multispecific antibody,
      particularly wherein the multispecific antibody is a bispecific antibody.

Further disclosed herein are the following items [1] to [55]:
[1] A method for producing a polypeptide multimer that comprises a first polypeptide having an antigen-binding activity and a second polypeptide having an antigen-binding activity or no antigen-binding activity, which comprises the steps of:
   (a) expressing a DNA that encodes the first polypeptide having an antigen-binding activity and a DNA that encodes the second polypeptide having an antigen-binding activity or no antigen-binding activity; and
   (b) collecting the expression product of step (a),
   wherein one or more amino acid residues in either or both of the first polypeptide having an antigen-binding activity and the second polypeptide having an antigen-binding activity or no antigen-binding activity have been modified, so that there is a larger difference of protein A-binding ability between the first polypeptide having an antigen-binding activity and the second polypeptide having an antigen-binding activity or no antigen-binding activity.
[2] The method of [1], wherein the expression product is collected using protein A affinity chromatography in step (b).
[3] The method of [1] or [2], wherein one or more amino acid residues in either or both of the first polypeptide having an antigen-binding activity and the second polypeptide having an antigen-binding activity or no antigen-binding activity have been modified, so that there is a larger difference between the solvent pH for eluting the first polypeptide having an antigen-binding activity from protein A and that for eluting the second polypeptide having an antigen-binding activity or no antigen-binding activity from protein A.
[4] The method of any one of [1] to [3], wherein one or more amino acid residues in the first polypeptide having an antigen-binding activity or the second polypeptide having an antigen-binding activity or no antigen-binding activity have been modified, so as to increase or reduce the protein A-binding ability of either one of the first polypeptide having an antigen-binding activity and the second polypeptide having an antigen-binding activity or no antigen-binding activity.
[5] The method of any one of [1] to [4], wherein one or more amino acid residues in the first polypeptide having an antigen-binding activity and the second polypeptide having an antigen-binding activity or no antigen-binding activity have been modified, so as to increase the protein A-binding ability of either one of the first polypeptide having an antigen-binding activity and the second polypeptide having an antigen-binding activity or no antigen-binding activity, and reduce the protein A-binding ability of the other polypeptide.
[6] The method of any one of [1] to [5], wherein the purity of the collected polypeptide multimer is 95% or more.
[7] The method of any one of [1] to [6], wherein the first polypeptide having an antigen-binding activity and the second polypeptide having an antigen-binding activity or no antigen-binding activity comprise an amino acid sequence of an antibody Fc domain or an amino acid sequence of an antibody heavy-chain constant region.
[8] The method of [7], wherein at least one amino acid residue selected from the amino acid residues of positions 250 to 255, 308 to 317, and 430 to 436 (EU numbering) in the amino acid sequence of the antibody Fc domain or antibody heavy-chain constant region has been modified.
[9] The method of any one of [1] to [8], wherein the first polypeptide having an antigen-binding activity and the second polypeptide having an antigen-binding activity comprise an amino acid sequence of an antibody heavy-chain variable region.
[10] The method of [9], wherein at least one amino acid residue has been modified in the amino acid sequences of FR1, CDR2, and FR3 of the antibody heavy-chain variable region.
[11] The method of any one of [1] to [10], wherein the polypeptide multimer comprises one or two third polypeptides having an antigen-binding activity, and step (a) comprises expressing a DNA that encodes the third polypeptide having an antigen-binding activity.
[12] The method of [11], wherein the third polypeptide having an antigen-binding activity comprises an amino acid sequence of an antibody light chain.
[13] The method of [11] or [12], wherein the polypeptide multimer additionally comprises a fourth polypeptide having an antigen-binding activity, and step (a) comprises expressing a DNA that encodes the fourth polypeptide having an antigen-binding activity.
[14] The method of [13], wherein at least one of the third and fourth polypeptides having an antigen-binding activity comprises an amino acid sequence of an antibody light chain.
[15] The method of [13], wherein the first polypeptide having an antigen-binding activity comprises amino acid sequences of an antibody light-chain variable region and an antibody heavy-chain constant region; the second polypeptide having an antigen-binding activity comprises an amino acid sequence of an antibody heavy chain; the third polypeptide having an antigen-binding activity comprises amino acid sequences of an antibody heavy-chain variable region and an antibody light-chain constant region; and the fourth polypeptide having an antigen-binding activity comprises an amino acid sequence of an antibody light chain.
[16] The method of any one of [1] to [15], wherein the polypeptide multimer is a multispecific antibody.
[17] The method of [16], wherein the multispecific antibody is a bispecific antibody.
[18] The method of any one of [1] to [8], which comprises the first polypeptide having an antigen-binding activity and the second polypeptide having no antigen-binding activity, and wherein the first polypeptide having an antigen-binding activity comprises an amino acid sequence of an antigen-binding domain of a receptor and an amino acid sequence of an antibody Fc domain, and the second polypeptide having no antigen-binding activity comprises an amino acid sequence of an antibody Fc domain.
[19] The method of any one of [7] to [18], wherein the antibody Fc domain or antibody heavy-chain constant region is derived from human IgG.
[20] A polypeptide multimer produced by the method of any one of [1] to [19].
[21] A method for purifying a polypeptide multimer that comprises a first polypeptide having an antigen-binding activity and a second polypeptide having an antigen-binding activity or no antigen-binding activity, which comprises the steps of:
   (a) expressing a DNA that encodes the first polypeptide having an antigen-binding activity and a DNA that encodes the second polypeptide having an antigen-binding activity or no antigen-binding activity; and
   (b) collecting the expression product of step (a) by protein A affinity chromatography,
   wherein one or more amino acid residues in either or both of the first polypeptide having an antigen-binding activity and the second polypeptide having an antigen-binding activity or no antigen-binding activity have been modified, so that there is a larger difference of protein A-binding ability between the first polypeptide having an antigen-binding activity and the second polypeptide having an antigen-binding activity or no antigen-binding activity.
[22] The method of [21], wherein one or more amino acid residues in the first polypeptide having an antigen-binding activity or the second polypeptide having an antigen-binding activity or no antigen-binding activity have been modified, so as to increase or reduce the protein A-binding ability of the first polypeptide having an antigen-binding activity or the second polypeptide having an antigen-binding activity or no antigen-binding activity.
[23] The method of [20] or [21], wherein one or more amino acid residues in the first polypeptide having an antigen-binding activity and the second polypeptide having an antigen-binding activity or no antigen-binding activity have been modified, so as to increase the protein A-binding ability of either one of the first polypeptide having an antigen-binding activity and the second polypeptide having an antigen-binding activity or no antigen-binding activity, and reduce the protein A-binding ability of the other polypeptide.
[24] The method of any one of [21] to [23], wherein the purity of the collected polypeptide multimer is 95% or more.
[25] The method of any one of [21] to [24], wherein the first polypeptide having an antigen-binding activity and the second polypeptide having an antigen-binding activity or no antigen-binding activity comprise an amino acid sequence of an antibody Fc domain or an amino acid sequence of an antibody heavy-chain constant region.
[26] The method of [25], wherein at least one amino acid residue selected from the amino acid residues of positions 250 to 255, 308 to 317, and 430 to 436 (EU numbering) in the amino acid sequence of the antibody Fc domain or antibody heavy-chain constant region has been modified.
[27] The method of any one of [21] to [26], wherein the first polypeptide having an antigen-binding activity and the second polypeptide having an antigen-binding activity comprise an amino acid sequence of an antibody heavy-chain variable region.
[28] The method of [27], wherein at least one amino acid residue has been modified in the amino acid sequences of FR1, CDR2, and FR3 of the antibody heavy-chain variable region.
[29] The method of any one of [21] to [28], wherein the polypeptide multimer comprises one or two third polypeptides having an antigen-binding activity, and step (a) comprises expressing a DNA that encodes the third polypeptide having an antigen-binding activity.
[30] The method of [29], wherein the third polypeptide having an antigen-binding activity comprises an amino acid sequence of an antibody light chain.
[31] The method of [29] or [30], wherein the polypeptide multimer additionally comprises a fourth polypeptide having an antigen-binding activity, and step (a) comprises expressing a DNA that encodes the fourth polypeptide having an antigen-binding activity.
[32] The method of [31], wherein at least one of the third and fourth polypeptides having an antigen-binding activity comprises an amino acid sequence of an antibody light chain.
[33] The method of [31], wherein the first polypeptide having an antigen-binding activity comprises amino acid sequences of an antibody light-chain variable region and an antibody heavy-chain constant region; the second polypeptide having an antigen-binding activity comprises an amino acid sequence of an antibody heavy chain; the third polypeptide having an antigen-binding activity comprises amino acid sequences of an antibody heavy-chain variable region and an antibody light-chain constant region; and the fourth polypeptide having an antigen-binding activity comprises an amino acid sequence of an antibody light chain.
[34] The method of any one of [21] to [33], wherein the polypeptide multimer is a multispecific antibody.
[35] The method of [34], wherein the multispecific antibody is a bispecific antibody.
[36] The method of any one of [25] to [35], wherein the antibody Fc domain or antibody heavy-chain constant region is derived from human IgG.
[37] A polypeptide multimer that comprises a first polypeptide having an antigen-binding activity and a second polypeptide having an antigen-binding activity or no antigen-binding activity, wherein the protein A-binding ability is different for the first polypeptide having an antigen-binding activity and the second polypeptide having an antigen-binding activity or no antigen-binding activity.
[38] The polypeptide multimer of [37], wherein there is a difference between the solvent pH for eluting the first polypeptide having an antigen-binding activity from protein A and that for eluting the second polypeptide having an antigen-binding activity or no antigen-binding activity from protein A.
[39] The polypeptide multimer of [37] or [38], wherein the first polypeptide having an antigen-binding activity or the second polypeptide having an antigen-binding activity or no antigen-binding activity comprises an amino acid sequence of an antibody Fc domain or an amino acid sequence of an antibody heavy-chain constant region, and wherein at least one amino acid residue selected from the amino acid residues of positions 250 to 255, 308 to 317, and 430 to 436 (EU numbering) in the amino acid sequence of the antibody Fc domain or antibody heavy-chain constant region has been modified.
[40] The polypeptide multimer of any one of [37] to [39], wherein the first polypeptide having an antigen-binding activity and the second polypeptide having an antigen-binding activity or no antigen-binding activity comprise an amino acid sequence of an antibody Fc domain or an amino acid sequence of anantibody heavy-chain constant region;
   wherein the amino acid residue of position 435 (EU numbering) in the amino acid sequence of the antibody Fc domain or antibody heavy-chain constant region is histidine or arginine in either one of the first polypeptide having an antigen-binding activity and the second polypeptide having an antigen-binding activity or no antigen-binding activity; and
   wherein the amino acid residue of position 435 (EU numbering) in the amino acid sequence of the antibody Fc domain or antibody heavy-chain constant region in either one of said polypeptides is different from that in the other polypeptide.
[41] The polypeptide multimer of any one of [37] to [40], wherein the first polypeptide having an antigen-binding activity and the second polypeptide having an antigen-binding activity or no antigen-binding activity comprise an amino acid sequence of an antibody Fc domain or an amino acid sequence of an antibody heavy-chain constant region;
   wherein the amino acid residue of position 435 (EU numbering) in the amino acid sequence of the antibody Fc domain or antibody heavy-chain constant region is histidine in either one of the first polypeptide having an antigen-binding activity and the second polypeptide having an antigen-binding activity or no antigen-binding activity; and
   wherein the amino acid residue of position 435 (EU numbering) in the amino acid sequence of the antibody Fc domain or antibody heavy-chain constant region is arginine in the other polypeptide.
[42] The polypeptide multimer of any one of [37] to [41], wherein the first polypeptide having an antigen-binding activity and the second polypeptide having an antigen-binding activity comprise an amino acid sequence of an antibody heavy-chain variable region, and at least one amino acid residue has been modified in the amino acid sequences of FR1, CDR2, and FR3 of the heavy-chain variable region.
[43] The polypeptide multimer of any one of [37] to [42], which additionally comprises one or two third polypeptides having an antigen-binding activity.
[44] The polypeptide multimer of [43], wherein the third polypeptide having an antigen-binding activity comprises an amino acid sequence of an antibody light chain.
[45] The polypeptide multimer of [43] or [44], which additionally comprises a fourth polypeptide having an antigen-binding activity.
[46] The polypeptide multimer of [45], wherein at least one of the third and fourth polypeptides having an antigen-binding activity comprises an amino acid sequence of an antibody light chain.
[47] The polypeptide multimer of [45], wherein the first polypeptide having an antigen-binding activity comprises amino acid sequences of an antibody light-chain variable region and an antibody heavy-chain constant region; the second polypeptide having an antigen-binding activity comprises an amino acid sequence of an antibody heavy chain; the third polypeptide having an antigen-binding activity comprises amino acid sequences of an antibody heavy-chain variable region and an antibody light-chain constant region; and the fourth polypeptide having an antigen-binding activity comprises an amino acid sequence of an antibody light chain.
[48] The polypeptide multimer of any one of [37] to [47], which is a multispecific antibody.
[49] The polypeptide multimer of [48], wherein the multispecific antibody is a bispecific antibody.
[50] The polypeptide multimer of any one of [37] to [41], which comprises the first polypeptide having an antigen-binding activity and the second polypeptide having no antigen-binding activity, and wherein the first polypeptide having an antigen-binding activity comprises an amino acid sequence of an antigen-binding domain of a receptor and an amino acid sequence of an antibody Fc domain, and the second polypeptide having no antigen-binding activity comprises an amino acid sequence of an antibody Fc domain.
[51] The polypeptide multimer of any one of [39] to [50], wherein the antibody Fc domain or antibody heavy-chain constant region is derived from human IgG.
[52] A nucleic acid encoding a polypeptide that constitutes the polypeptide multimer of any one of [20] and [37] to [51].
[53] A vector inserted with the nucleic acid of [52].
[54] A cell comprising the nucleic acid of [52] or the vector of [53].
[55] A pharmaceutical composition comprising the polypeptide multimer of any one of [20] and [37] to [51] as active ingredient.

### [Effects of the Invention]

The present invention provides methods that use only a protein A-based purification step for efficiently purifying or producing a highly pure polypeptide multimer having binding activity against two or more antigens (multispecific antibody), by altering its protein A-binding ability. The methods of the present invention enable efficient purification or production of a highly pure polypeptide multimer of interest without impairing the effects of other amino acid modifications of interest. In particular, by combining these methods with a method for regulating the association between two protein domains, polypeptide multimers of interest can be more efficiently produced or purified to higher purity.

The methods of the present invention for producing or purifying multispecific antibodies are characterized in that amino acid residues in their antibody heavy chain constant region and/or antibody heavy chain variable region are modified as defined in the claims. The amino acid modifications in context with the present invention are introduced into these regions to modify their protein A-binding ability. In addition, other effects of amino acid modification of interest, for example, comparable or longer plasma retention time than that of human IgG1 can also be obtained. The methods of the present invention enable efficient preparation of highly pure multispecific antibodies having such amino acid modification effects.

In general, the production of highly pure IgG-type multispecific antibodies requires a purification step using ion exchange chromatography. However, the addition of this purification step complicates the production and increases production cost. On the other hand, purification that uses only ion exchange chromatography may not be robust enough as a purification method for pharmaceuticals. Thus, it is a task to develop a method for producing an IgG-type bispecific antibody using only a protein A-based purification step, or develop a robust production method using a protein A-based purification step and an ion exchange chromatography step.

### Brief Description of the Drawings

Fig. 1 is a graph showing an assessment of the plasma retention time of MRA-IgG1 and MRA-z106/z107k in human FcRn transgenic mice.
Fig. 2 is a diagram showing that the same region in the antibody Fc domain binds to protein A and FcRn.
Fig. 3 shows a time course of the plasma concentrations of Q499-z1 18/J339-z1 19/L377-k and Q499-z121/J339-z119/L377-k after administration to human FcRn transgenic mice.
Fig. 4 is a schematic diagram of a GC33-IgG1-CD3-scFv molecule which divalently binds to cancer specific antigen glypican-3 (GPC3) and monovalently binds to T cell antigen CD3.
Fig. 5 shows the result of size exclusion chromatography analysis of protein A-purified NTA1L/NTAIR/GC33-k0 and NTA2L/NTA2R/GC33-k0.
Fig. 6 is a schematic diagram of an anti-GPC3 IgG antibody molecule that monovalently binds to glypican-3.
Fig. 7 shows the result of size exclusion chromatography analysis of protein A-purified NTA4L-cont/NTA4R-cont/GC33-k0, NTA4L-G3/NTA4R-cont/GC33-k0, and NTA4L/NTA4R/GC33-k0.
Fig. 8 shows chromatograms of NTA4L-cont/NTA4R-cont/GC33-k0, NTA4L-G3/NTA4R-cont/GC33-k0, and NTA4L/NTA4R/GC33-k0 subjected to protein A column chromatography purification with pH gradient elution.
Fig. 9 is a schematic diagram of an Fc alpha receptor-Fc fusion protein molecule that monovalently binds to IgA.
Fig. 10 shows the result of size exclusion chromatography analysis of protein A-purified IAL-cont/IAR-cont and IAL/IAR.
Fig. 11 is a schematic diagram of no1, a naturally occurring anti-IL-6 receptor/anti-GPC3 bispecific antibody.
Fig. 12 is a schematic diagram of no2, which was obtained by interchanging the anti-GPC3 antibody VH domain and VL domain in no1.
Fig. 13 is a schematic diagram of no3, which was obtained by modifying no2 to alter the isoelectric point of each chain.
Fig. 14 is a schematic diagram of no5, which was obtained by modifying no3 to enhance the heteromeric association of H chains and to purify the heteromerically associated antibody using protein A.
Fig. 15 is a schematic diagram of no6, which was obtained by modifying no5 to enhance the association between the H chain of interest and the L chain of interest.
Fig. 16 is chromatograms of anti-IL-6 receptor/anti-GPC3 bispecific antibodies no1, no2, no3, no5, and no6 in cation exchange chromatography to assess their expression patterns.
Fig. 17 is a chromatogram of no6 CM eluted with a pH gradient from a HiTrap protein A HP column (GE Healthcare).
Fig. 18 is a chromatogram of cation exchange chromatography analysis to assess a main peak fraction obtained by purification of a protein A-purified fraction of no6 using an SP Sepharose HP column (GE Healthcare).

### Mode for Carrying Out the Invention

The present invention provides methods for producing a polypeptide multimer that comprises a first polypeptide having an antigen-binding activity and a second polypeptide having an antigen-binding activity or no antigen-binding activity as defined in the claims. The methods of the present invention for producing a polypeptide multimer comprise the steps of:
(a) expressing a DNA encoding a first polypeptide having an antigen-binding activity and a DNA encoding a second polypeptide having an antigen-binding activity or no antigen-binding activity; and
(b) collecting the expression products of step (a) by protein A affinity chromatography, as further defined in the claims.

The methods of the present invention for producing a polypeptide multimer may also be expressed as methods for producing a polypeptide multimer with an altered protein A-binding ability.

In the present invention, "a polypeptide having a first antigen-binding activity" may be referred to as "a first polypeptide having an antigen-binding activity". "A polypeptide having a second antigen-binding activity or no antigen-binding activity" may be referred to as "a second polypeptide having an antigen-binding activity or no antigen-binding activity". The same applies to "a polypeptide having a third antigen-binding activity" and "a polypeptide having a fourth antigen-binding activity" described below.

In the present invention, the term "comprise" means both "comprise" and "consist of'.

The present invention also provides methods for purifying a polypeptide multimer that comprises a first polypeptide having an antigen-binding activity and a second polypeptide having an antigen-binding activity or no antigen-binding activity as defined in the claims. The methods of the present invention for purifying a polypeptide multimer comprise the steps of:
(a) expressing a DNA that encodes a first polypeptide having an antigen-binding activity and a DNA that encodes a second polypeptide having an antigen-binding activity or no antigen-binding activity; and
(b) collecting the expression products of step (a) by protein A affinity chromatography, as further defined in the claims.

A polypeptide having an antigen-binding activity in which one or more amino acid residues have been modified can be obtained by:
preparing a DNA that encodes a polypeptide having an antigen-binding activity or no antigen-binding activity,
modifying one or more nucleotides in the DNA;
introducing the resulting DNA into cells known to those skilled in the art;
culturing the cells to express the DNA; and
collecting the expression product.

Thus, the methods of the present disclosure for producing a polypeptide multimer can also be expressed as respective methods comprising the steps of:
(a) providing a DNA that encodes a first polypeptide having an antigen-binding activity and a DNA that encodes a second polypeptide having an antigen-binding activity or no antigen-binding activity;
(b) altering one or more nucleotides in either or both of the DNAs of step (a) that encode the first and second polypeptides so that there is a larger difference of protein A-binding ability between the first polypeptide having an antigen-binding activity and the second polypeptide having an antigen-binding activity or no antigen-binding activity;
(c) introducing the DNAs of step (b) into host cells and culturing the host cells to express the DNAs; and
(d) collecting the expression products of step (c) from the culture of host cells.

The methods of the present disclosure for purifying a polypeptide multimer may also be expressed as respective methods comprising the steps of:
(a) providing a DNA that encodes a first polypeptide having an antigen-binding activity and a DNA that encodes a second polypeptide having an antigen-binding activity or no antigen-binding activity;
(b) altering one or more nucleotides in either or both of the DNAs of step (a) that encode the first and second polypeptides so that there is a larger difference of protein A-binding ability between the first polypeptide having an antigen-binding activity and the second polypeptide having an antigen-binding activity or no antigen-binding activity;
(c) introducing the DNAs of step (b) into host cells and culturing the host cells to express the DNAs; and
(d) collecting the expression products of step (c) from the culture of host cells by protein A affinity chromatography.

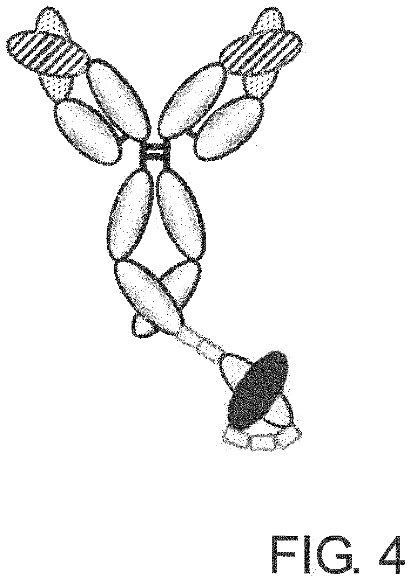

In the present invention, a polypeptide multimer refers to a heteromeric multimer containing first and second polypeptides. It is preferable that the first and second polypeptides each have an activity of binding to a different antigen. The first and second polypeptides each having a different antigen-binding activity are not particularly limited as long as one of the polypeptides has an antigen-binding domain (amino acid sequence) different from that of the other polypeptide. For example, as shown in Fig. 4 described below, one polypeptide may be fused with an antigen-binding domain that is different from that of the other polypeptide. Alternatively, as shown in Figs. 4, 6, and 9 described below, one polypeptide may be a polypeptide that monovalently binds to an antigen and does not have the antigen-binding domain possessed by the other polypeptide. Polypeptide multimers containing such first and second polypeptides are also included in the polypeptide multimers in context with the present invention.

The multimers include dimers, trimers, and tetramers, but are not limited thereto.

In present invention, a first polypeptide and/or a second polypeptide can form a multimer with one or two third polypeptides.

Thus, the present disclosure provides respective methods for producing a polypeptide multimer comprising a first polypeptide having an antigen-binding activity, a second polypeptide having an antigen-binding activity or no antigen-binding activity, and one or two third polypeptides having an antigen-binding activity, which comprise the steps of:
(a) expressing a DNA that encodes a first polypeptide having an antigen-binding activity, a DNA that encodes a second polypeptide having an antigen-binding activity, and a DNA that encodes two third polypeptides having an antigen-binding activity; and
(b) collecting the expression products of step (a);
or
(a) expressing a DNA that encodes a first polypeptide having an antigen-binding activity, a DNA that encodes a second polypeptide having no antigen-binding activity, and a DNA that encodes one third polypeptide having an antigen-binding activity; and
(b) collecting the expression products of step (a);
wherein one or more amino acid residues in either or both of the first polypeptide having an antigen-binding activity and the second polypeptide having an antigen-binding activity or no antigen-binding activity have been modified so that there is a larger difference of protein A-binding ability between the first polypeptide having an antigen-binding activity and the second polypeptide having an antigen-binding activity or no antigen-binding activity.

The above-described methods may also be expressed as methods comprising the steps of:
(a) providing a DNA that encodes a first polypeptide having an antigen-binding activity, a DNA that encodes a second polypeptide having an antigen-binding activity, and a DNA that encodes two third polypeptides having an antigen-binding activity;
(b) altering one or more nucleotides in either or both of the DNAs of step (a) that encode the first and second polypeptides so that there is a larger difference of protein A-binding ability between the first polypeptide having an antigen-binding activity and the second polypeptide having an antigen-binding activity;
(c) introducing the DNAs that encode the first, second, and two third polypeptides into host cells, and culturing the host cells to express the DNAs; and
(d) collecting the expression products of step (c) from the culture of host cells;
or
(a) providing a DNA that encodes a first polypeptide having an antigen-binding activity, a DNA that encodes a second polypeptide having no antigen-binding activity, and a DNA that encodes one third polypeptide having an antigen-binding activity;
(b) altering one or more nucleotides in either or both of the DNAs of step (a) that encode the first and second polypeptides so that there is a larger difference of protein A-binding activity between the first polypeptide having an antigen-binding activity and the second polypeptide having no antigen-binding activity;
(c) introducing the DNAs that encode the first, second, and third polypeptides into host cells and culturing the host cells to express the DNAs; and
(d) collecting the expression products of step (c) from the culture of host cells.

Furthermore, in the present invention, the first and second polypeptides can form a multimer with third and fourth polypeptides.

Thus, the present disclosure provides respective methods for producing a polypeptide multimer comprising a first polypeptide having an antigen-binding activity, a second polypeptide having an antigen-binding activity, a third polypeptide having an antigen-binding activity, and a fourth polypeptide having an antigen-binding activity, which comprise the steps of:
(a) expressing a DNA that encodes a first polypeptide having an antigen-binding activity, a DNA that encodes a second polypeptide having an antigen-binding activity, and a DNA that encodes a third polypeptide having an antigen-binding activity and a fourth polypeptide having an antigen-binding activity; and
(b) collecting the expression products of step (a);
wherein one or more amino acid residues in either or both of the first polypeptide having an antigen-binding activity and the second polypeptide having an antigen-binding activity have been modified so that there is a larger difference of protein A-binding ability between the first polypeptide having an antigen-binding activity and the second polypeptide having an antigen-binding activity.

The above-described methods can also be expressed as methods comprising the steps of:
(a) providing a DNA that encodes a first polypeptide having an antigen-binding activity, a DNA that encodes a second polypeptide having an antigen-binding activity, and a DNA that encodes a third polypeptide having an antigen-binding activity and a fourth polypeptide having an antigen-binding activity;
(b) altering one or more nucleotides in either or both of the DNAs of step (a) that encode the first and second polypeptides so that there is a larger difference of protein A-binding ability between the first polypeptide having an antigen-binding activity and the second polypeptide having an antigen-binding activity;
(c) introducing the DNAs that encode the first, second, third, and fourth polypeptides into host cells and culturing the host cells to express the DNAs; and
(d) collecting the expression products of step (c) from the culture of host cells.

The present disclosure provides respective methods for purifying a polypeptide multimer that comprises a first polypeptide having an antigen-binding activity, a second polypeptide having an antigen-binding activity or no antigen-binding activity, and one or two third polypeptides having an antigen-binding activity, which comprise the steps of:
(a) expressing a DNA that encodes a first polypeptide having an antigen-binding activity, a DNA that encodes a second polypeptide having an antigen-binding activity, and a DNA that encodes two third polypeptides having an antigen-binding activity; and
(b) collecting the expression products of step (a);
or
(a) expressing a DNA that encodes a first polypeptide having an antigen-binding activity, a DNA that encodes a second polypeptide having no antigen-binding activity, and a DNA that encodes one third polypeptide having an antigen-binding activity; and
(b) collecting the expression products of step (a);
wherein one or more amino acid residues in either or both of the first polypeptide having an antigen-binding activity and the second polypeptide having an antigen-binding activity or no antigen-binding activity have been modified so that there is a larger difference of protein A-binding ability between the first polypeptide having an antigen-binding activity and the second polypeptide having an antigen-binding activity or no antigen-binding activity.

The above-described methods can also be expressed as methods comprising the steps of:
(a) providing a DNA that encodes a first polypeptide having an antigen-binding activity, a DNA that encodes a second polypeptide having an antigen-binding activity or no antigen-binding activity, and a DNA that encodes two third polypeptides having an antigen-binding activity;
(b) altering one or more nucleotides in either or both of the DNAs of step (a) that encode the first and second polypeptides so that there is a larger difference of protein A-binding ability between the first polypeptide having an antigen-binding activity and the second polypeptide having an antigen-binding activity;
(c) introducing the DNAs that encode the first, second, and two third polypeptides into host cells and culturing the host cells to express the DNAs; and
(d) collecting the expression products of step (c) from the culture of host cells;
or
(a) providing a DNA that encodes a first polypeptide having an antigen-binding activity, a DNA that encodes a second polypeptide having no antigen-binding activity, and a DNA that encodes one third polypeptide having an antigen-binding activity;
(b) altering one or more nucleotides in either or both of the DNAs of step (a) that encode the first and second polypeptides so that there is a larger difference of protein A-binding ability between the first polypeptide having an antigen-binding activity and the second polypeptide having no antigen-binding activity;
(c) introducing the DNAs that encode the first, second, and third polypeptides into host cells and culturing the host cells to express the DNAs; and
(d) collecting the expression products of step (c) from the culture of host cells.

The present disclosure also provides respective methods for purifying a polypeptide multimer that comprises a first polypeptide having an antigen-binding activity, a second polypeptide having an antigen-binding activity, a third polypeptide having an antigen-binding activity, and a fourth polypeptide having an antigen-binding activity, which comprise the steps of:
(a) expressing a DNA that encodes a first polypeptide having an antigen-binding activity, a DNA that encodes a second polypeptide having an antigen-binding activity, a DNA that encodes a third polypeptide having an antigen-binding activity, and a DNA that encodes a fourth polypeptide having an antigen-binding activity; and
(b) collecting the expression products of step (a) by protein A affinity chromatography;
wherein one or more amino acid residues in either or both of the first polypeptide having an antigen-binding activity and the second polypeptide having an antigen-binding activity have been modified so that there is a larger difference of protein A-binding ability between the first polypeptide having an antigen-binding activity and the second polypeptide having an antigen-binding activity.

The above-described methods can also be expressed as respective methods comprising the steps of:
(a) providing a DNA that encodes a first polypeptide having an antigen-binding activity, a DNA that encodes a second polypeptide having an antigen-binding activity, a DNA that encodes a third polypeptide having an antigen-binding activity, and a DNA that encodes a fourth polypeptide having an antigen-binding activity;
(b) altering one or more nucleotides in either or both of the DNAs of step (a) that encode the first and second polypeptides so that there is a larger difference of protein A-binding ability between the first polypeptide having an antigen-binding activity and the second polypeptide having an antigen-binding activity;
(c) introducing the DNAs that encode the first, second, third, and fourth polypeptides into host cells and culturing the host cells to express the DNAs; and
(d) collecting the expression products of step (c) from the culture of host cells by protein A affinity chromatography.

In a polypeptide multimer in context with the present invention containing a first polypeptide, a second polypeptide, and one or two third polypeptides, the first and second polypeptides can each form a multimer (dimer) with the third polypeptide. Furthermore, the resulting two dimers can form a multimer with each other. The two third polypeptides may have completely the same amino acid sequence (may have a binding activity to the same antigen). Alternatively, the third polypeptides may have the same amino acid sequence and two or more activities (for example, may have binding activities to two or more different antigens). When only one third polypeptide is present, the third polypeptide can form a polypeptide multimer via dimerization with either the first polypeptide or the second polypeptide.

In a polypeptide multimer in context with the present invention, the first and second polypeptides preferably have binding activity to different antigens. Meanwhile, the third polypeptide may have binding activity to the same antigen as that of either or both of the first and second polypeptides. Alternatively, the third polypeptide may have binding activity to an antigen different from those of the first and second polypeptides.

Alternatively, a polypeptide multimer in context with the present invention may contain a first polypeptide, second polypeptide, third polypeptide, and fourth polypeptide. In such a polypeptide multimer, the first polypeptide and second polypeptide can form a multimer (dimer) with the third polypeptide and fourth polypeptide, respectively. For example, through formation of disulfide bonds in between, the first polypeptide and third polypeptide can form a dimer, and the second polypeptide and fourth polypeptide can form a dimer.

In a polypeptide multimer in context with the present invention, the first and second polypeptides preferably have binding activity to different antigens. Meanwhile, the third polypeptide may have binding activity to the same antigen as that of either or both of the first and second polypeptides. Alternatively, the third polypeptide may have binding activity to an antigen different from those of the first and second polypeptides. Furthermore, the fourth polypeptide may have binding activity to the same antigen as that of either or both of the first and second polypeptides. Alternatively, the fourth polypeptide may have binding activity to an antigen different from those of the first and second polypeptides.

Specifically, for example, when the first and second polypeptides contain the amino acid sequence of an antibody heavy chain against antigen A and the amino acid sequence of an antibody heavy chain against antigen B, respectively, the third and fourth polypeptides may contain the amino acid sequence of an antibody light chain against antigen A and the amino acid sequence of an antibody light chain against antigen B, respectively. When a polypeptide multimer in context with the present invention has third and fourth polypeptides that contain two different antibody light chain amino acid sequences, a highly pure polypeptide multimer of interest can be efficiently produced or purified by making the pI values of the third and fourth polypeptide different using the methods described below, or by differentiating their protein L-binding ability, in addition to differentiating the protein A-binding ability between the first and second polypeptides.

Alternatively, for example, when the first polypeptide has the amino acid sequence of an antibody heavy chain against antigen A, the second polypeptide has the amino acid sequence of an antibody light chain variable region against antigen B and the amino acid sequence of an antibody heavy chain constant region, the third polypeptide has the amino acid sequence of an antibody light chain against antigen A, and the fourth polypeptide has the amino acid sequence of an antibody heavy chain variable region against antigen B and the amino acid sequence of an antibody light chain constant region, a highly pure polypeptide multimer of interest having the first, second, third, and fourth polypeptides can also be efficiently produced or purified by using the present invention. In this case, as described in Example 12 below, introduction of amino acid mutations to alter the pI value of a polypeptide or introduction of amino acid mutations to promote the association of polypeptides of interest (WO2006/106905) enables more efficient purification or production of a polypeptide multimer of interest having the first, second, third, and fourth polypeptides to higher purity. Amino acid mutations to be introduced to promote the association of polypeptides may be those used in the methods described in Protein Eng. 1996 Jul., 9(7):617-21; Protein Eng Des Sel. 2010 Apr., 23(4):195-202; J Biol Chem. 2010 Jun. 18, 285(25):19637-46; WO2009080254; and such, in which two polypeptides having a heavy chain constant region are heteromerically associated by modifying the CH3 domain of heavy chain constant region; and those used in the methods described in WO2009080251, WO2009080252, WO2009080253, and such, by which the association of a particular pair of heavy chain and light chain is promoted.

In the present invention, "polypeptide having an antigen-binding activity" refers to a peptide or protein of five or more amino acids in length having a domain (region) capable of binding to a protein or peptide such as an antigen or ligand, e.g., an antibody heavy chain or light chain variable region, receptor, receptor-Fc domain fusion peptide, scaffold, or a fragment thereof. Specifically, a polypeptide having an antigen-binding activity can contain the amino acid sequence of an antibody variable region, receptor, receptor-Fc domain fusion peptide, scaffold, or a fragment thereof.

Scaffold may be any polypeptide as long as it is a conformationally stable polypeptide capable of binding to at least one antigen. Such polypeptides include, but are not limited to, for example, antibody variable region fragments, fibronectin, protein A domains, LDL receptor A domains, lipocalins, and molecules mentioned in Nygren et al. (Current Opinion in Structural Biology, 7:463-469 (1997); Journal of Immunol. Methods, 290:3-28 (2004)), Binz et al. (Nature Biotech 23:1257-1266 (2005)), and Hosse et al. (Protein Science 15:14-27 (2006)).

Methods for obtaining antibody variable regions, receptors, receptor-Fc domain fusion peptides, scaffold, and fragments thereof are known to those skilled in the art.

Such polypeptides having an antigen-binding activity may be derived from a living organism or designed artificially. The polypeptides may be derived from natural proteins, synthetic proteins, recombinant proteins, and such. Furthermore, the polypeptides may be peptides or protein fragments of 10 or more amino acids in length which have a domain (region) capable of binding to a protein or peptide such as an antigen or ligand, as long as they have ability to bind to an antigen. The polypeptides may have more than one domain capable of binding to an antigen (including ligand).

A polypeptide having an antigen-binding activity may also be referred to as a polypeptide having an antigen-binding protein domain(s).

In the present invention, "polypeptide having no antigen-binding activity" refers to a peptide or protein of five or more amino acids in length, such as an antibody fragment having no antigen-binding activity, Fc domain, scaffold, or a fragment thereof. Specifically, a polypeptide having no antigen-binding activity may contain the amino acid sequence of an antibody constant region, Fc domain, scaffold, or fragment thereof, but the amino acid sequence is not limited to the above examples. A polypeptide having no antigen-binding activity can be combined with a polypeptide having an antigen-binding activity to produce a polypeptide multimer that monovalently binds to an antigen.

In the present invention, the first polypeptide having an antigen-binding activity and the second polypeptide having an antigen-binding activity or no antigen-binding activity may contain the amino acid sequence of an antibody heavy chain constant region or the amino acid sequence of an antibody Fc domain. The amino acid sequence of an antibody Fc domain or an antibody heavy chain constant region includes those of human IgG-type constant regions and Fc domains. IgG-type constant regions or Fc domains may be of natural IgG1, IgG2, IgG3, or IgG4 isotype, or may be variants thereof.

Meanwhile, in the present invention, the third polypeptide having an antigen-binding activity and the fourth polypeptide having an antigen-binding activity may contain the amino acid sequence of an antibody light chain constant region. The amino acid sequence of an antibody light chain constant region includes, but is not limited to, those of human kappa- and human lambda-type constant regions, and variants thereof.

Furthermore, in the present invention, polypeptides having an antigen-binding activity may contain the amino acid sequence of an antibody variable region (for example, the amino acid sequences of CDR1, CDR2, CDR3, FR1, FR2, FR3, and FR4).

Moreover, in the present invention, the polypeptides having an antigen-binding activity may contain the amino acid sequence of an antibody heavy chain or an antibody light chain. More specifically, the first polypeptide having an antigen-binding activity and the second polypeptide having an antigen-binding activity or no antigen-binding activity may contain the amino acid sequence of an antibody heavy chain. Meanwhile, the third polypeptide having an antigen-binding activity and the fourth polypeptide having an antigen-binding activity may contain the amino acid sequence of an antibody light chain.

When a polypeptide multimer of interest is a tetramer that is formed by multimerization between a dimer formed by the first and third polypeptides and a dimer formed by the second and fourth polypeptides, for example, a polypeptide in which the first and second polypeptides having an antigen-binding activity contain the amino acid sequence of an antibody heavy chain, and a polypeptide in which the third and fourth polypeptides having an antigen-binding activity contain the amino acid sequence of an antibody light chain, can be used for the polypeptide multimer in context with the present invention. Alternatively, a polypeptide in which the first polypeptide having an antigen-binding activity contains the amino acid sequence of an antibody heavy chain, a polypeptide in which the second polypeptide having an antigen-binding activity contains the amino acid sequence of an antibody light chain variable region and the amino acid sequence of an antibody heavy chain constant region, a polypeptide in which the third polypeptide having an antigen-binding activity contains the amino acid sequence of an antibody light chain, and a polypeptide in which the fourth polypeptide having an antigen-binding activity contains the amino acid sequence of an antibody heavy chain variable region, can also be used.

Specifically, a polypeptide multimer in context with the present invention can be a multispecific antibody.

In the present invention, a "multispecific antibody" refers to an antibody capable of specifically binding to at least two different antigens.

In the present invention, "different antigens" refers not only to different antigen molecules *per se,* but also to different antigen determinants present in the same antigen molecules. Accordingly, for example, different antigen determinants present within a single molecule are included in the "different antigens" in context with the present invention. In the present invention, antibodies that recognize various different antigen determinants in a single molecule are regarded as "antibodies capable of specifically binding to different antigens".

In the present invention, multispecific antibodies include, but are not limited to, bispecific antibodies capable of specifically binding to two types of antigens. Preferred bispecific antibodies of the present invention include H2L2-type IgG antibodies (composed of two types of H chains and two types of L chains) having a human IgG constant region. More specifically, such antibodies include, but are not limited to, for example, IgG-type chimeric antibodies, humanized antibodies, and human antibodies.

Moreover, a polypeptide having an antigen-binding activity may be, for example, a molecule in which at least two of a heavy chain variable region, light chain variable region, heavy chain constant region, and light chain constant region, are linked together as a single chain. Alternatively, the polypeptide may be an antibody in which at least two of a heavy chain variable region, light chain variable region, Fc domain (constant region without CH1 domain), and light chain constant region, are linked together as a single chain.

In the present disclosure, the phrase "there is a larger difference of protein A-binding ability between polypeptides having an antigen-binding activity" means that the protein A-binding ability is not the same (is different) between two or more polypeptides as a result of amino acid modifications on the surface of polypeptides having an antigen-binding activity. More specifically, this phrase means that, for example, the protein A-binding ability of the first polypeptide having an antigen-binding activity is different from that of the second polypeptide having an antigen-binding activity. The difference of protein A-binding ability can be examined, for example, by using protein A affinity chromatography.

The strength of protein A-binding ability of a polypeptide having an antigen-binding activity is correlated with the pH of solvent used for elution. The greater the protein A-binding ability of the polypeptide is, the lower the pH of the solvent used for elution becomes. Thus, the phrase "there is a larger difference of protein A-binding ability between polypeptides having an antigen-binding activity" can also be expressed as "when two or more polypeptides having an antigen-binding activity are eluted using protein A affinity chromatography, each polypeptide is eluted at a different solvent pH". The difference in the pH of the elution solvent is 0.1 or more, preferably 0.5 or more, and still more preferably 1.0 or more, but is not limited thereto.

Furthermore, in the present disclosure, it is preferable to alter the protein A-binding ability without lowering other activities (for example, plasma retention) of the polypeptides having an antigen-binding activity.

A polypeptide multimer of interest that comprises the first polypeptide having an antigen-binding activity and the second polypeptide having an antigen-binding activity or no antigen-binding activity can be produced or purified using protein A affinity chromatography based on the difference of protein A-binding ability between the first polypeptide having an antigen-binding activity and the second polypeptide having an antigen-binding activity or no antigen-binding activity. Specifically, for example, when the polypeptide multimer in context with the present invention is a bispecific antibody that has a common L chain (i.e., same amino acid sequence in the third and fourth polypeptides), the polypeptide multimer can be produced or purified by the method described below. First, host cells are introduced with the following: a nucleic acid that encodes the first polypeptide having an antigen-binding activity (more specifically, the first antibody heavy chain) whose amino acid at position 435 (EU numbering) in the amino acid sequence of the antibody heavy chain constant region is arginine (R); a nucleic acid that encodes the second polypeptide having an antigen-binding activity (more specifically, the second antibody heavy chain) whose amino acid at position 435 (EU numbering) in the amino acid sequence of the antibody heavy chain constant region is histidine (H); and a nucleic acid that encodes the third polypeptide having an antigen-binding activity (common L chain). The cells are cultured to express the DNAs transiently. Then, the resulting expression products are loaded onto a protein A column. After washing, elution is performed first with a high pH elution solution and then with a low pH elution solution. A homomeric antibody comprising two units of the first antibody heavy chain and two units of the common L chain does not have any protein A-binding site in its heavy chain constant region. Meanwhile, a bispecific antibody comprising the first antibody heavy chain, the second antibody heavy chain, and two units of the common L chain has a single protein A-binding site in its heavy chain constant region. A homomeric antibody comprising two units of the second antibody heavy chain and two units of the common L chain has two protein A-binding sites in its heavy chain constant region. As described above, the protein A-binding ability of a polypeptide correlates with the solvent pH for eluting the polypeptide in protein A affinity chromatography. The greater the protein A-binding ability is, the lower the solvent pH for elution becomes. Thus, when elution is carried out first with a high pH elution solution and then with a low pH elution solution, the antibodies are eluted in the following order:
- a homomeric antibody comprising two units of the first antibody heavy chain and two units of the common L chain
- a bispecific antibody comprising the first antibody heavy chain, the second antibody heavy chain, and two units of the common L chain
- a homomeric antibody comprising two units of the second antibody heavy chain and two units of the common L chain
This allows production or purification of the polypeptide multimers (bispecific antibodies) of interest.

The purity of the polypeptide multimers obtained by the production or purification methods of the present invention is preferably at least 95% or higher (for example, 96%, 97%, 98%, 99% or higher).

Modifications of amino acid residues to create a difference in the protein A-binding ability between the first polypeptide having an antigen-binding activity and the second polypeptide having an antigen-binding activity or no antigen-binding activity include, but are not limited to:
(1) modification of one or more amino acid residues in the amino acid sequence of either one of the first polypeptide having an antigen-binding activity and the second polypeptide having an antigen-binding activity or no antigen-binding activity, such that the protein A-binding ability of one of the polypeptides is increased;
(2) modification of one or more amino acid residues in the amino acid sequence of either one of the first polypeptide having an antigen-binding activity and the second polypeptide having an antigen-binding activity or no antigen-binding activity, such that the protein A-binding ability of one of the polypeptides is decreased; and
(3) modification of one or more amino acid residues in the first polypeptide having an antigen-binding activity and the second polypeptide having an antigen-binding activity or no antigen-binding activity, such that the protein A-binding ability of either one of the first polypeptide having an antigen-binding activity and the second polypeptide having an antigen-binding activity or no antigen-binding activity is increased, and the protein A-binding ability of the other polypeptide is decreased.

In the present disclosure, it is preferred that amino acids on the surface of a polypeptide having an antigen-binding activity or no antigen-binding activity are modified. Furthermore, it is also preferred to consider reducing the influence of the modification on other activities of the polypeptide.

Accordingly, in the present disclosure, it is preferred to modify, for example, the amino acid residues at the following positions (EU numbering) in the antibody Fc domain or heavy chain constant region:
TLMISR at positions 250-255, VLHQDWLNGK at positions 308-317, and EALHNHY at positions 430-436;
preferably, TLMIS at positions 250-254, LHQD at positions 309-312, LN at positions 314 and 315, E at position 430, and LHNHY at positions 432-436;
more preferably, LMIS at positions 251-254, LHQ at positions 309-311, L at position 314, and LHNH at positions 432-435; and in particular, MIS at positions 252-254, L at position 309, Q at position 311, and NHY at positions 434-436.

As for amino acid modifications of the antibody heavy chain variable region, preferred mutation sites include FR1, CDR2, and FR3. More preferred mutation sites include, for example, positions H15-H23, H56-H59, H63-H72, and H79-H83 (EU numbering).

Of the above amino acid modifications, modifications that do not reduce the binding to FcRn or the plasma retention in human FcRn transgenic mice are more preferred.

More specifically, modifications that increase the protein A-binding ability of a polypeptide include, but are not limited to, substitution of histidine (His) for the amino acid residue at position 435 (EU numbering) in the amino acid sequence of an antibody Fc domain or an antibody heavy chain constant region.

Meanwhile, modifications that reduce the protein A-binding ability of a polypeptide include, but are not limited to, substitution of arginine for the amino acid residue at position 435 (EU numbering) in the amino acid sequence of an antibody Fc domain or an antibody heavy chain constant region.

As for the antibody heavy chain variable region, the heavy chain variable region of the VH3 subclass has protein A-binding activity. Thus, to increase the protein A-binding ability, the amino acid sequences at the above modification sites are preferably identical to those of the heavy chain variable region of the VH3 subclass. To reduce the protein A-binding ability, the amino acid sequences are preferably identical to those of the heavy chain variable region of another subclass.

As described below, modification of amino acid residues can be achieved by altering one or more nucleotides in a DNA encoding a polypeptide, and expressing the DNA in host cells. Those skilled in the art can readily determine the number, site, and type of altered nucleotides depending on the type of amino acid residues after modification.

Herein, modification (alteration) refers to substitution, deletion, addition, or insertion, or combinations thereof.

The polypeptide having an antigen-binding activity may comprise other modifications in addition to the above modifications of amino acid residues. Such additional modifications can be selected from, for example, substitutions, deletions, and modifications of amino acids, and combinations thereof. Specifically, all polypeptides whose amino acid sequences comprise a modification described below are included in the present invention:
- amino acid modification for increasing the rate of heteromeric association of two types of H chains in a bispecific antibody
- amino acid modification for stabilizing the disulfide bonds between the first polypeptide having an antigen-binding activity and the second polypeptide having an antigen-binding activity or no antigen-binding activity
- amino acid modification for improving the plasma retention of an antibody
- modification for increasing the stability under acidic conditions
- modification for reducing the heterogeneity
- modification for suppressing deamidation reaction
- modification for introducing a difference in between the isoelectric points of two types of polypeptides
- modification for altering the Fcγ receptor-binding ability

These amino acid modifications are described below.

### Amino acid modification for increasing the rate of heteromeric association between the two types of H chains in a bispecific antibody

The amino acid modifications in context with the present invention can be combined with the amino acid modifications described in WO2006106905. There is no limitation on the modification sites as long as the amino acids form the interface between two polypeptides having an antigen-binding activity. Specifically, for example, when a heavy chain constant region is modified, such modifications include modifications that make the amino acids of at least one of the combinations of positions 356 and 439, positions 357 and 370, and positions 399 and 409 (EU numbering) in the amino acid sequence of the heavy chain constant region of the first polypeptide having an antigen-binding activity have the same electric charge; and the amino acids of at least one of the combinations of positions 356 and 439, positions 357 and 370, and positions 399 and 409 (EU numbering) in the heavy chain constant region of the second polypeptide having an antigen-binding activity or no antigen-binding activity have electric charge opposite to that of the first polypeptide having an antigen-binding activity. More specifically, such modifications include, for example, introduction of a mutation that substitutes Glu at position 356 (EU numbering) with Lys in the amino acid sequence of the heavy chain constant region of either one of the first polypeptide having an antigen-binding activity and the second polypeptide having an antigen-binding activity, and a mutation that substitutes Lys at position 439 (EU numbering) with Glu in the amino acid sequence of the heavy chain constant region of the other polypeptide. When these modifications are combined with the modifications in context with the present invention, the polypeptide of interest can be obtained with a higher purity by protein A-based purification alone.

Alternatively, the polypeptide multimer of interest that comprises the first, second, third, and fourth polypeptides having an antigen-binding activity can be efficiently produced or purified to a higher purity, when modification is performed to make the amino acids at position 39 (Kabat numbering) in the heavy chain variable region and/or at position 213 (EU numbering) in the heavy chain constant region of the first polypeptide having an antigen-binding activity have an electric charge opposite to that of the amino acid at position 39 (Kabat numbering) in the heavy chain variable region and/or the amino acid at position 213 (EU numbering) in the heavy chain constant region of the second polypeptide having an antigen-binding activity or no antigen-binding activity, and the amino acid at position 38 (Kabat numbering) and/or the amino acid at position 123 (EU numbering) in the light chain variable region of the third polypeptide having an antigen-binding activity have an electric charge opposite to that of the amino acid at position 38 (Kabat numbering) and/or the amino acid at position 123 (EU numbering) in the light chain variable region of the fourth polypeptide having an antigen-binding activity.

### Amino acid modification for stabilizing the disulfide bonds between the first polypeptide having an antigen-binding activity and the second polypeptide having an antigen-binding activity or no antigen-binding activity

As described in published documents (Mol. Immunol. 1993, 30, 105-108; and Mol. Immunol. 2001, 38, 1-8), the heterogeneity of IgG4 is eliminated and its stable structure can be maintained by substituting Pro for Ser at position 228 (EU numbering) in the amino acid sequence of the heavy chain constant region of IgG4.

### Amino acid modification for improving the plasma retention of an antibody

In order to regulate plasma retention, it is possible to combine the amino acid modifications in context with the present invention with amino acid modifications that alter the antibody pI value. Modifications to constant regions include, for example, amino acid modifications at positions 250 and 428 (EU numbering) and such described in published documents (J. Immunol. 2006, 176 (1):346-356; and Nat. Biotechnol. 1997 15 (7):637-640). Modifications to variable regions include the amino acid modifications described in WO2007/114319 and WO2009/041643. Amino acids to be modified are preferably exposed on the surface of a polypeptide having an antigen-binding activity. The modifications include, for example, amino acid substitution at position 196 (EU numbering) in the amino acid sequence of a heavy chain constant region. In the case of the heavy chain constant region of IgG4, the plasma retention can be enhanced, for example, by substituting glutamine for lysine at position 196 thereby reducing the pI value.

Furthermore, the plasma retention can be regulated by altering the FcRn-binding ability. Amino acid modifications that alter the FcRn-binding ability include, for example, the amino acid substitutions in the antibody heavy chain constant region described in published documents (The Journal of Biological Chemistry vol.276, No.9 6591-6604, 2001; Molecular Cell, Vol.7, 867-877, 2001; Curr Opin Biotechnol. 2009, 20 (6):685-91). Such amino acid substitutions include, for example, substitutions at positions 233, 238, 253, 254, 255, 256, 258, 265, 272, 276, 280, 285, 288, 290, 292, 293, 295, 296, 297, 298, 301, 303, 305, 307, 309, 311, 312, 315, 317, 329, 331, 338, 360, 362, 376, 378, 380, 382, 415, 424, 433, 434, 435, and 436 (EU numbering).

### Modification for improving the stability under acidic conditions

When the heavy chain constant region of IgG4 is used, the stable four-chain structure (H2L2 structure) is preferably maintained by suppressing the conversion of IgG4 into the half-molecule form under acidic conditions. Thus, arginine at amino acid position 409 (EU numbering system) which plays an important role in the maintenance of the four-chain structure (Immunology 2002, 105, 9-19) is preferably substituted with lysine of the IgG1 type that maintains a stable four-chain structure even under acidic conditions. Furthermore, to improve the acidic stability of IgG2, methionine at amino acid position 397 (EU numbering system) can be substituted with valine. These modifications can be used in combination with the amino acid modifications in context with the present invention.

### Modification for reducing heterogeneity

The amino acid modifications in context with the present invention may be combined with the methods described in WO2009041613. Specifically, for example, the modification in which the two amino acids at the C-terminus of the IgG1 heavy chain constant region (i.e., glycine and lysine at positions 446 and 447 [EU numbering], respectively) are deleted can be combined with the amino acid modifications described in the Examples herein.

### Modification for suppressing deamidation reaction

The amino acid modifications in context with the present invention may be combined with amino acid modifications for suppressing deamidation reaction. Deamidation reaction has been reported to occur more frequently at a site where asparagine (N) and glycine (G) are adjacent to each other (---NG---) (Geiger et al., J. Bio. Chem. (1987) 262:785-794). When a polypeptide multimer (multispecific antibody) in context with the present invention has a site where asparagine and glycine are adjacent to each other, deamidation reaction can be suppressed by modifying the amino acid sequence. Specifically, for example, either or both of asparagine and glycine are substituted with other amino acids. More specifically, for example, asparagine is substituted with aspartic acid.

### Modification for introducing a difference in isoelectric point between two types of polypeptides

The amino acid modifications in context with the present invention may be combined with amino acid modifications for introducing a difference in isoelectric point. Specific methods are described, for example, in WO2007/114325. In addition to the modifications in context with the present invention, the amino acid sequences of the first polypeptide having an antigen-binding activity and the second polypeptide having an antigen-binding activity or no antigen-binding activity are modified so that there is a larger difference in isoelectric point between these polypeptides. This enables efficient production or purification of the polypeptide of interest to a higher purity. Furthermore, a larger difference in isoelectric point can be produced between the third polypeptide having an antigen-binding activity and the fourth polypeptide having an antigen-binding activity. This allows the polypeptide multimer of interest comprising the first, second, third, and fourth polypeptides to be efficiently produced or purified to a higher purity. Specifically, when the first and second polypeptides each comprises an amino acid sequence of an antibody heavy chain, the modification sites include, for example, positions 1, 3, 5, 8, 10, 12, 13, 15, 16, 19, 23, 25, 26, 39, 42, 43, 44, 46, 68, 71, 72, 73, 75, 76, 81, 82b, 83, 85, 86, 105, 108, 110, and 112 (Kabat numbering). When the third and fourth polypeptides each comprises an amino acid sequence of an antibody light chain, the modification sites include, for example, positions 1, 3, 7, 8, 9, 11, 12, 16, 17, 18, 20, 22, 38, 39, 41, 42, 43, 45, 46, 49, 57, 60, 63, 65, 66, 68, 69, 70, 74, 76, 77, 79, 80, 81, 85, 100, 103, 105, 106, 107, and 108 (Kabat numbering). A larger difference in isoelectric point can be produced by modifying at least one of the amino acid residues at the above positions in one polypeptide to have an electric charge, and modifying at least one of the amino acid residues at the above positions in the other polypeptide to have no charge or opposite electric charge to the above.

### Modification for altering the Fcγ receptor-binding ability

The amino acid modifications in context with the present invention may be combined with amino acid modifications that alter (increase or reduce) the Fcγ receptor-binding ability. Modifications for altering the Fcγ receptor-binding ability include, but are not limited to, the modifications described in Curr Opin Biotechnol. 2009, 20(6):685-91. Specifically, the Fcγ receptor-binding ability can be altered, for example, by combining the modifications in context with the present invention with a modification that substitutes leucine at positions 234 and 235 and asparagine at position 272 (EU numbering) of an IgG1 heavy chain constant region with other amino acids. The amino acids after substitution include, but are not limited to, alanine.

Preparation of DNAs that encode polypeptides having an antigen-binding activity, modification of one or more nucleotides, DNA expression, and recovery of expression products are described below

### Preparation of DNAs that encode polypeptides having an antigen-binding activity

In the present invention, a DNA that encodes a polypeptide having an antigen-binding activity or a polypeptide having no antigen-binding activity may be the whole or a portion of a known sequence (naturally-occurring or artificial sequence), or combinations thereof. Such DNAs can be obtained by methods known to those skilled in the art. The DNAs can be isolated, for example, from antibody libraries, or by cloning antibody-encoding genes from hybridomas producing monoclonal antibodies.

With regard to antibody libraries, many are already well known, and those skilled in the art can appropriately obtain antibody libraries since methods for producing antibody libraries are known. For example, regarding antibody phage libraries, one can refer to the literature such as Clackson et al., Nature 1991, 352: 624-8; Marks et al., J. Mol. Biol. 1991, 222: 581-97; Waterhouses et al., Nucleic Acids Res. 1993, 21: 2265-6; Griffiths et al., EMBO J. 1994, 13: 3245-60; Vaughan et al., Nature Biotechnology 1996, 14: 309-14; or Japanese Patent Kohyo Publication No. (JP-A) H20-504970 (unexamined Japanese national phase publication corresponding to a non-Japanese international publication). In addition, known methods such as methods that use eukaryotic cells as libraries (WO95/15393) and ribosome display methods may be used. Furthermore, techniques to obtain human antibodies by panning using human antibody libraries are also known. For example, variable regions of human antibodies can be expressed on the surface of phages as single chain antibodies (scFvs) using phage display methods, and phages that bind to antigens can be selected. Genetic analysis of the selected phages can determine the DNA sequences encoding the variable regions of human antibodies that bind to the antigens. Once the DNA sequences of scFvs that bind to the antigens is revealed, suitable expression vectors can be produced based on these sequences to obtain human antibodies. These methods are already well known, and one can refer to WO92/01047, WO92/20791, WO93/06213, WO93/11236, WO93/19172, WO95/01438, and WO95/15388.

As for methods for obtaining genes encoding antibodies from hybridomas, basically, known techniques may be used. Specifically, desired antigens or cells expressing the desired antigens are used as sensitizing antigens for immunization according to conventional immunization methods. The immune cells thus obtained are fused with known parent cells by ordinary cell fusion methods, and monoclonal antibody producing cells (hybridomas) are screened by ordinary screening methods. cDNAs of antibody variable regions (V regions) can be obtained by reverse transcription of mRNAs of the obtained hybridomas using reverse transcriptase. Antibody-encoding genes can be obtained by linking them with DNAs encoding the desired antibody constant regions (C regions).

More specifically, without limitations, the following methods are examples.

Sensitizing antigens for obtaining the antibody genes encoding the antibody heavy and light chains include both complete antigens with immunogenicity and incomplete antigens composed of haptens and such that do not show antigenicity. For example, full length proteins and partial peptides of proteins of interest can be used. In addition, it is known that substances composed of polysaccharides, nucleic acids, lipids, and such may become antigens. Thus, there are no particular limitations on antigens in the present invention. Antigens can be prepared by methods known to those skilled in the art, and they can be prepared, for example, by the following methods using baculoviruses (for example, WO98/46777). Hybridomas can be produced, for example, the following methods of Milstein *et al.* (G. Kohler and C. Milstein, Methods Enzymol. 1981, 73: 3-46), and such. When the immunogenicity of an antigen is low, it can be linked to a macromolecule that has immunogenicity, such as albumin, and then used for immunization. Furthermore, by linking antigens with other molecules if necessary, they can be converted into soluble antigens. When transmembrane molecules such as receptors are used as antigens, portions of the extracellular regions of the receptors can be used as a fragment, or cells expressing transmembrane molecules on their cell surface may be used as immunogens.

Antibody-producing cells can be obtained by immunizing animals using suitable sensitizing antigens described above. Alternatively, antibody-producing cells can be prepared by *in vitro* immunization of lymphocytes that can produce antibodies. Various mammals can be used as the animals for immunization, where rodents, lagomorphas and primates are generally used. Examples of such animals include mice, rats, and hamsters for rodents, rabbits for lagomorphas, and monkeys including the cynomolgus monkey, rhesus monkey, hamadryas, and chimpanzees for primates. In addition, transgenic animals carrying human antibody gene repertoires are also known, and human antibodies can be obtained by using these animals (see WO96/34096; Mendez et al., Nat. Genet. 1997, 15: 146-56). Instead of using such transgenic animals, for example, desired human antibodies having binding activity against antigens can be obtained by *in vitro* sensitization of human lymphocytes with desired antigens or cells expressing the desired antigens, and then fusing the sensitized lymphocytes with human myeloma cells such as U266 (see Japanese Patent Application Kokoku Publication No. (JP-B) H1-59878 (examined, approved Japanese patent application published for opposition)). Furthermore, desired human antibodies can be obtained by immunizing transgenic animals carrying a complete repertoire of human antibody genes, with desired antigens (see WO93/12227, WO92/03918, WO94/02602, WO96/34096, and WO96/33735).

Animal immunization can be carried out by appropriately diluting and suspending a sensitizing antigen in Phosphate-Buffered Saline (PBS), physiological saline, or such, and forming an emulsion by mixing an adjuvant if necessary, followed by an intraperitoneal or subcutaneous injection into animals. After that, the sensitizing antigen mixed with Freund's incomplete adjuvant is preferably administered several times every four to 21 days. Antibody production can be confirmed by measuring the target antibody titer in animal sera using conventional methods.

Antibody-producing cells obtained from lymphocytes or animals immunized with a desired antigen can be fused with myeloma cells to generate hybridomas using conventional fusing agents (for example, polyethylene glycol) (Goding, Monoclonal Antibodies: Principles and Practice, Academic Press, 1986, 59-103). When required, hybridoma cells can be cultured and grown, and the binding specificity of the antibody produced from these hybridomas can be measured using known analysis methods, such as immunoprecipitation, radioimmunoassay (RIA), and enzyme-linked immunosorbent assay (ELISA). Thereafter, hybridomas that produce antibodies of interest whose specificity, affinity, or activity has been determined can be subcloned by methods such as limiting dilution.

Next, genes encoding the selected antibodies can be cloned from hybridomas or antibody-producing cells (sensitized lymphocytes, and such) using probes that may specifically bind to the antibodies (for example, oligonucleotides complementary to sequences encoding the antibody constant regions). Cloning from mRNA using RT-PCR is also possible. Immunoglobulins are classified into five different classes, IgA, IgD, IgE, IgG, and IgM. These classes are further divided into several subclasses (isotypes) (for example, IgG-1, IgG-2, IgG-3, and IgG-4; IgA-1 and IgA-2; and such). Heavy chains and light chains used in the present invention to produce antibodies are not particularly limited and may derive from antibodies belonging to any of these classes or subclasses; however, IgG is particularly preferred.

Herein, it is possible to modify heavy-chain-encoding genes and light-chain-encoding genes using genetic engineering techniques. Genetically modified antibodies, such as chimeric antibodies, humanized antibodies that have been artificially modified for the purpose of decreasing heterologous antigenicity and such against humans, can be appropriately produced if necessary for antibodies such as mouse antibodies, rat antibodies, rabbit antibodies, hamster antibodies, sheep antibodies, and camel antibodies. Chimeric antibodies are antibodies composed of a nonhuman mammal antibody heavy chain and light chain variable regions, such as mouse antibody, and the heavy chain and light chain constant regions of human antibody. They can be obtained by ligating the DNA encoding a variable region of a mouse antibody to the DNA encoding a constant region of a human antibody, incorporating them into an expression vector, and introducing the vector into a host for production of the antibody. A humanized antibody, which is also called a reshaped human antibody, can be synthesized by PCR from a number of oligonucleotides produced so that they have overlapping portions at the ends of DNA sequences designed to link the complementary determining regions (CDRs) of an antibody of a nonhuman mammal such as a mouse. The obtained DNA can be ligated to a DNA encoding a human antibody constant region. The ligated DNA can be incorporated into an expression vector, and the vector can be introduced into a host to produce the antibody (see EP239400 and WO96/02576). Human antibody FRs that are ligated via the CDR are selected when the CDR forms a favorable antigen-binding site. If necessary, amino acids in the framework region of an antibody variable region may be substituted such that the CDR of the reshaped human antibody forms an appropriate antigen-binding site (K. Sato et al., Cancer Res. 1993, 53: 851-856). The monoclonal antibodies in context with the present invention include such humanized antibodies and chimeric antibodies.

When the antibodies in context with the present invention are chimeric antibodies or humanized antibodies, the constant regions of these antibodies are preferably derived from human antibodies. For example, Cγ1, Cγ2, Cγ3, and Cγ4 can be used for the heavy chain, while Cκ and Cλ can be used for the light chain. Furthermore, the human antibody constant region may be modified as necessary to improve antibody or its production stability. A chimeric antibody in context with the present invention preferably comprises a variable region of an antibody derived from a nonhuman mammal and a constant region of a human antibody. Meanwhile, a humanized antibody in context with the present invention preferably comprises CDRs of an antibody derived from a nonhuman mammal, and FRs and C regions of a human antibody. The constant regions derived from human antibodies comprise specific amino acid sequences, which vary depending on the isotype such as IgG (IgG1, IgG2, IgG3, and IgG4), IgM, IgA, IgD, and IgE. The constant regions used to prepare the humanized antibodies in context with the present invention may be constant regions of antibodies of any isotype. A constant region of human IgG is preferably used, but the constant regions are not limited thereto. Meanwhile, there is no particular limitation on the human antibody-derived FRs which are used to prepare humanized antibodies, and they may be derived from an antibody of any isotype.

The variable and constant regions of chimeric or humanized antibodies in context with the present invention may be modified by deletion, substitution, insertion, and/or addition, as long as the antibodies exhibit the same binding specificity as the original antibodies.

Chimeric and humanized antibodies that use human-derived sequences are expected to be useful when administered to humans for therapeutic purposes or such, since their antigenicity in the human body has been attenuated.

In the present disclosure, amino acids may be modified to alter the biological properties of an antibody.

Minibodies (low-molecular-weight antibodies) are useful as the antibodies because of their *in vivo* kinetic properties and low-cost production using *E. coli,* plant cells, or such.

Antibody fragments are one type of minibody. Minibodies include antibodies that comprise an antibody fragment as their partial structure. The minibodies in context with the present invention are not particularly limited by their structure or production method, as long as they have antigen-binding ability. Some minibodies have an activity greater than that of a whole antibody (Orita et al., Blood (2005) 105: 562-566). Herein, "antibody fragments" are not particularly limited as long as they are a portion of a whole antibody (for example, whole IgG). However, the antibody fragments preferably comprise a heavy chain variable region (VH) or a light chain variable region (VL). Preferred antibody fragments include, for example, Fab, F (ab')2, Fab', and Fv. The amino acid sequence of a heavy chain variable region (VH) or light chain variable region (VL) in an antibody fragment may be modified by substitution, deletion, addition, and/or insertion. Furthermore, some portions of a heavy chain variable region (VH) or light chain variable region (VL) may be deleted, as long as the fragments retain their antigen-binding ability. For example, of the above antibody fragments, "Fv" is a minimal antibody fragment that comprises the complete antigen recognition and binding sites. "Fv" is a dimer (VH-VL dimer) in which one heavy chain variable region (VH) and one light chain variable region (VL) are linked tightly by non-covalent bonding. The three complementarity determining regions (CDRs) of each variable region form an antigen-binding site on the surface of the VH-VL dimer. Six CDRs confer an antigen-binding site to the antibody. However, even one variable region (or half of an Fv comprising only three antigen-specific CDRs) has the ability to recognize and bind to an antigen, although its affinity is lower than that of the complete binding site. Thus, such molecules which are smaller than Fv are also included in the antibody fragments in context with the present invention. Furthermore, the variable regions of an antibody fragment may be chimerized or humanized.

It is preferable that the minibodies comprise both a heavy chain variable region (VH) and a light chain variable region (VL). The minibodies include, for example, antibody fragments such as Fab, Fab', F(ab')2, and Fv, and scFv (single-chain Fv) which can be prepared using antibody fragments (Huston et al., Proc. Natl. Acad. Sci. USA (1988) 85: 5879-83; Plickthun "The Pharmacology of Monoclonal Antibodies" Vol. 113, Resenburg and Moore (eds.), Springer Verlag, New York, pp. 269-315, (1994)); diabodies (Holliger et al., Proc. Natl. Acad. Sci. USA (1993) 90:6444-8; EP 404097; WO93/11161; Johnson et al., Method in Enzymology (1991) 203: 88-98; Holliger et al., Protein Engineering (1996) 9:299-305; Perisic et al., Structure (1994) 2:1217-26; John et al., Protein Engineering (1999) 12(7):597-604; Atwell et al., Mol. Immunol. (1996) 33:1301-12); sc(Fv)2 (Hudson et al., J Immunol. Methods (1999) 231:177-89; Orita et al., Blood (2005) 105:562-566); triabodies (Journal of Immunological Methods (1999) 231: 177-89); and tandem diabodies (Cancer Research (2000) 60:4336-41).

An antibody fragment can be prepared by treating an antibody with an enzyme, for example, a protease such as papain and pepsin (see Morimoto et al., J. Biochem. Biophys. Methods (1992) 24:107-17; Brennan et al., Science (1985) 229:81). Alternatively, an antibody fragment can also be produced by genetic recombination based on its amino acid sequence.

A minibody comprising a structure that results from modification of an antibody fragment can be constructed using an antibody fragment obtained by enzyme treatment or genetic recombination. Alternatively, after constructing a gene that encodes a whole minibody and introducing it into an expression vector, the minibody may be expressed in appropriate host cells (see, for example, Co et al., J. Immunol. (1994) 152:2968-76; Better and Horwitz, Methods Enzymol. (1989) 178:476-96; Pluckthun and Skerra, Methods Enzymol. (1989) 178: 497-515; Lamoyi, Methods Enzymol. (1986) 121:652-63; Rousseaux et al., Methods Enzymol. (1986) 121:663-9; Bird and Walker, Trends Biotechnol. (1991) 9:132-7).

The above scFv is a single-chain polypeptide comprising two variable regions linked together via a linker or such, as necessary. The two variable regions contained in an scFv are typically one VH and one VL, but an scFv may have two VH or two VL. In general, scFv polypeptides comprise a linker between the VH and VL domains, thereby forming a paired portion of VH and VL required for antigen binding. A peptide linker of ten or more amino acids is typically used as the linker between VH and VL for forming an intramolecularly paired portion between VH and VL. However, the linkers of the scFv in context with the present invention are not limited to such peptide linkers, as long as they do not inhibit scFv formation. To review scFv, see Pluckthun "The Pharmacology of Monoclonal Antibody", Vol. 113 (Rosenburg and Moore ed., Springer Verlag, NY, pp.269-315 (1994)).

Meanwhile, "diabodies (Db)" refers to divalent antibody fragments constructed by gene fusion (P. Holliger et al., Proc. Natl. Acad. Sci. USA 90: 6444-6448 (1993); EP 404,097; WO93/11161; etc.). Diabodies are dimers comprising two polypeptide chains, in which each polypeptide chain comprises within the same chain a light chain variable region (VL) and a heavy chain variable region (VH) linked via a linker short enough to prevent interaction of these two domains, for example, a linker of about five residues. VL and VH encoded on the same polypeptide chain will form a dimer because the linker between VL and VH is too short to form a single-chain V region fragment. Therefore, diabodies have two antigen-binding sites. In this case, when VL and VH directed against two different epitopes (a and b) are expressed simultaneously as combinations of VLa-VHb and VLb-VHa connected with a linker of about five residues, they are secreted as bispecific Db.

Diabodies comprise two molecules of scFv and thus have four variable regions. As a result, diabodies have two antigen binding sites. Unlike situations in which scFv does not form dimers, in diabody formation, the length of the linker between the VH and VL in each scFv molecule is generally about five amino acids when the linker is a peptide linker. However, the linker of scFv that forms a diabody is not limited to such a peptide linker, as long as it does not inhibit scFv expression and diabody formation.

Furthermore, it is preferable that minibodies and antibody fragments in context with the present invention additionally comprise an amino acid sequence of an antibody heavy chain constant region and/or an amino acid sequence of a light chain constant region.

### Alteration of one or more nucleotides

Herein, "alteration of nucleotides" means that gene manipulation or mutagenesis is performed to insert, delete, or substitute at least one nucleotide in a DNA so that the polypeptide encoded by the DNA has amino acid residues of interest. Specifically, this means that the codon encoding the original amino acid residue is substituted with a codon encoding the amino acid residue of interest. Such nucleotide alterations can be introduced using methods such as site-directed mutagenesis (see, for example, Kunkel (1985) Proc. Natl. Acad. Sci. USA 82: 488), PCR mutagenesis, and cassette mutagenesis. In general, mutant antibodies whose biological properties have been improved show an amino acid sequence homology and/or similarity of 70% or higher, more preferably 80% or higher, and even more preferably 90% or higher (for example, 95% or higher, 97%, 98%, 99%, etc.), when compared to the amino acid sequence of the original antibody variable region. Herein, the sequence homology and/or similarity is defined as the ratio of amino acid residues that are homologous (the same residue) or similar (amino acid residues classified into the same group based on the general properties of amino acid side chains) to the original amino acid residues, after maximizing the value of the sequence homology by performing sequence alignment and gap introduction as necessary. In general, naturally-occurring amino acid residues are classified into the following groups based on the characteristics of their side chains: (1) hydrophobic: alanine, isoleucine, valine, methionine, and leucine; (2) neutral hydrophilic: asparagine, glutamine, cysteine, threonine, and serine; (3) acidic: aspartic acid and glutamic acid; (4) basic: arginine, histidine, and lysine; (5) residues that have an influence on the chain conformation: glycine and proline; and (6) aromatic: tyrosine, tryptophan, and phenylalanine. The number of modified amino acids is, for example, ten, nine, eight, seven, six, five, four, three, two, or one, but is not limited thereto.

In general, a total of six complementarity determining regions (CDRs; hypervariable regions) present in the heavy chain and light chain variable regions interact to form the antigen binding site(s) of an antibody. It is known that one of these variable regions has the ability to recognize and bind to the antigen, even though the affinity will be lower than when all binding sites are included. Thus, polypeptides in context with the present invention having an antigen-binding activity may encode fragment portions containing the respective antigen binding sites of antibody heavy chain and light chain as long as they maintain the desired antigen-binding activity.

The methods of the present invention allow efficient preparation of, for example, desired polypeptide multimers that actually have the activity described above.

In a preferred embodiment of the present disclosure, appropriate amino acid residues to be "modified" can be selected from, for example, the amino acid sequences of antibody heavy chain and light chain variable regions and the amino acid sequences of antibody light chain and light chain variable region.

### DNA expression

DNAs encoding the modified polypeptides are cloned (inserted) into an appropriate vector and then introduced into host cells. There is no particular limitation on the vectors as long as they stably carry the inserted nucleic acids. For example, when using *E. coli* as the host, the vectors include cloning vectors. Preferred cloning vectors include pBluescript vectors (Stratagene). It is possible to use various commercially available vectors. Expression vectors are particularly useful as vectors for producing the polypeptide multimers or polypeptides in context with the present invention. There is no particular limitation on the expression vectors as long as they express polypeptides *in vitro,* in *E. coli,* culture cells, or organisms. Preferred vectors include, for example, pBEST vectors (Promega) for *in vitro* expression; pET vectors (Invitrogen) for expression in *E. coli;* the pME18S-FL3 vector (GenBank Accession No. AB009864) for expression in culture cells; and the pME18S vector (Mol. Cell. Biol. 8:466-472 (1988)) for expression in organisms. DNAs can be inserted into vectors by conventional methods such as ligase reaction using restriction enzyme sites (Current protocols in Molecular Biology edit. Ausubel et al. (1987) Publish. John Wiley & Sons. Section 11.4-11.11).

There is no particular limitation on the above host cells, and various host cells can be used depending on the purpose. Cells for expressing polypeptides include, for example, bacterial cells (e.g., Streptococcus, Staphylococcus, *E. coli,* Streptomyces, and Bacillus subtilis), fungal cells (e.g., yeast and Aspergillus), insect cells (e.g., Drosophila S2 and Spodoptera SF9), animal cells (e.g., CHO, COS, HeLa, C127, 3T3, BHK, HEK293, Bowes melanoma cell), and plant cells. Vectors can be introduced into host cells using known methods such as the calcium phosphate precipitation method, electroporation method (Current protocols in Molecular Biology edit. Ausubel et al. (1987) Publish. John Wiley & Sons. Section 9.1-9.9), lipofection method, and microinjection method.

In order to secrete host cell-expressed polypeptides into the lumen of the endoplasmic reticulum, periplasmic space, or extracellular environment, appropriate secretion signals can be incorporated into the polypeptides of interest. These signals may be intrinsic or foreign to the polypeptides of interest.

Expression vectors for the first, second, third, and fourth polypeptides can be constructed by inserting DNAs encoding the polypeptides individually into separate vectors. Alternatively, some of the DNAs encoding the first, second, third, and fourth polypeptides (for example, a DNA encoding the first polypeptide and a DNA encoding the second polypeptide) may be inserted into a single vector to construct expression vectors. When an expression vector is constructed by inserting multiple DNAs into a single vector, there is no limitation on the combination of polypeptide-encoding DNAs to be inserted.

### Recovery of expression products

When polypeptides are secreted to a culture medium, the expression products are recovered by collecting the medium. When polypeptides are produced in cells, the cells are first lysed and then the polypeptides are collected.

The polypeptides can be collected and purified from a culture of recombinant cells by known methods including ammonium sulfate or ethanol precipitation, acid extraction, anion or cation exchange chromatography, phosphocellulose chromatography, hydrophobic interaction chromatography, affinity chromatography, hydroxylapatite chromatography, and lectin chromatography.

Protein A affinity chromatography is used in the present invention.

Protein A columns include, but are not limited to, Hyper D (PALL), POROS (Applied Biosystems), Sepharose F.F. (GE), and ProSep (Millipore). Alternatively, protein A affinity chromatography can be performed using a resin bound by a ligand that mimics the IgG-binding ability of protein A. Also when such a protein A mimic is used, polypeptide multimers of interest can be isolated and purified by creating a difference in the binding ability as a result of the amino acid modifications in context with the present invention. Such protein A mimics include, but are not limited to, for example, mabSelect SuRE (GE Healthcare).

Furthermore, the present invention provides polypeptide multimers obtained by the production or purification methods of the present invention.

The present invention also contemplates polypeptide multimers comprising the first polypeptide having an antigen-binding activity and the second polypeptide having an antigen-binding activity or no antigen-binding activity, wherein the protein A-binding ability is different between the first and second polypeptides.

Such polypeptide multimers can be obtained by the methods described herein. The structures and properties of the polypeptide multimers are as described above, and summarized below.

As compared to before modification of amino acids, the protein A-binding ability of the polypeptide multimers in context with the present invention has been altered. More specifically, the protein A-binding ability has been altered in either or both of the first polypeptide having an antigen-binding activity and the second polypeptide having an antigen-binding activity or no antigen-binding activity. In a polypeptide multimer in context with the present invention, the protein A-binding ability of the first polypeptide having an antigen-binding activity is different from that of the second polypeptide having an antigen-binding activity or no antigen-binding activity. Accordingly, the solvent pH for protein A elution is different for the first polypeptide and the second polypeptide in affinity chromatography.

Furthermore, the first polypeptide and/or the second polypeptide can form a multimer with one or two third polypeptides.

Thus, the present invention contemplates polypeptide multimers that comprise the first polypeptide having an antigen-binding activity, the second polypeptide having an antigen-binding activity or no antigen-binding activity, and one or two third polypeptides having an antigen-binding activity, wherein the protein A-binding ability is different for the first and second polypeptides. Such polypeptide multimers can also be obtained by the methods described herein.

The polypeptide multimers may additionally comprise a fourth polypeptide. Either one of the first polypeptide and the second polypeptide can form a multimer with the third polypeptide, while the other can form another multimer with the fourth polypeptide.

Thus, the present invention contemplates polypeptide multimers that comprise the first polypeptide having an antigen-binding activity, the second polypeptide having an antigen-binding activity or no antigen-binding activity, the third polypeptide having an antigen-binding activity, and the fourth polypeptide having an antigen-binding activity, wherein the protein A-binding ability is different for the first and second polypeptides. Such polypeptide multimers can also be obtained by the methods described herein.

The above first polypeptide having an antigen-binding activity and second polypeptide having an antigen-binding activity or no antigen-binding activity may comprise an amino acid sequence of an antibody heavy chain constant region or an amino acid sequence of an antibody Fc domain. The amino acid sequence of an antibody heavy chain constant region or an antibody Fc domain includes, but is not limited to, an amino acid sequence of a human IgG-derived constant region.

Meanwhile, the above third polypeptide having an antigen-binding activity and fourth polypeptide having an antigen-binding activity may comprise an amino acid sequence of an antibody light chain constant region.

Furthermore, the polypeptides having an antigen-binding activity may comprise an amino acid sequence of an antibody variable region (for example, amino acid sequences of CDR1, CDR2, CDR3, FR1, FR2, FR3, and FR4).

The above first polypeptide having an antigen-binding activity and second polypeptide having an antigen-binding activity or no antigen-binding activity may comprise an amino acid sequence of an antibody heavy chain, or an amino acid sequence comprising an antibody light chain variable region and an antibody heavy chain constant region. The above third polypeptide having an antigen-binding activity and fourth polypeptide having an antigen-binding activity may comprise an amino acid sequence of an antibody light chain, or an amino acid sequence comprising an antibody heavy chain variable region and an antibody light chain constant region.

A polypeptide multimer in context with the present invention can be a multispecific antibody. Multispecific antibodies in context with the present invention include, but are not limited to, bispecific antibodies capable of specifically binding to two types of antigens.

In a polypeptide multimer in context with the present disclosure, one or more amino acid residues have been modified so that there is a (larger) difference of protein A-binding ability between the first polypeptide having an antigen-binding activity and the second polypeptide having an antigen-binding activity or no antigen-binding activity. As described above, the modification sites include, but are not limited to, for example, the following amino acid residues: TLMISR at positions 250-255, VLHQDWLNGK at positions 308-317, EALHNHY at positions 430-436, preferably TLMIS at positions 250-254, LHQD at positions 309-312, LN at positions 314-315, E at position 430, LHNHY at positions 432-436, more preferably LMIS at positions 251-254, LHQ at positions 309-311, L at position 314, LHNH at positions 432-435, and particularly LMIS at positions 252-254, L at position 309, Q at position 311, and NHY at positions 434-436 (EU numbering) in an antibody Fc domain or a heavy chain constant region. Meanwhile, as for amino acid modifications of an antibody heavy chain variable region, preferred modification sites include FR1, CDR2, and FR3.

More specifically, the polypeptide multimers of the present disclosure, include, but are not limited to, polypeptide multimers in which the amino acid residue at position 435 (EU numbering) in the amino acid sequence of an antibody Fc domain or antibody heavy chain constant region is histidine or arginine in either one of the first polypeptide having an antigen-binding activity and the second polypeptide having an antigen-binding activity or no antigen-binding activity, while the other polypeptide has a different amino acid residue at position 435 (EU numbering) in the amino acid sequence of an antibody Fc domain or antibody heavy chain constant region.

In the methods of the present invention, the amino acid residue at position 435 (EU numbering) in the amino acid sequence of the Fc domain or heavy-chain constant region is histidine or arginine in the first polypeptide having an antigen-binding activity and arginine or histidine respectively in the second polypeptide having or not having an antigen-binding activity.

Furthermore, the polypeptide multimers in context with the present invention include, but are not limited to, polypeptide multimers in which the amino acid residue at position 435 (EU numbering) in the amino acid sequence of an antibody heavy chain constant region is histidine in either one of the first polypeptide having an antigen-binding activity and the second polypeptide having an antigen-binding activity or no antigen-binding activity, while the amino acid residue at position 435 (EU numbering) in the amino acid sequence of an antibody heavy chain constant region is arginine in the other polypeptide.

Furthermore, the polypeptide multimers of the present disclosure, comprising the first and second polypeptides include, but are not limited to the examples below.
(1) Polypeptide multimers that comprise the first or second polypeptide comprising an amino acid sequence in which the amino acid residues at positions 435 and 436 (EU numbering) in the amino acid sequence of an antibody heavy chain constant region derived from a human IgG have been modified to histidine (His) and tyrosine (Tyr), respectively.
   Such polypeptide multimers include, but are not limited to, for example, polypeptide multimers that comprise the first or second polypeptide comprising the amino acid sequence of SEQ ID NO: 9, 11, 13, or 15.
(2) Polypeptide multimers that comprise the first or second polypeptide comprising an amino acid sequence in which the amino acid residues at positions 435 and 436 (EU numbering) in the amino acid sequence of an antibody heavy chain constant region derived from a human IgG have been modified to arginine (Arg) and phenylalanine (Phe), respectively.
   Such polypeptide multimers include, but are not limited to, for example, polypeptide multimers that comprise the first or second polypeptide comprising the amino acid sequence of SEQ ID NO: 10 or 12.
(3) Polypeptide multimers that comprise the first or second polypeptide comprising an amino acid sequence in which the amino acid residues at positions 435 and 436 (EU numbering) in the amino acid sequence of an antibody heavy chain constant region derived from a human IgG have been modified to arginine (Arg) and tyrosine (Tyr), respectively.
   Such polypeptide multimers include, but are not limited to, for example, polypeptide multimers that comprise the first or second polypeptide comprising the amino acid sequence of SEQ ID NO: 14.
(4) Polypeptide multimers that comprise the first and second polypeptides, wherein either one of the polypeptides comprises an amino acid sequence in which the amino acid residues at positions 435 and 436 (EU numbering) in the amino acid sequence of an antibody heavy chain constant region derived from a human IgG have been modified to histidine (His) and tyrosine (Tyr), respectively; and the other polypeptide comprises an amino acid sequence in which the amino acid residues at positions 435 and 436 (EU numbering) in the amino acid sequence of an antibody heavy chain constant region have been modified to arginine (Arg) and phenylalanine (Phe), respectively.
   Such polypeptide multimers include, but are not limited to, for example, polypeptide multimers that comprise the first polypeptide comprising the amino acid sequence of SEQ ID NO: 9, 11, 13, or 15 and the second polypeptide comprising the amino acid sequence of SEQ ID NO: 10 or 12.
(5) Polypeptide multimers that comprise the first and second polypeptides, wherein either one of the polypeptides comprises an amino acid sequence in which the amino acid residues at positions 435 and 436 (EU numbering) in the amino acid sequence of an antibody heavy chain constant region derived from a human IgG have been modified to histidine (His) and tyrosine (Tyr), respectively; and the other polypeptide comprises an amino acid sequence in which the amino acid residues at positions 435 and 436 (EU numbering) in the amino acid sequence of an antibody heavy chain constant region have been modified to arginine (Arg) and tyrosine (Tyr), respectively.
   Such polypeptide multimers include, but are not limited to, for example, polypeptide multimers that comprise the first polypeptide comprising the amino acid sequence of SEQ ID NO: 9, 11, 13, or 15 and the second polypeptide comprising the amino acid sequence of SEQ ID NO: 14.
(6) Polypeptide multimers that comprise the first and second polypeptides, wherein either one of the polypeptides comprises an amino acid sequence in which the amino acid residues at positions 435 and 436 (EU numbering) in the amino acid sequence of an antibody heavy chain constant region derived from a human IgG have been modified to arginine (Arg) and phenylalanine (Phe), respectively; and the other polypeptide comprises an amino acid sequence in which the amino acid residues at positions 435 and 436 (EU numbering) in the amino acid sequence of an antibody heavy chain constant region have been modified to arginine (Arg) and tyrosine (Tyr), respectively.
   Such polypeptide multimers include, but are not limited to, for example, polypeptide multimers that comprise the first polypeptide comprising the amino acid sequence of SEQ ID NO: 10 or 12 and the second polypeptide comprising the amino acid sequence of SEQ ID NO: 14.

The above first and second polypeptides may additionally comprise an antibody heavy chain variable region. The polypeptide multimers of (1) to (6) above may also comprise the third polypeptide and/or the fourth polypeptide.

Furthermore, the present invention contemplates polypeptide variants that comprise a polypeptide comprising a mutation in the amino acid residue at either position 435 or 436 (EU numbering). Such polypeptide variants include, but are not limited to, polypeptide variants comprising a polypeptide described in the Examples.

Furthermore, the present invention contemplates nucleic acids encoding a polypeptide (polypeptide having an antigen-binding activity) that constitutes a polypeptide multimer in context the present invention. The present invention also contemplates vectors carrying such nucleic acids.

The present invention also contemplates host cells comprising the above nucleic acids or vectors. There is no particular limitation on the host cells, and they include, for example, *E*. *coli* and various plant and animal cells. The host cells may be used, for example, as a production system for producing and expressing the polypeptide multimers or polypeptides in context with the present invention. There are *in vitro* and *in vivo* production systems for producing the polypeptide multimers or polypeptides. *In vitro* production systems include those using eukaryotic cells and prokaryotic cells.

Eukaryotic cells that can be used as host cells include, for example, animal cells, plant cells, and fungal cells. Animal cells include: mammalian cells, for example, CHO (J. Exp. Med. (1995) 108, 945), COS, HEK293, 3T3, myeloma, BHK (baby hamster kidney), HeLa, and Vero; amphibian cells such as *Xenopus laevis* oocytes (Valle, et al., Nature (1981) 291: 338-340); and insect cells such as Sf9, Sf21, and Tn5. For expressing the polypeptide multimers or polypeptides in context with the present invention, CHO-DG44, CHO-DX11B, COS7 cells, HEK293 cells, and BHK cells can be suitably used. Of the animal cells, CHO cells are particularly preferable for large-scale expression. Vectors can be introduced into a host cell by, for example, calcium phosphate methods, DEAE-dextran methods, methods using cationic liposome DOTAP (Boehringer-Mannheim), electroporation methods, or lipofection methods.

It is known that plant cells such as *Nicotiana tabacum*-derived cells and *Lemna minor* cells are protein production systems, and these cells can be used to produce polypeptide multimers or polypeptides in context with the present invention by methods that culture calluses from these cells. Protein expression systems that use fungal cells including yeast cells, for example, cells of the genus Saccharomyces (*Saccharomyces cerevisiae, Saccharomyces pombe,* etc.), and cells of filamentous fungi, for example, the genus Aspergillus (*Aspergillus niger,* etc.) are known, and these cells can be used as a host to produce polypeptide multimers or polypeptides in context with the present invention.

When prokaryotic cells are used, production systems that use bacterial cells are available. Production systems that use bacterial cells including *Bacillus subtilis* as well as *E*. *coli* described above are known, and they can be used to produce polypeptide multimers or polypeptides in context with the present invention.

When a polypeptide multimer or polypeptide is produced using a host cell contemplated in the present invention, a polynucleotide encoding the polypeptide multimer or polypeptide in context with the present invention may be expressed by culturing the host cell transformed with an expression vector comprising the polynucleotide. Culturing can be performed according to known methods. For example, when animal cells are used as a host, DMEM, MEM, RPMI 1640, or IMDM may be used as the culture medium. The culture medium may be used with serum supplement solutions such as FBS or fetal calf serum (FCS). Alternatively, cells can be cultured in serum-free cultures. The preferred pH is about 6 to 8 during the course of culturing. Incubation is carried out typically at about 30 to 40°C for about 15 to 200 hours. Medium is exchanged, aerated, or agitated, as necessary.

On the other hand, systems for producing polypeptides *in vivo* include, for example, those using animals and those using plants. A polynucleotide of interest is introduced into an animal or plant to produce the polypeptide in the body of the animal or the plant, and then the polypeptide is collected. The "host" contemplated in the present invention includes such animals and plants.

When animals are used, production systems that use mammals or insects are available. Mammals such as goat, pig, sheep, mouse, and cattle may be used (Vicki Glaser, SPECTRUM Biotechnology Applications (1993)). When mammals are used, transgenic animals may be used.

For example, a polynucleotide encoding a polypeptide multimer or polypeptide in context with the present invention may be prepared as a fusion gene with a gene encoding a polypeptide specifically produced in milk, such as goat β-casein. Next, polynucleotide fragments containing this fusion gene are injected into goat embryos, which are then introduced back into female goats. The antibody of interest can be obtained from milk produced by the transgenic goats, which are born from the goats that received the embryos, or by their offspring. Appropriate hormones may be administered to the transgenic goats to increase the volume of milk containing the antibody produced by the transgenic goats (Ebert et al., Bio/Technology (1994) 12: 699-702).

Insects such as silkworms may be used for producing polypeptide multimers or polypeptides in context with the present invention. When silkworms are used, baculo viruses carrying a polynucleotide encoding a polypeptide multimer or polypeptide of interest can be used to infect silkworms, so that the polypeptide multimer or polypeptide of interest can be obtained from the body fluids of these silkworms (Susumu et al., Nature (1985) 315:592-594).

Plants used for producing polypeptide multimers or polypeptides in context with the present invention include, for example, tobacco. When tobacco is used, a polynucleotide encoding a polypeptide multimer or polypeptide of interest is inserted into a plant expression vector, for example, pMON 530, and then the vector is introduced into a bacterium such as *Agrobacterium tumefaciens.* The bacteria are then used to infect tobacco such as *Nicotiana tabacum,* and the desired polypeptide multimer or polypeptide can be obtained from the leaves of the tobacco (Ma et al., Eur. J. Immunol. (1994) 24: 131-138). Alternatively, the same bacteria can be used to infect *Lemna minor,* and after cloning, the desired polypeptide multimer or polypeptide can be obtained from the cells of *Lemna minor* (Cox K.M. et al., Nat. Biotechnol. 2006 Dec; 24(12):1591-1597).

The polypeptide multimer or polypeptide thus obtained may be isolated from the inside or outside (such as the medium and milk) of host cells, and purified as a substantially pure and homogenous polypeptide multimer or polypeptide. Methods used for separating and purifying a polypeptide multimer or polypeptide are not limited, and methods used in standard polypeptide purification may be applied. Antibodies may be isolated and purified by selecting an appropriate combination of, for example, chromatographic columns, filtration, ultrafiltration, salting-out, solvent precipitation, solvent extraction, distillation, immunoprecipitation, SDS-polyacrylamide gel electrophoresis, isoelectric focusing, dialysis, recrystallization, and such.

Chromatographies include, for example, affinity chromatography, ion exchange chromatography, hydrophobic chromatography, gel filtration, reverse-phase chromatography, and adsorption chromatography (Strategies for Protein Purification and Characterization: A Laboratory Course Manual. Ed Daniel R. Marshak et al., (1996) Cold Spring Harbor Laboratory Press). These chromatographies can be carried out using liquid phase chromatography such as HPLC and FPLC. Examples of columns for affinity chromatography include protein A columns and protein G columns. Examples of the columns that use protein A include, but are not limited to, Hyper D, POROS, and Sepharose F. F. (Pharmacia).

As necessary, modifications can be added and peptides can be deleted from a polypeptide multimer or polypeptide arbitrarily by treatment with an appropriate protein modification enzyme before or after purification of the polypeptide multimer or polypeptide. Such protein modification enzymes include, for example, trypsin, chymotrypsin, lysyl endopeptidase, protein kinase, and glucosidase.

Another preferred embodiment of the present invention includes a respective method for producing a polypeptide multimer or polypeptide of the present invention, which comprises the steps of culturing the host cells of the present invention as described above and collecting the polypeptide from the cell culture.

Furthermore, the present disclosure contemplates pharmaceutical compositions (agents) comprising a polypeptide multimer or polypeptide of the present disclosure and a pharmaceutically acceptable carrier. Herein, "pharmaceutical compositions" generally refers to agents for treating or preventing, or testing and diagnosing diseases.

The pharmaceutical compositions contemplated in the present disclosure can be formulated by methods known to those skilled in the art. For example, such pharmaceutical compositions can be used parenterally in the form of injections, which are sterile solutions or suspensions prepared with water or another pharmaceutically acceptable liquid. For example, such compositions may be formulated by appropriately combining with a pharmaceutically acceptable carrier or medium, specifically, sterile water, physiological saline, vegetable oil, emulsifier, suspension, surfactant, stabilizer, flavoring agent, excipient, vehicle, preservative, binder, or such, and mixed in a unit dose form that meets the generally accepted requirements for preparation of pharmaceuticals. In such preparations, the amount of active ingredient is adjusted such that a suitable amount within a specified range is obtained.

Sterile compositions for injection can be formulated using vehicles such as distilled water for injection, according to standard protocols for formulation.

Aqueous solutions for injection include, for example, physiological saline and isotonic solutions containing glucose or other adjuvants (for example, D-sorbitol, D-mannose, D-mannitol, and sodium chloride). Appropriate solubilizers, for example, alcohols (ethanol and such), polyalcohols (propylene glycol, polyethylene glycol, and such), non-ionic surfactants (polysorbate 80™, HCO-50, and such) may be used in combination.

Oils include sesame and soybean oils. Benzyl benzoate and/or benzyl alcohol can be used as solubilizers in combination. Buffers (for example, phosphate buffer and sodium acetate buffer), soothing agents (for example, procaine hydrochloride), stabilizers (for example, benzyl alcohol and phenol), and/or antioxidants can also be combined. Prepared injections are generally filled into appropriate ampules.

The pharmaceutical compositions contemplated in the present disclosure are preferably administered parenterally. For example, the compositions may be in the form of injections, transnasal agents, transpulmonary agents, or transdermal agents. For example, such compositions can be administered systemically or locally by intravenous injection, intramuscular injection, intraperitoneal injection, subcutaneous injection, or such.

The administration methods can be appropriately selected in consideration of a patient's age and symptoms. The dosage of a pharmaceutical composition comprising a polypeptide multimer or polypeptide or a polynucleotide encoding a polypeptide multimer or polypeptide may be set, for example, within the range of 0.0001 to 1000 mg/kg weight for each administration. Alternatively, the dosage may be, for example, from 0.001 to 100,000 mg per patient. However, herein, the dosage is not necessarily limited to the ranges described above. Although the dosage and administration method vary depending on a patient's weight, age, symptoms, and such, those skilled in the art can select appropriate dosage and administration methods in consideration of these factors.

The multispecific antibodies in context with the present invention can be formulated by combining them with other pharmaceutical components as necessary.

### Examples

Hereinbelow, the present invention will be specifically described with reference to the Examples, but it is not to be construed as being limited thereto.

### [Example 1] Construction of expression vectors for antibody genes and expression of respective antibodies

The antibody H chain variable regions used were:
Q153 (the H chain variable region of an anti-human F.IX antibody, SEQ ID NO: 1), Q407 (the H chain variable region of an anti-human F.IX antibody, SEQ ID NO: 2), J142 (the H chain variable region of an anti-human F.X antibody, SEQ ID NO: 3), J300 (the H chain variable region of an anti-human F.X antibody, SEQ ID NO: 4), and MRA-VH (the H chain variable region of an anti-human interleukin-6 receptor antibody, SEQ ID NO: 5).

The antibody L chain variable regions used were:
L180-k (an L chain common to an anti-human F.IX antibody and an anti-human F.X antibody, SEQ ID NO: 6), L210-k (an L chain common to an anti-human F.IX antibody/anti-human F.X antibody, SEQ ID NO: 7), and MRA-k (the L chain of an anti-human interleukin-6 receptor antibody, SEQ ID NO: 8).

The antibody H chain constant regions used were:
G4d (SEQ ID NO: 9), which was constructed from IgG4 by introducing a substitution mutation of Pro for Ser at position 228 (EU numbering) and deleting the C-terminal Gly and Lys; z72 (SEQ ID NO: 10), which was constructed from G4d by introducing the following mutations: a substitution mutation of Arg for His at position 435 (EU numbering); a substitution mutation of Phe for Tyr at position 436 (EU numbering); and a substitution mutation of Pro for Leu at position 445 (EU numbering); z7 (SEQ ID NO: 11), which was constructed from G4d by introducing a substitution mutation of Lys for Glu at position 356 (EU numbering); z73 (SEQ ID NO: 12), which was constructed from z72 by introducing a substitution mutation of Glu for Lys at position 439 (EU numbering); z106 (SEQ ID NO: 13), which was constructed from z7 by introducing the following mutations: a substitution mutation of Gln for Lys at position 196 (EU numbering); a substitution mutation of Tyr for Phe at position 296 (EU numbering); and a substitution mutation of Lys for Arg at position 409 (EU numbering); z107 (SEQ ID NO: 14), which was constructed from z73 by introducing the following mutations: a substitution mutation of Gln for Lys at position 196 (EU numbering); a substitution mutation of Tyr for Phe at position 296 (EU numbering); a substitution mutation of Lys for Arg at position 409 (EU numbering); and a substitution mutation of Tyr for Phe at position 436 (EU numbering); and G1d (SEQ ID NO: 15), which was constructed by deleting the C-terminal Gly and Lys from IgG1. Substitution mutations of Lys for Glu at position 356 (EU numbering) and Glu for Lys at position 439 (EU numbering) were introduced for efficient formation of heteromeric molecules from the respective H chains in the production of heteromeric antibodies ((WO 2006/106905) PROCESS FOR PRODUCTION OF POLYPEPTIDE BY REGULATION OF ASSEMBLY).

The anti-human F.IX antibody H chain genes Q153-G4d and Q153-z7 were constructed by linking respectively G4d and z7 downstream of Q153. The anti-human F.IX antibody H chain gene Q407-z106 was constructed by linking z106 downstream of Q407. The anti-human F.X antibody H chain genes J142-G4d, J142-z72, and J142-z73 were constructed by linking respectively G4d, z72, and z73 downstream of J142. The anti-human F.X antibody H chain gene J300-z107 was constructed by linking z107 downstream of J300. The anti-human interleukin-6 receptor antibody H chain genes MRA-G1d, MRA-z106, and MRA-z107 were constructed by linking respectively G1d, z106, and z107 downstream of MRA-VH.

The respective antibody genes (Q153-G4d, Q153-z7, Q407-z106, J142-G4d, J142-z72, J142-z73, J300-z106, MRA-G1d, MRA-z106, MRA-z107, L180-k, L210-k, and MRA-k) were inserted into animal cell expression vectors.

The following antibodies were expressed transiently in FreeStyle293 cells (Invitrogen) by transfection using the constructed expression vectors. As shown below, antibodies were named using the combinations of transfected antibody genes.
MRA-G1d/MRA-k
MRA-z106/MRA-z107/MRA-k
Q153-G4d/J142-G4d/L180-k
Q153-G4d/J142-z72/L180-k
Q153-z7/J142-z73/L180-k
Q407-z106/J300-z107/L210-k

### [Example 2] Assessment of the elution conditions for protein A affinity chromatography

Q153-G4d/J142-G4d/L180-k and Q153-G4d/J142-z72/L180-k were expressed transiently, and the medium of the resulting FreeStyle293 cell culture (hereinafter abbreviated as CM) was used as a sample for assessing the elution conditions for protein A affinity chromatography. The CM samples were filtered through a filter with a pore size of 0.22 µm, and loaded onto an rProtein A Sepharose Fast Flow column (GE Healthcare) equilibrated with D-PBS. The column was subjected to washes 1 and 2 and elutions 1 to 5 in a stepwise manner as shown in Table 1. The volume of CM to be loaded onto the column was adjusted to 20 mg antibody/ml resin. Fractions eluted under each condition were collected, and the respective eluted fractions were analyzed by cation exchange chromatography to identify their components. To prepare controls, each CM was loaded onto rProtein G Sepharose Fast Flow resin (GE Healthcare). Samples purified by batchwise elution were used as controls. Since protein G binds to the Fab domain of an antibody, all antibody species (a bispecific antibody of interest in which two types of H chains are associated in a heteromeric manner (heteromeric antibody) and as an impurity monospecific homomeric antibodies in which single-type H chains are homomerically associated) in CM can be purified by using protein G, regardless of their protein A-binding affinity.

**[Table 1]**

| **Equilibration** | D-PBS |
|---|---|
| **Wash** 1 | 400 mM Arg-HCl/ D-PBS |
| **Wash** 2 | 20 mM NaCitrate, pH5.0 |
| **Elution** 1 | 20 mM NaCitrate, pH4.0 |
| **Elution** 2 | 20 mM NaCitrate, pH3.8 |
| **Elution** 3 | 20 mM NaCitrate, pH3.6 |
| **Elution** 4 | 20 mM NaCitrate, pH3.4 |
| **Elution** 5 | 20 mM NaCitrate, pH3.2 |

CM in which Q153-G4d/J142-G4d/L180-k or Q153-G4d/J142-z72/L180-k had been expressed was eluted from a protein A column (elution 1 to 5), and the respective eluted fractions were analyzed by cation exchange chromatography. As for Q153-G4d/J142-G4d/L180-k, the analysis revealed that as the elution condition was altered from 1 to 5, i.e., as the pH of the elution buffer was reduced, the antibody composition of the eluted fractions changed gradually in the order from the homomeric antibody J142-G4d/L180-k to the heteromeric antibody

Q153-G4d/J142-G4d/L180-k, and then to the homomeric antibody Q153-G4d/L180-k. The order of elution is understood to be in accordance with the binding ability for protein A. This implies that the homomeric antibody Q153-G4d/L180-k, which remained bound until exposed to low pH, has a greater binding ability for protein A than the homomeric species J142-G4d/L180-k (a homomeric antibody against FX) eluted at a high pH. It is known that the variable region J142 is a sequence incapable of binding to protein A. Specifically, the homomeric species J142-G4d/L180-k (a homomeric antibody against FX) has two protein A-binding sites; the heteromeric antibody Q153-G4d/J142-G4d/L180-k has three; and the homomeric antibody Q153-G4d/L180-k (homomeric antibody against FX) has four protein A-binding sites. Thus, it was revealed that more protein A-binding sites resulted in stronger protein A binding, and thus a lower pH was required for elution.

Meanwhile, as for Q153-G4d/J142-z72/L180-k, it was revealed that as the elution condition was altered from 1 to 5, the antibody composition in the eluted fraction changed from the heteromeric antibody Q153-G4d/J142-z72/L180-k to the homomeric antibody Q153-G4d/L180-k. The homomeric antibody J142-z72/L180-k (a homomeric antibody against FX) was almost undetectable in any eluted fractions. This suggests that J142-z72/L180-k has no protein A-binding ability. It is thought that the lack of protein A-binding ability of J142-z72 might be due to the introduced substitution mutation of Arg for His at position 435 (EU numbering). The homomeric antibody J142-z72/L180-k (a homomeric antibody against FX) has no protein A-binding site, while the heteromeric antibody Q153-G4d/J142-z72/L180-k has two protein A-binding sites and the homomeric antibody Q153-G4d/L180-k (a homomeric antibody against FIX) has four. The homomeric antibody J142-z72/L180-k (a homomeric antibody against FX) passes through the column because it does not bind to protein A. This is the reason why J142-z72/L180-k was undetectable in any eluted fractions. Furthermore, in both cases of Q153-G4d/J142-G4d/L180-k and Q153-G4d/J142-z72/L180-k, it was suggested that the heteromeric antibody and homomeric antibody Q153-G4d/L180-k (a homomeric antibody against FIX) were separable from each other at pH 3.6 or a lower pH.

### [Example 3] Isolation and purification of heteromeric antibodies by protein A chromatography

CM samples containing the following antibodies were used:
Q153-G4d/J142-G4d/L180-k
Q153-G4d/J142-z72/L180-k
Q153-z7/J142-z73/L180-k
Q407-z106/J300-z107/L210-k
The CM samples were filtered through a filter with a pore size of 0.22 µm, and loaded onto an rProtein A Sepharose Fast Flow column (GE Healthcare) equilibrated with D-PBS. The column was subjected to washes 1 and 2 and elutions 1 and 2 as shown in Table 2 (except that Q407-z106/J300-z107/L210-k was subjected to elution 1 only). The elution conditions were determined based on the result described in Example 2. The volume of CM to be loaded onto the column was adjusted to 20 mg antibody/ml resin. Respective fractions eluted under each condition were collected and analyzed by cation exchange chromatography to identify their components. To prepare controls, each CM was loaded onto rProtein G Sepharose Fast Flow resin (GE Healthcare) in the same manner as described in Example 2. Samples purified by batchwise elution were used as controls.

**[Table 2]**

| **Equilibration** | D-PBS |
|---|---|
| **Wash** 1 | 400 mM Arg-HCl/ D-PBS |
| **Wash** 2 | 20 mM NaCitrate, pH5.0 |
| **Elution** 1 | 20 mM NaCitrate, pH3.6 |
| **Elution** 2 | 20 mM NaCitrate, pH2.7 |

The result of cation exchange chromatography analysis for each eluted fraction is shown in Table 3 below. The values represent the area of elution peak expressed in percentage. Except for the Q153-G4d/J142-G4d/L180-k antibody, homomeric antibodies against FX were almost undetectable in any fractions eluted. Thus, it was revealed that not only the homomeric antibody J142-z72 (a homomeric antibody against FX) described in Example 2 but also the homomeric antibodies J142-z73 and J300-z107 (a homomeric antibody against FX) were incapable of binding to protein A. It is thought that the lack of protein A-binding ability in the homomeric antibody against FX was due to the substitution mutation of Arg for His at position 435 (EU numbering), which was introduced into the H chain constant region of the antibody against FX. The heteromeric antibody, which is a bispecific antibody of interest, was detected mostly in the fraction of elution 1. Meanwhile, the majority of homomeric antibodies against FIX were eluted by elution 2, although they were also detected at a very low level in the fraction of elution 1. As compared to Q153-G4d/J142-z72/L180-k, in the cases of Q153-z7/J142-z73/L180-k and Q407-z106/J300-z107/L210-k, the proportion of the heteromeric antibody (bispecific antibody of interest) was considerably increased in the fraction eluted at pH 3.6. Thus, it was demonstrated that when the substitution mutations of Lys for Glu at position 356 (EU numbering) and of Glu for Lys at position 439 (EU numbering) for efficient formation of heteromeric molecules from the respective H chains were introduced in combination with the substitution mutation of Arg for His at position 435 (EU numbering), the heteromeric antibody (bispecific antibody of interest) could be purified to a purity of 98% or higher through the protein A-based purification step alone.

As described above, the present inventors revealed that based on differences in the number of protein A-binding sites between the heteromeric antibody and homomeric antibodies, the heteromeric antibody could be isolated and purified to high purity through the protein A chromatography step alone.

**[Table 3]**

| Q153-G4d/J142-G4d/L180-k | | | |
|---|---|---|---|
| **Peak area** (%) | **Control** | **Fraction eluted at pH 3.6** | **Fraction eluted at pH 2.7** |
| J142-G4d/L180-k | 17.6 | 27.5 | - |
| Q153-G4d/J142-G4d/L180-k | 48.3 | 58.4 | 9.0 |
| Q153-G4d/L180-k | 34.1 | 14.1 | 91.0 |

**[Table 4]**

| Q153-G4d/J142-z72/L180-k | | | |
|---|---|---|---|
| **Peak area** (%) | **Control** | **Fraction eluted at pH 3.6** | **Fraction eluted at pH 2.7** |
| J142-z72/L180-k | 8.4 | 0.9 | - |
| Q153-G4d/J142-z72/L180-k | 50.8 | 81.0 | 2.2 |
| Q153-G4d/L180-k | 40.8 | 18.1 | 97.8 |

**[Table 5]**

| Q153-z7/J142-z73/L180-k | | | |
|---|---|---|---|
| **Peak area** (%) | **Control** | **Fraction eluted at pH 3.6** | **Fraction eluted at pH 2.7** |
| J142-z73/L180-k | 3.2 | - | - |
| Q153-z7/J142-z73/L180-k | 90.7 | 98.1 | 2.7 |
| Q153-z7/L180-k | 6.1 | 1.9 | 97.3 |

**[Table 6]**

| Q407-z106/J300-z107/L210-k | | | |
|---|---|---|---|
| **Peak area** (%) | **Control** | **Fraction eluted at pH 3.6** | **Fraction eluted at pH 2.7** |
| J300-z107/L210-k | 5.8 | - | |
| Q407-z106/J300-z107/L210-k | 84.6 | 98.9 | |
| Q407-z106/L210-k | 9.7 | 1.1 | |

### [Example 4] Assessment of pharmacokinetics in human FcRn transgenic mice

As described in Example 3 above, the present inventors demonstrated that by using z106 (SEQ ID NO: 13) and z107 (SEQ ID NO: 14) for the respective H chain constant regions of the bispecific antibody, the heteromeric antibody (bispecific antibody of interest) could be purified to a purity of 98% or higher through the protein A step alone. Meanwhile, the loss of protein A-binding affinity probably results in loss of human FcRn-binding activity because protein A and human FcRn recognize the same site in an IgG antibody (J Immunol. 2000, 164(10):5313-8). Actually, there is a reported method for purifying a bispecific antibody to a purity of 95% using protein A. The method uses a rat IgG2b H chain which does not bind to protein A. Catumaxomab (a bispecific antibody) purified by this method has a half-life of about 2.1 days in human. Its half-life is significantly shorter than the half-life of a normal human IgG1 which is 2 to 3 weeks (Non-patent Document 2). In this context, antibodies that have z106 (SEQ ID NO: 13) and z107 (SEQ ID NO: 14) described in Example 3 as constant regions were assessed for their pharmacokinetics.

In a pharmacokinetic experiment for calculating the half-life in human, the pharmacokinetics in human FcRn transgenic mice (B6.mFcRn-/-.hFcRn Tg line 276 +/+ mice, Jackson Laboratories) was assessed by the following procedure. MRA-Gld/MRA-k (hereinafter abbreviated as MRA-IgG1) having the IgG1 constant region and MRA-z106/MRA-z107/MRA-k (hereinafter abbreviated as MRA-z106/z107) that has z106/z107 as constant region was each intravenously administered once at a dose of 1 mg/kg to mice, and blood was collected at appropriate time points. The collected blood was immediately centrifuged at 15,000 rpm and 4°C for 15 minutes to obtain blood plasma. The separated plasma was stored in a freezer at -20°C or below until use. The plasma concentration was determined by ELISA.

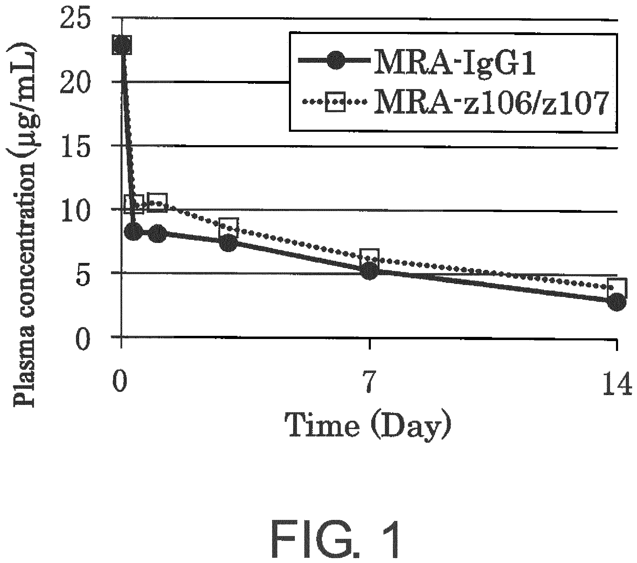

MRA-IgG1 and MRA-z106/z107k were assessed for their plasma retention in human FcRn transgenic mice. As shown in Fig. 1, the result indicates that the retention of MRA-z106/z107 in plasma was comparable to or longer than that of MRA-IgG1. As described above, z106/z107, a constant region that allows for efficient production or purification of the heteromeric antibody to high purity by the protein A-based purification step alone, was demonstrated to be comparable or superior to human IgG1 in terms of plasma retention.

### [Example 5] Construction of expression vectors for antibody genes and expression of respective antibodies

The antibody H chain variable regions used were:
Q499 (the H chain variable region of an anti-human F.IX antibody, SEQ ID NO: 16).
J339 (the H chain variable region of an anti-human F.X antibody, SEQ ID NO: 17).

The antibody L chain used was:
L377-k (the L chain common to an anti-human F.IX antibody and an anti-human F.X antibody, SEQ ID NO: 18).

The antibody H chain constant regions used were:
z118 (SEQ ID NO: 19), which was constructed from z106 described in Example 1, by introducing a substitution mutation of Phe for Leu at position 405 (EU numbering);
z121 (SEQ ID NO: 20), which was constructed from z118 by introducing a substitution mutation of Arg for His at position 435 (EU numbering); and
z119 (SEQ ID NO: 21), which was constructed from z118 by introducing substitution mutations of Glu for Lys at position 356 (EU numbering) and at position 439 (EU numbering).

The anti-human F.IX antibody H chain genes Q499-z118 and Q499-z121 were constructed by linking respectively z118 and z121 downstream of Q499. The anti-human F. X antibody H chain gene J339-zl 19 was constructed by linking z119 downstream of J339.

Each of the antibody genes (Q499-z118, Q499-z121, J339-z119, and L377-k) was inserted into an animal cell expression vector.

The following antibodies were expressed transiently in FreeStyle293 cells (Invitrogen) by transfection using the constructed expression vectors. As shown below, antibodies were named using the combinations of transfected antibody genes.
Q499-z118/J339-z119/L377-k
Q499-z121/J339-z119/L377-k

The above two antibodies are only different at the amino acid of position 435 in the EU numbering system in the H chain of the anti-human F.IX antibody. z118 has His at position 435 and it has protein A-binding affinity. Meanwhile, z121 has Arg at position 435, and it is predicted to have no protein A-binding activity based on the finding described in Example 2. Q499 is predicted to bind to protein A based on its sequence. Thus, as for Q499-z118/J339-z119/L377-k, the homomeric species J339-z119/L377-k (a homomeric antibody against FX) has two protein A-binding sites; the heteromeric antibody Q499-z118/J339-z119/L377-k has three; and the homomeric antibody Q499-z118/L377-k (homomeric antibody against FIX) has four protein A-binding sites. Meanwhile, as for Q499-z121/J339-z119/L377-k introduced with a modification that leads to loss of protein A-binding affinity, the homomeric species J339-z119/L377-k has two protein A-binding sites; the heteromeric antibody Q499-z121/J339-z119/L377-k has two; and the homomeric antibody Q499-z121 /L377-k has two. Specifically, even if a modification that leads to loss of protein A-binding affinity (for example, a modification that substitutes Arg for the amino acid at position 435, EU numbering) was introduced into only the H chain which binds to protein A through its variable region, it would not produce the effect that allows for efficient isolation/purification of the heteromeric antibody to high purity by the protein A-based purification step alone. However, the modification that leads to the loss of protein A-binding ability can produce the effect when MabSelct SuRe (GE Healthcare) is used. MabSelect SuRe is a modified protein A incapable of binding to Q499 and a chromatographic carrier for use in the purification of antibodies. The carrier was developed to meet industrial requirements. The ligand is a recombinant protein A that has been modified by genetic engineering to be resistant to alkaline conditions. The great pH stability enables efficient and low-cost NaOH wash.

Furthermore, the carrier is characteristic in that it does not bind to the heavy chain variable region of the VH3 subclass, such as Q499. With respect to Q499-z118/J339-z119/L377-k, the homomeric species J339-z119/L377-k has two MabSelect SuRe-binding sites; the heteromeric antibody Q499-z118/J339-z119/L377-k has two; and the homomeric antibody Q499-z118 /L377-k has two. Meanwhile, as for Q499-z121/J339-z119/L377-k, the homomeric species J339-z119/L377-k has two MabSelect SuRe-binding sites; the heteromeric antibody Q499-z121/J339-z119/L377-k has a single site; and the homomeric antibody Q499-z121 /L377-k does not have any MabSelect SuRe-binding site. Specifically, it is understood that by combining a modified protein A incapable of binding to the antibody variable region, such as MabSelect SuRe, with a modification that leads to loss of protein A-binding affinity, the heteromeric antibody can be efficiently isolated and purified to high purity by the protein A-based purification step alone regardless of the protein A-binding activity of the heavy chain variable region.

### [Example 6] Isolation and purification of heteromeric antibodies by affinity chromatography using modified protein A

CM in which Q499-z118/J339-z119/L377-k or Q499-z121/J339-z119/L377-k had been expressed was subjected to chromatography using modified protein A. The CM samples were filtered through a filter with a pore size of 0.22 µm, and loaded onto a Mab Select SuRe column (GE Healthcare) equilibrated with D-PBS. The column was subjected to washes 1 and 2 and elution as shown in Table 7. Recombinant protein A consists of five domains (A to E) which have IgG-binding activity. In Mab Select SuRe, domain B has been modified by genetic engineering to have a tetrameric structure. Mab Select SuRe lacks affinity for the antibody variable region, and is advantageous in that it allows for antibody elution even under milder conditions as compared to conventional recombinant protein A. In addition, the resin has improved alkaline resistance and enables for cleaning in place using 0.1 to 0.5 M NaOH, and is thus more suitable for production. In the experiment described in this Example as shown in Table 7, 50 mM acetic acid (the pH was not adjusted and the measured pH was around 3.0) was used for the elution instead of the stepwise elution at pH3.6 and pH 2.7 described in Example 3. The respective eluted fractions were collected and analyzed by cation exchange chromatography to identify their components. To prepare controls, each CM was loaded onto rProtein G Sepharose Fast Flow resin (GE Healthcare) in the same manner as described in Example 2. Samples purified by batchwise elution were used as controls.

Next, the fractions eluted from protein A were subjected to ion exchange chromatography. An SP Sepharose High Performance column (GE Healthcare) was equilibrated with an equilibration buffer (20 mM sodium phosphate buffer, pH 6.0). Then, the fractions eluted from protein A were neutralized with 1.5 M Tris-HCl, (pH7.4), and diluted three times with equilibration buffer, and loaded. Antibodies bound to the column were eluted with 25 column volumes (CV) of an NaCl concentration gradient of 50 to 350 mM. The eluted fractions containing the heteromeric antibody were purified by gel filtration chromatography using superdex200. The resulting monomer fractions were collected, and used in the assessment of pharmacokinetics in human FcRn transgenic mice described in Example 7.

**[Table 7]**

| **Equilibration** | D-PBS |
|---|---|
| **Wash** 1 | 400 mM Arg-HCl/D-PBS |
| **Wash** 2 | 50 mM NaAcetate buffer, pH6.0 |
| **Elution** | 50 mM Acetic acid |

The result of cation exchange chromatography analysis of each eluted fraction is shown in Tables 8 and 9. As shown in Table 8, with respect to Q499-z118/J339-z119/L377-k, the component ratio of each eluted fraction is not much different from that of the control. The reason is probably that all three species J339-z119/L377-k (a homomeric antibody against F.X), Q499-z118/L377-k (a homomeric antibody against F.IX), and Q499-z118/J339-z119/L377-k (a heteromeric antibody) had two binding sites for the modified protein, and thus there was no difference in terms of the association/dissociation during the protein A-based purification step.

Meanwhile, in the case of Q499-z121/J339-z119/L377-k, the ratio of Q499-z121/L377-k (a homomeric antibody against F.IX) in the eluted fraction was significantly reduced as compared to the control as shown in Table 9. In contrast, the ratios of J339-z119/L377-k (a homomeric antibody against F.X) and Q499-z121/J339-z119/L377-k (a heteromeric antibody) in the eluted fraction were relatively increased as compared to the control along with a decrease of Q499-z121/L377-k. It was believed that this is because J339-z119/L377-k (a homomeric antibody against F.X) has two binding sites for the modified protein A and Q499-z121/J339-z119/L377-k (a heteromeric antibody) has one. However, Q499-z121/L377-k (a homomeric antibody against F.IX) has no binding site, and accordingly the majority of Q499-z121/L377-k passed through the column without binding to the modified protein A.

As described above, the present invention also demonstrates that with respect to antibodies whose variable regions have protein A-binding activity, when the modified protein A is combined with a modification that leads to loss of protein A-binding affinity, one of the homomeric antibodies can be significantly decreased, and as a result the purity of the heteromeric antibody is increased by the protein A-based purification step alone.

**[Table 8]**

| Q499-z118/J339-z119/L377-k | | |
|---|---|---|
| **Peak area** (%) | **Control** | **Eluted fraction** |
| J339-z119/L377-k | 2.3 | 4.2 |
| Q499-z118/J339-z119/L377-k | 75.5 | 79.1 |
| Q499-z118/L377-k | 22.3 | 16.7 |

**[Table 9]**

| Q499-z121/J339-z119/L377-k | | |
|---|---|---|
| **Peak area** (%) | **Control** | **Eluted fraction** |
| J339-z119/L377-k | 3.2 | 5.9 |
| Q499-z121/J339-z119/L377-k | 76.6 | 91.6 |
| Q499-z121/L377-k | 20.2 | 2.5 |

### [Example 7] Assessment of pharmacokinetics in human FcRn transgenic mice Q499-z118/J339-z119/L377-k and Q499-z121/J339-z119/L377-k prepared as described in Example 6 were assessed for their pharmacokinetics.

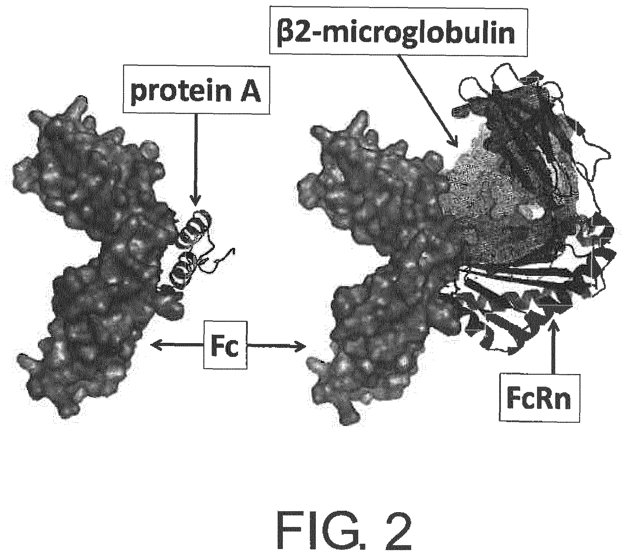

It is likely to be difficult to adjust the protein A-binding activity without loss of the human FcRn binding, because protein A and human FcRn recognize the same site in an antibody IgG (J Immunol. 2000 164 (10):5313-8) as shown in Fig. 2. To retain the binding affinity for human FcRn is very important for the long plasma retention (long half-life) in human, which is characteristic of IgG-type antibodies. In this context, pharmacokinetics was compared between Q499-z118/J339-z119/L377-k and Q499-z121/J339-z119/L377-k prepared as described in Example 6.

In a pharmacokinetic experiment to predict the half-life in human, the pharmacokinetics in human FcRn transgenic mice (B6.mFcRn-/-.hFcRn Tg line 276 +/+ mice, Jackson Laboratories) was assessed by the following procedure. Q499-z118/J339-z119/L377-k and Q499-z121/J339-z119/L377-k were each intravenously administered once at a dose of 5 mg/kg to mice, and blood was collected at appropriate time points. The collected blood was immediately centrifuged at 15,000 rpm and 4°C for 15 minutes to obtain blood plasma. The separated plasma was stored in a freezer at -20°C or below until use. The blood concentration was determined by ELISA.

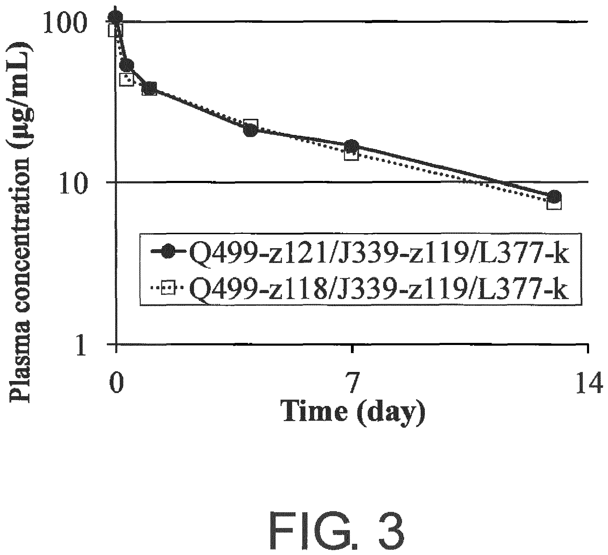

As shown in Fig. 3, the result indicates that Q499-z118/J339-z119/L377-k and Q499-z121/J339-z119/L377-k were comparable to each other in terms of plasma retention. Thus, z121/z119, a constant region in which either of the H chains is introduced with a modification that leads to loss of protein A-binding ability was demonstrated to be comparable in terms of plasma retention to z118/z119 which does not have the modification that leads to loss of protein A-binding affinity. As described above, the present inventors revealed a modification (for example, a substitution mutation of Arg for the amino acid at position 435,EU numbering) that leads to loss of protein A-binding ability but has no influence on the pharmacokinetics, and which allows for efficient isolation/purification of the heteromeric antibody to high purity through the protein A-based purification step alone regardless of the variable region.

### [Example 8] Introduction of mutations into the CH3 domain of GC33-IgG1-CD3-scFv and preparation of designed molecules through the protein A-based purification step alone

### Introduction of mutations for protein A-based purification of the GC33-IgG1-CD3-scFv molecule

The inventors designed an anti-GPC3 IgG antibody molecule in which an anti-CD3 scFv antibody is linked to one of the two H chains (Fig. 4). This molecule was expected to be capable of killing cancer cells by recruiting T cells to cancer cells through divalent binding to glypican-3 (GPC3), a cancer-specific antigen, and monovalent binding to CD3, a T-cell antigen. An anti-CD3 scFv antibody must be linked to only one of the two H chains to achieve the monovalent binding to CD3. In this case, it is necessary to purify the molecule formed via heteromeric association of the two types of H chains.

Thus, using the same method described in Example 3, a substitution mutation of Arg for His at position 435 (EU numbering) was introduced into one of the H chains. Furthermore, the above mutation was combined with the mutations (a substitution of Lys for Asp at position 356, EU numbering, is introduced into one H chain and a substitution of Glu for Lys at position 439, EU numbering, is introduced into the other H chain) described in WO 2006/106905 (PROCESS FOR PRODUCTION OF POLYPEPTIDE BY REGULATION OF ASSEMBLY) as a modification to enhance the heteromeric association of the two H chains. The present inventors tested whether it was possible with the combined mutations to purify the molecule of interest by protein A chromatography alone.

### Construction of expression vectors for antibody genes and expression of respective antibodies

The gene encoding GPC3 (anti-human Glypican-3 antibody H chain variable region, SEQ ID NO: 22) as an antibody H chain variable region was constructed by a method known to those skilled in the art. Furthermore, the gene encoding GC33-k0 (anti-human Glypican-3 antibody L chain, SEQ ID NO: 23) as an antibody L chain was constructed by a method known to those skilled in the art. In addition, the genes described below were constructed as an antibody H chain constant region by a method known to those skilled in the art.
LALA-G1d (SEQ ID NO: 24), which was constructed from IgG1 by substituting Ala for Leu at positions 234 and 235 (EU numbering), and Ala for Asn at position 297 (EU numbering), and deleting the C-terminal Gly and Lys
LALA-G1d-CD3 (SEQ ID NO: 25), which was constructed from LALA-G1d by linking an anti-CD3 scFv (in which the anti-human CD3 antibody H chain variable region is linked via a peptide linker to the C terminus of the anti-human CD3 antibody L chain variable region)
LALA-G3S3E-G1d (SEQ ID NO: 26), which was constructed from LALA-G1d by substituting Arg for His at position 435 (EU numbering) and Glu for Lys at position 439 (EU numbering); and
LALA-S3K-G1d-CD3 (SEQ ID NO: 27), which was constructed from LALA-G1d-CD3 by substituting Lys for Asp at position 356 (EU numbering).
Anti-human GPC3 antibody H chain genes NTA1L and NTA1R were constructed by linking respectively LALA-G1d-CD3 (in which an anti-CD3 scFv antibody is linked to the H chain constant region) and LALA-G1d (an H chain constant region) downstream of GPC3, which is the H chain variable region of an anti-human Glypican-3 antibody. Furthermore, anti-human GPC3 antibody H chain genes NTA2L and NTA2R were constructed by linking an anti-CD3 scFv antibody downstream of GPC3 as an H chain constant region, and linking LALA-S3K-G1d-CD3 introduced with a substitution mutation of Lys for Asp at position 356 (EU numbering) or LALA-G3S3E-G1d introduced with substitution mutations of Arg for His at position 435 (EU numbering) and of Glu for Lys at position 439 (EU numbering). The constructed genes were listed below.
H chain

| | |
|---|---|
| NTA1L : | GPC3-LALA-G1d-CD3 |
| NTA1R : | GPC3-LALA-G1d |
| NTA2L : | GPC3-LALA-S3K-G1d-CD3 |
| NTA2R : | GPC3-LALA-G3S3E-G1d |

L chain
GC33-k0 Each of the antibody genes (H chains: NTA1L, NTA1R, NTA2L, and NTA2R; L chain: GC33-k0) was inserted into an animal cell expression vector. Using a method known to those skilled in the art, the antibodies listed below were expressed transiently in FreeStyle293 cells (Invitrogen) by transfecting the cells with the constructed expression vectors. As shown below, antibodies were named using the combinations of transfected antibody genes (first H chain/second H chain/L chain).
NTA1L/NTA1R/GC33-k0
NTA2L/NTA2R/GC33-k0

### Protein purification of the expressed samples and assessment of heterodimer yield

Culture supernatants of FreeStyle293 cells (CM) containing the following antibodies were used as a sample.
NTA1L/NTA1R/GC33-k0
NTA2L/NTA2R/GC33-k0
The CM samples were filtered through a filter with a pore size of 0.22 µm, and loaded onto an rProtein A Sepharose Fast Flow column (GE Healthcare) equilibrated with D-PBS. The column was subjected to washes 1 and 2 and elution 1 as shown in Table 10. The volume of CM to be loaded onto the column was adjusted to 20 mg antibody/ml resin. Respective fractions eluted under each condition were collected and analyzed by size exclusion chromatography to identify their components.

**[Table 10]**

| **Equilibration** | D-PBS |
|---|---|
| **Wash** 1 | 1mM sodium acetate, 150mM NaCl, pH6.5 |
| **Wash** 2 | 0.3mM HCl, 150mM NaCl, pH3.7 |
| **Elution** 1 | 2mM HCl, pH2. 7 |

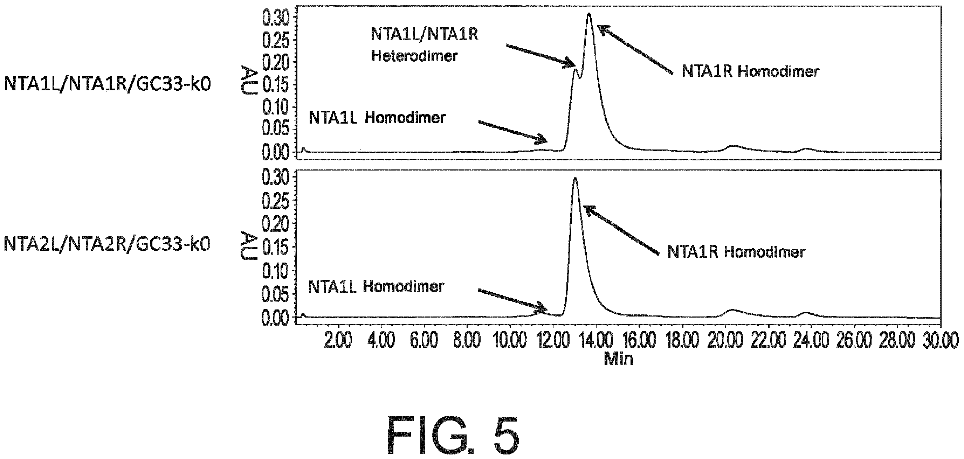

The result of size exclusion chromatography of each eluted fraction is shown in Fig. 5 and Table 11 below. The values represent the area of elution peak expressed in percentage. For NTA1L/NTA1R/GC33-k0 and NTA2L/NTA2R/GC33-k0, the homomeric antibodies (antibodies with homomeric NTA1L or homomeric NTA2L) that have the anti-CD3 scFv antibody in both chains were almost undetectable. This is thought to be caused by the extremely low expression level of the H chains containing the anit-CD3 scFv antibody because the expression level of an scFv molecule is generally low. As for homomeric antibodies that do not contain the anti-CD3 scFv antibody in its two chains, about 76% of the NTA1R homomeric antibody was observed in the case of NTA1L/NTA1R/GC33-k0, while only about 2% of the homomeric NTA2R antibody was observed in the case of NTA2L/NTA2R/GC33-k0. Thus, the present invention demonstrated that when the substitution mutations of Lys for Glu at position 356 (EU numbering) and of Glu for Lys at position 439 (EU numbering) for efficient formation of heteromeric molecules from the respective H chains, was combined with the substitution mutation of Arg for His at position 435 (EU numbering), the heteromeric antibody (bispecific antibody of interest) could be efficiently purified to a purity of 98% or higher through the protein A-based purification step alone.

**[Table 11]**

| | NTA1R homodimer | NTA1L/NTA1R heterodimer | NTA1R homodimer |
|---|---|---|---|
| NTA1L/NTA1R/GC33-k0 | 0.7 | 23.5 | 75.8 |
| NTA2L/NTA2R/GC33-k0 | 1.8 | 98.2 | - |

### [Example 9] Introduction of mutations into the CH3 domain of monovalent antibodies and preparation of designed molecules through the protein A-based purification step alone

### Introduction of mutations for the purification of monovalent antibody molecules using protein A

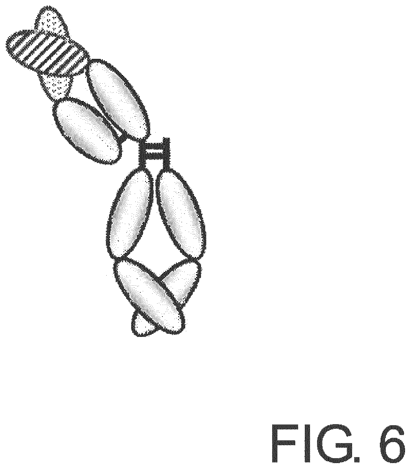

An ordinary anti-GPC3 IgG antibody binds divalently via the two H chains to glypican-3 (GPC3), a cancer-specific antigen. In the experiment described in this Example, the inventors designed and assessed an anti-GPC3 IgG antibody molecule (Fig. 6) that monovalently binds to glypican-3. It is thought that when compared to ordinary divalent antibodies, the monovalent binding of the molecule to glypican-3 (GPC3), a cancer-specific antigen, was based on affinity and not avidity. Thus, it was expected that the molecule was capable of binding to the antigen without crosslinking. To achieve the monovalent binding of the two H chains to glypican-3 (GPC3), one has to be an H chain consisting of a hinge-Fc domain that lacks the variable region and CH1 domain, while the other is an ordinary H chain. In this case, it is necessary to purify the molecule that results from heteromeric association of the two types of H chains.

Thus, using the same method as described in Example 3, a substitution mutation of Arg for His at position 435 (EU numbering) was introduced into one of the H chains. Furthermore, the above mutation was combined with the mutations (a substitution of Lys for Asp at position 356, EU numbering, is introduced into one H chain and a substitution of Glu for Lys at position 439, EU numbering, is introduced into the other H chain) described in WO 2006/106905 (PROCESS FOR PRODUCTION OF POLYPEPTIDE BY REGULATION OF ASSEMBLY) as a modification to enhance the heteromeric association of the two H chains. The present inventors assessed whether it was possible with the combined mutations to purify the molecule of interest by protein A chromatography alone.

### Construction of expression vectors for antibody genes and expression of respective antibodies

The antibody H chain variable region used was:
GPC3 (the H chain variable region of an anti-human Glypican-3 antibody, SEQ ID NO: 22). The antibody L chain used was:
GC33-k0 (the L chain of an anti-human Glypican-3 antibody, SEQ ID NO: 23).
The antibody H chain constant regions used were:
LALA-G1d (SEQ ID NO: 24), which was constructed from IgG1 by introducing substitution mutations of Ala for Leu at positions 234 and 235 (EU numbering), and of Ala for Asn at position 297 (EU numbering), and deleting the C-terminal Gly and Lys;
LALA-G3-G1d (SEQ ID NO: 28), which was constructed from LALA-G1d by introducing a substitution mutation of Arg for His at position 435 (EU numbering);
LALA-G3S3E-G1d (SEQ ID NO: 26), which was constructed from LALA-G3-G1d by introducing a substitution mutation of Glu for Lys at position 439 (EU numbering) ;
LALA-G1Fc (SEQ ID NO: 29), which was constructed from LALA-G1d by deleting the region of positions 1 to 215 (EU numbering); and
LALA-G1Fc-S3K (SEQ ID NO: 30), which was constructed from G1Fc by introducing a substitution mutation of Lys for Asp at position 356 (EU numbering).
Anti-human GPC3 antibody H chain genes NTA4L-cont, NTL4L-G3, and NTA4L were constructed by linking downstream of GPC3 (the H chain variable region of an anti-human Glypican-3 antibody), respectively, LALA-G1d (an H chain constant region), LALA-G3-G1d introduced with a substitution mutation of Arg for His at position 435 (EU numbering), and LALA-G3S3E-G1d introduced with substitution mutations of Arg for His at position 435 (EU numbering) and of Glu for Lys at position 439 (EU numbering). Furthermore, Fc genes NTA4R-cont and NTA4R were constructed by using LALA-G1Fc (an anti-human hinge Fc domain) and LALA-G1Fc-S3K (a hinge Fc domain introduced with a substitution mutation of Lys for Asp at position 356, EU numbering). The constructed genes are:
H chain
   NTA4L-cont : GPC3-LALA-G1d
   NTA4L-G3 : GPC3-LALA-G3-G1d
   NTA4L : GPC3-LALA-G3S3E-G1d
   NTA4R-cont: LALA-G1Fc
   NTA4R: LALA-G1Fc-S3K
L chain
   GC33-k0
The antibody genes (NTA4L, NTA4L-cont, NTA4L-G3, NTA4R, NTA4R-cont, and GC33-k0) were each inserted into an animal cell expression vector.
The following antibodies were expressed transiently in FreeStyle293 cells (Invitrogen) by transfection using the constructed expression vectors. As shown below, antibodies were named using the combinations of transfected antibody genes.
NTA4L-cont/NTA4R-cont/GC33-k0
NTA4L-G3/NTA4R-cont/GC33-k0
NTA4L/NTA4R/GC33-k0

### Protein purification of expressed samples and assessment of heterodimer yield

CM containing the following antibody was used as a sample:
NTA4L-cont/NTA4R-cont/GC33-k0
NTA4L-G3/NTA4R-cont/GC33-k0
NTA4L/NTA4R/GC33-k0
The CM samples were filtered through a filter with a pore size of 0.22 µm, and loaded onto an rProtein A Sepharose Fast Flow column (GE Healthcare) equilibrated with D-PBS. The column was subjected to washes 1 and 2 and elution 1 as shown in Table 12. The volume of CM to be loaded onto the column was adjusted to 20 mg antibody/ml resin. Respective fractions eluted under each condition were collected and analyzed by size exclusion chromatography to identify their components.

**[Table 12]**

| **Equilibration** | D-PBS |
|---|---|
| **Wash** 1 | 1mM sodium acetate, 150mM NaCl, pH6.5 |
| **Wash** 2 | 0.3mM HCl, 150mM NaCl, pH3.7 |
| **Elution** 1 | 2mM HCl, pH2. 7 |

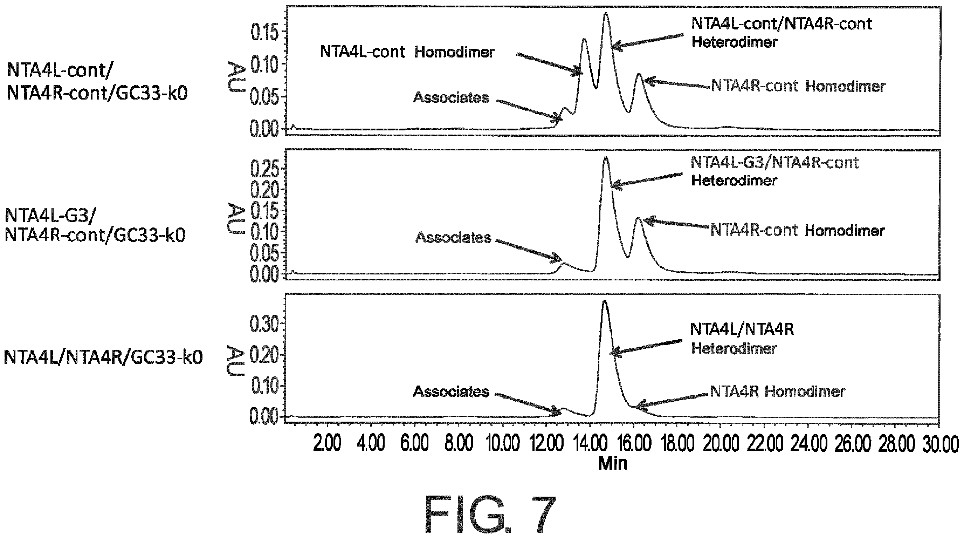

The result of size exclusion chromatography analysis of each eluted fraction is shown in Fig. 7 and Table 13 below. The values represent the area of elution peak expressed in percentage.

As for NTA4L-cont/NTA4R-cont/GC33-k0, the homomeric antibody that divalently binds to GPC3 (homomeric antibody NTA4L-cont) and the homomeric molecule that has no GPC3-binding domain (homomeric antibody NTA4R-cont) were eluted, while the heteromeric antibody of interest, NTA4L-cont/NTA4R-cont, accounted for only 46.5%.

In the case of NTA4L-G3/NTA4R-cont/GC33-k0, the homomeric antibody that divalently binds to GPC3 (homomeric antibody NTA4L-G3) was almost undetectable, while the homomeric molecule having no GPC3-binding domain (homomeric antibody NTA4R-cont) was abundant. The heteromeric antibody of interest, NTA4L-G3/NTA4R-cont, accounted for 66.7%. In the case of NTA4L/NTA4R/GC33-k0, the homomeric antibody that divalently binds to GPC3 (homomeric antibody NTA4L) was almost undetectable, and the proportion of the homomeric molecule having no GPC3-binding domain (NTA4R) was considerably reduced, resulting in a significant increase of up to 93.0% in the proportion of the heteromeric antibody of interest, NTA4L/NTA4R. Thus, the present invention demonstrated that when the substitution mutations of Lys for Asp at position 356 (EU numbering) and of Glu for Lys at position 439 (EU numbering) for efficient formation of heteromeric molecules from the respective H chains were introduced in combination with the substitution mutation of Arg for His at position 435 (EU numbering), the heteromeric antibody (a bispecific antibody of interest) could be efficiently purified to a purity of 93% or higher through the protein A-based purification step alone.

**[Table 13]**

| | Homomeric anti-GPC3 antibody | Heteromeric antibody | Homomeric Fc molecule |
|---|---|---|---|
| NTA4L-cont/NTA4R-cont/GC33-k0 | 30.0 | 46.5 | 23.5 |
| NTA4L-G3/NTA4R-cont/GG33-k0 | - | 66.7 | 33.3 |
| NTA4L/NTA4R/GG33-k0 | - | 93.0 | 7.0 |

### [Example 10] Preparation of heteromeric antibodies through a purification step by protein A column chromatography using pH gradient elution

As described in Example 9, the present inventors demonstrated that in the case of an antibody having the variable region only at one arm, the heteromeric antibody could be efficiently purified through the protein A-based purification step alone by combining the substitution mutation of Arg for His at position 435 (EU numbering) with the mutations (a substitution of Lys for Asp at position 356, EU numbering, is introduced into one H chain and a substitution of Glu for Lys at position 439, EU numbering, is introduced into the other H chain) described in WO 2006/106905 (PROCESS FOR PRODUCTION OF POLYPEPTIDE BY REGULATION OF ASSEMBLY). However, the heteromeric antibody is not purified to a sufficiently high purity with elution 1 (elution buffer: 2 mM HCl, pH 2.7) alone. An additional purification step is needed.

Then, in this Example, the present inventors assessed whether the heteromeric antibody can be isolated and purified to high purity by protein A column chromatography using elution with a pH gradient. This was based on the assumption that more protein A-binding sites lead to stronger binding of the heteromeric antibody to protein A, and as a result lower pH is required for elution. Purification can be achieved more efficiently at a lower cost when the purity of the heteromeric antibody can be increased to almost 100% by using such pH gradient elution.
CM samples containing the following antibodies were used:
NTA4L-cont/NTA4R-cont/GC33-k0
NTA4L-G3/NTA4R-cont/GC33-k0
NTA4L/NTA4R/GC33-k0
The CM samples were filtered through a filter with a pore size of 0.22 µm, and loaded onto a HiTrap protein A HP column (GE Healthcare) equilibrated with D-PBS. The column was sequentially subjected to washes 1 and 2, and then elution with a pH gradient using elution A and B as shown in Table 14. The pH gradient elution was achieved with the following linear gradient: elution A/elution B = (100:0) → (30:70) for 35 minutes. Eluted fractions were collected and analyzed by size exclusion chromatography analysis to identify their components.

**[Table 14]**

| **Equilibration** | D-PBS |
|---|---|
| **Wash** 1 | D-PBS |
| **Wash** 2 | 20 mM NaCitrate, pH5.0 |
| **Elution** A | 20 mM NaCitrate, pH5.0 |
| **Elution** B | 20 mM NaCitrate, pH2.7 |

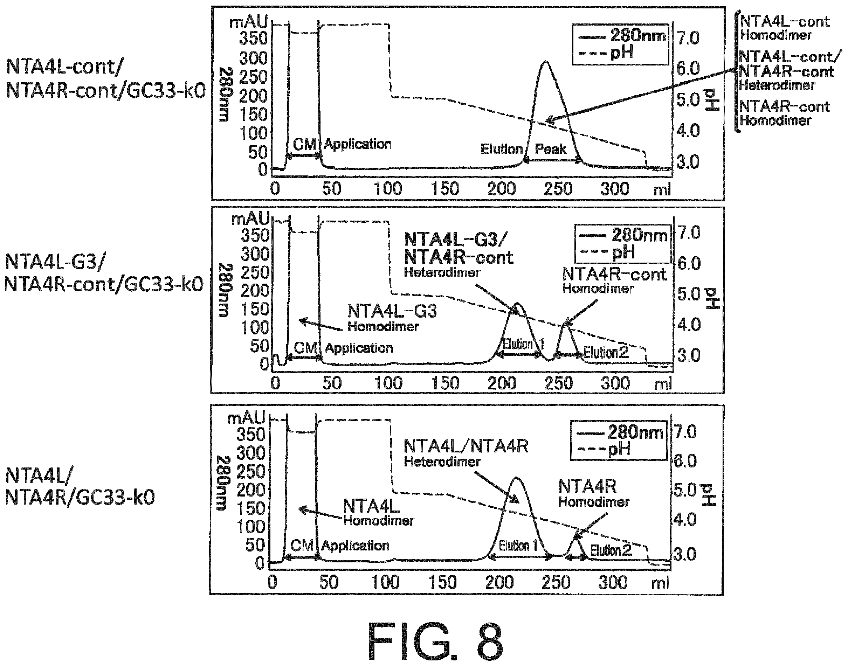

NTA4L-cont/NTA4R-cont/GC33-k0, NTA4L-G3/NTA4R-cont/GC33-k0, and NTA4L/NTA4R/GC33-k0 were purified by protein A column chromatography under the pH gradient elution condition. The resulting chromatograms are shown in Fig. 8. The elution of NTA4L-cont/NTA4R-cont/GC33-k0 resulted in a broad peak. Meanwhile, the pH gradient elution of NTA4L-G3/NTA4R-cont/GC33-k0 gave two elution peaks. The peaks of high and low pHs were labeled as "elution 1" and "elution 2", respectively. The result for NTA4L/NTA4R/GC33-k0 was roughly the same as that for NTA4L-G3/NTA4R-cont/GC33-k0, except that the peak area of elution 2 was smaller.

The result of size exclusion chromatography analysis of each peak is shown in Table 15. NTA4L-cont/NTA4R-cont/GC33-k0 gave three components eluted in this order: a homomeric antibody that divalently binds to GPC3 (homomeric antibody NTA4L-cont), a heteromeric antibody that monovalently binds to GPC3 (heteromeric antibody NTA4L-cont/NTA4R-conc), and a homomeric molecule having no GPC3-binding domain (homomeric antibody NTA4R-cont). It is thought that the reason why these components were not separated by pH gradient elution is that they have the same number (two) of protein A-binding sites. Meanwhile, it was revealed that in elution 1 of NTA4L-G3/NTA4R-cont/GC33-k0, the levels of homomeric antibody that divalently binds to GPC3 (homomeric antibody NTA4L-G3) and homomeric molecule having no GPC3-binding domain (homomeric antibody NTA4R-cont) were below the detection limit, while the heteromeric antibody that monovalently binds to GPC3 (NTA4L-G3/NTA4R-conc heteromeric antibody) accounted for 99.6%. In elution 2, the homomeric molecule having no GPC3-binding domain (homomeric antibody NTA4R-cont) was found to account for 98.8%. The homomeric antibody NTA4L-G3 passes through the protein A column because it cannot bind to protein A due to the substitution mutation of Arg for His at position 435 (EU numbering). Meanwhile, the heteromeric antibody NTA4L-G3/NTA4R-conc has a single protein A-binding site, while the homomeric antibody NTA4R-cont has two. More protein A-binding sites means stronger protein A binding, and as a result lower pH was required for elution. This is thought to be the reason why homomeric antibody NTA4R-cont was eluted at a lower pH than heteromeric antibody NTA4L-G3/NTA4R-conc. Almost the same result was obtained for NTA4L/NTA4R/GC33-k0. The result of size exclusion chromatography analysis shows that the component ratio was comparable to that of NTA4L-G3/NTA4R-cont/GC33-k0. There was a difference between the protein A chromatograms, and the peak area ratio of elution 2 to elution 1 was smaller in NTA4L/NTA4R/GC33-k0. The expression ratio of the homomeric antibody NTA4R-cont, which is the major component of elution 2, was reduced due to the mutations introduced for efficient generation of the heteromeric antibody NTA4L-G3/NTA4R-conc. The amino acid mutations described above improved the purification yield of the heteromeric antibody and the robustness of purification by protein A column chromatography with pH gradient elution.

As described above, the present inventors demonstrated that the heteromeric antibody could be efficiently isolated and purified to high purity through the purification step using protein A column chromatography alone with pH gradient elution.

**[Table 15]**

| **Peak area** (%) | | **Homomeric antibody that divalently binds to GPC3** | **Heteromeric antibody that monovalently binds to GPC3** | **Homomeric molecule having no GPC3-binding domain** |
|---|---|---|---|---|
| NTA4L-cont/NTA4R-cont/GC33-k0 | **Elution** | 25.4 | 54.4 | 20.2 |
| NTA4L-G3/NTA4R-cont/GG33-k0 | **Elution** 1 | ND | 99.6 | ND |
| | **Elution** 2 | - | 1.2 | 98.8 |
| NTA4L/NTA4R/GC33-k0 | **Elution** 1 | ND | 99.6 | ND |
| | **Elution** 2 | - | 1.4 | 98.6 |

### [Example 11] Introduction of mutation into the CH3 domain of monovalent Fcalpha receptor-Fc fusion protein and preparation of designed molecules through the protein A-based purification step alone

### Introduction of mutation into CH3 domain and preparation of monovalent Fcalpha receptor-Fc fusion protein through the protein A-based purification step

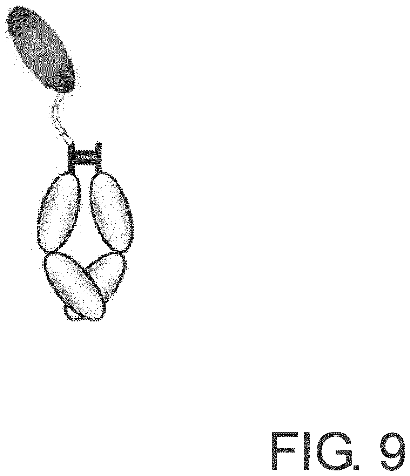

Conventional Fc receptor-Fc fusion proteins such as Eternercept and Abatacept are homodimers that can divalently bind to ligands. In the experiment described in this Example, the inventors designed and assessed an Fc receptor-Fc fusion protein that monovalently binds to IgA as a ligand (Fig. 9). To achieve the monovalent binding of the Fcalpha receptor to IgA, one of the two Fc receptor-Fc fusion protein H chains must be the whole H chain having the hinge-Fc domain. In this case, it is necessary to purify the molecule that results from heteromeric association of the two types of H chains

Thus, using the same method described in Example 6, a substitution mutation of Arg for His at position 435 (EU numbering) was introduced into one of the two H chains. Furthermore, the above mutation was combined with the mutations (a substitution of Lys for Asp at position 356, EU numbering, is introduced into one H chain and a substitution of Glu for Lys at position 439, EU numbering is introduced into the other H chain) described in WO 2006/106905 (PROCESS FOR PRODUCTION OF POLYPEPTIDE BY REGULATION OF ASSEMBLY) as a modification to enhance the heteromeric association of the two types of H chains. The present inventors assessed whether it was possible with the combined mutations to purify the molecule of interest by protein A chromatography alone.

### Construction of expression vectors for antibody genes and expression of respective antibodies

The Fc receptor used was FcalphaR (human IgA1 receptor, SEQ ID NO: 31). The fusion H chain constant regions used were:
G1Fc (SEQ ID NO: 32), which is a human hinge-Fc domain constructed from IgG1 by deleting the C-terminal Gly and Lys, and residues of positions 1 to 223 (EU numbering);
G1Fc-G3S3K (SEQ ID NO: 33), which was constructed from G1Fc by introducing substitution mutations of Lys for Asp at position 356 (EU numbering) and of Arg for His at position 435 (EU numbering); and
G1Fc-S3E (SEQ ID NO: 34), which was constructed from G1Fc by introducing a substitution mutation of Glu for Lys at position 439 (EU numbering).
FcalphaR-Fc fusion proteins IAL-cont and IAL were constructed by linking downstream of FcalphaR via a polypeptide linker (SEQ ID NO: 35), G1Fc (an H chain constant region) and G1Fc-G3S3K introduced with substitution mutations of Lys for Asp at position 356 (EU numbering) and of Arg for His at position 435 (EU numbering).
Furthermore, Fc genes IAR-cont and IAR were constructed to encode G1Fc (a human hinge-Fc domain) and G1Fc-S3E (a hinge Fc domain introduced with a substitution mutation of Glu for Lys at position 439, EU numbering), respectively. The constructed genes were:
H chain
IAL-cont : FcalphaR-G1Fc
IAL : FcalphaR-G1Fc-G3S3K
IAR-cont: G1Fc
IAR: G1Fc-S3E
The antibody genes (IAL-cont, IAL, IAR-cont, and IAR) were each inserted into an animal cell expression vector.

The following antibodies were expressed transiently in FreeStyle293 cells (Invitrogen) by transfection using the constructed expression vectors. As shown below, antibodies were named using the combinations of transfected antibody genes.
IAL-cont/IAR-cont
IAL/IAR

### Protein purification of expressed sample and assessment of heterodimer yield

CM samples containing the following antibody were used:
IAL-cont/IAR-cont
IAL/IAR
The CM samples were filtered through a filter with a pore size of 0.22 µm, and loaded onto an rProtein A Sepharose Fast Flow column (GE Healthcare) equilibrated with D-PBS. The column was subjected to washes 1 and 2 and elution 1 as shown in Table 16. The volume of CM to be loaded onto the column was adjusted to 20 mg antibody/ml resin. Respective fractions eluted under each condition were collected and analyzed by size exclusion chromatography to identify their components.

**[Table 16]**

| **Equilibration** | D-PBS |
|---|---|
| **Wash** 1 | 1mM sodium acetate, 150mM NaCl, pH6.5 |
| **Wash** 2 | 0.3mM HCl, 150mM NaCl, pH3.7 |
| **Elution** 1 | 2mM HCl, pH2. 7 |

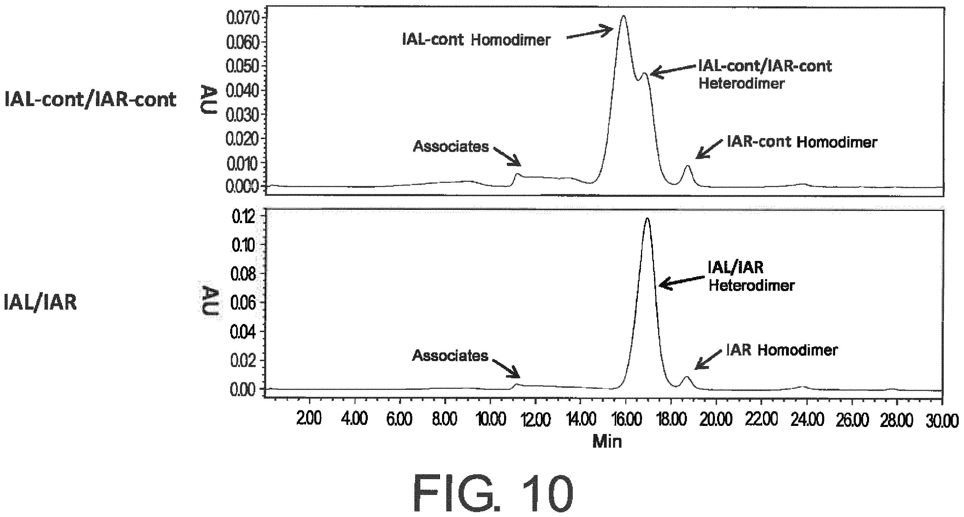

The result of size exclusion chromatography analysis of each eluted fraction is shown in Fig. 10 and Table 17 below. The values represent the area of elution peak expressed in percentage. As for IAL-cont/IAR-cont, a homomeric antibody that divalently binds to IgA (homomeric antibody IAL-cont) and a homomeric molecule having no IgA-binding site (homomeric antibody IAR-cont) were eluted, while the heteromeric antibody IAL-cont/IAR-cont of interest accounted for only 30%. In the case of IAL/IAR, the homomeric antibody that divalently binds to IgA (homomeric antibody IAL) was not detectable, and the proportion of the homomeric molecule having no IgA-binding site (homomeric antibody IAR) was considerable reduced; thus, the heteromeric antibody IAL/IAR of interest was significantly increased up to about 96%. Thus, the present invention demonstrated that when the substitution mutations of Lys for Asp at position 356 (EU numbering) and of Glu for Lys at position 439 (EU numbering) for efficient formation of heteromeric molecules from the respective H chains were introduced in combination with the substitution mutation of Arg for His at position 435 (EU numbering), the heteromeric antibody, a bispecific antibody of interest, could be efficiently purified to a purity of 95% or higher through the protein A-based purification step alone.

**[Table 17]**

| | **Homomeric IgA antibody** | **Heteromeric antibody** | **Homomeric Fc molecule** |
|---|---|---|---|
| IAL-cont/IAR-cont | 66.2% | 30.0% | 3.8% |
| IAL/IAR | - | 95.8% | 4.2% |

### [Example 12] Construction of a bispecific antibody of the four-chain IgG type

### Construction of expression vectors for antibody genes and expression of respective antibodies

The bispecific antibody against human F.IX and human F.X, which was designed as described in Example 1, consists of a common L chain and two types of H chains that each recognizes a different antigen. Obtaining a bispecific antibody with such a common L chain is not easy, because it is difficult for a common L chain sequence to recognize two different types of antigens. As described above, obtaining such a common L chain is extremely difficult. Thus, one may suspect that a more preferred option is a bispecific antibody consisting of two types of H chains and two types of L chains that recognize two types of antigens. If two types of H chains and two types of L chains are expressed, they form ten types of H2L2 IgG molecules in random combinations. It is very difficult to purify the bispecific antibody of interest from the ten types of antibodies.

In the experiment described in this Example, the present inventors prepared and assessed bispecific antibodies consisting of two types of H chains and two types of L chains against human IL-6 receptor and human glypican-3 (GPC3). To efficiently prepare bispecific antibodies consisting of two types of H chains and two types of L chains, it is necessary to enhance the association of H chains and L chains against the same antigen as well as the heteromeric association of two types of H chains. In addition, it is essential that the bispecific antibody with the right combination can be purified from the obtained expression products.

To enhance the association between H chains and L chains against the same antigen, the variable region (VH) of H chain (GC33-VH-CH1-hinge-CH2-CH3) and the variable region (VL) of L chain (GC33-VL-CL) of GC33 (an anti-GPC3 antibody) were swapped with each other to produce H chain GC33-VL-CH1-hinge-CH2-CH3 and L chain (GC33-VH-CL) (the VH domain and VL domain were exchanged with each other). GC33-VL-CH1-hinge-CH2-CH3 is associated with GC33-VH-CL; however, its association with the L chain (MRA-VL-CL) of the anti-IL-6 receptor antibody is inhibited due to the instability of VL/VL interaction. Likewise, the H chain (MRA-VH-CH1-hinge-CH2-CH3) of the anti-IL-6 receptor antibody is associated with MRA-VL-CL; however, its association with the L chain (GC33-VH-CL) of the anti-GPC3 antibody is inhibited due to the instability of VH/VH interaction. As described above, it is possible to enhance the association between H chains and L chains against the same antigen. However, the VH/VH interaction and VL/VL interaction also occur although they are less stable than the VH/VL interaction (for VH/VH, see: FEBS Lett. 2003 Nov 20, 554(3):323-9; J Mol Biol. 2003 Oct 17, 333(2):355-65; for VL/VL, see: J Struct Biol. 2002 Jun, 138(3):171-86; Proc Natl Acad Sci USA. 1985 Jul, 82(14):4592-6), and thus although infrequently, unfavorable self association of H chains and L chains also occurs. Hence, although the percentage of the bispecific antibody of interest is increased by simply swapping the VH domain and VL domain with each other, the expressed products still contain about ten types of combinations.

In general, it is extremely difficult to purify the bispecific antibody of interest from the ten types. However, it is possible to improve the separation of the ten types of components in ion exchange chromatography by introducing a modification so that the ten types of components each have a different isoelectric point. In this context, MRA-VH, which is the H chain variable region of an anti-IL-6 receptor antibody, was modified to lower the isoelectric point, and this yielded H54-VH with a lower isoelectric point. In the same manner, MRA-VL, which is the L chain variable region of an anti-IL-6 receptor antibody, was modified to lower the isoelectric point, and this yielded L28-VL with a lower isoelectric point. Furthermore, GC33-VH, which is the H chain variable region of an anti-GPC3 antibody, was modified to increase the isoelectric point. This yielded Hu22-VH with an increased isoelectric point.

The combination of the H and L chains of interest was improved by swapping the VH and VL between the H chains and L chains of an anti-GPC3 antibody. However, although infrequently, the unfavorable H chain/L chain association occurs because it is impossible to completely suppress the H54-VH/Hu22-VH interaction and L28-VL/GC33-VL interaction. An ordinary antibody sequence has glutamine at position 39 in VH. In VH/VH interaction, glutamines are believed to form hydrogen bonds at the VH/VH interface. Then, lysine was substituted for the glutamine at position 39 (Kabat numbering) to impair the H54-VH/Hu22-VH interaction. The VH/VH interaction was thus expected to be significantly impaired due to the electrostatic repulsion between two lysines at the VH/VH interface. Next, H54-VH-Q39K and Hu22-VH-Q39K were constructed by substituting lysine for the glutamine at position 39 (Kabat numbering) in the sequences of H54-VH and Hu22-VH. Likewise, an ordinary antibody sequence has glutamine at position 38 in VL. In the VL/VL interaction, glutamines are expected to form hydrogen bonds at the VL/VL interface. Then, glutamic acid was substituted for the glutamine at position 38 (Kabat numbering) to impair the L28-VL/GC33-VL interaction. The VL/VL interaction was thus expected to be significantly impaired due to the electrostatic repulsion between two glutamic acids at the VL/VL interface. Next, L28-VL-Q38E and GC33-VL-Q38E were constructed by substituting glutamic acid for the glutamine at position 39 (Kabat numbering) in the sequences of L28-VL and GC33-VL.

To further improve the efficiency of expression/purification of the bispecific antibody of interest, a substitution mutation of Arg for His at position 435 (EU numbering) was introduced into one H chain using the same method described in Example 3. Furthermore, the above mutation was combined with the mutations (a substitution of Lys for Asp at position 356, EU numbering, is introduced into one H chain and a substitution of Glu for Lys at position 439, EU numbering, is introduced into the other H chain) described in WO 2006/106905 (PROCESS FOR PRODUCTION OF POLYPEPTIDE BY REGULATION OF ASSEMBLY) as a modification to enhance the heteromeric association of the two types of H chains. The combined mutations enable purification of the molecule resulting from heteromeric association of the two types of H chains by protein A chromatography alone.

Specifically, the antibody H chain variable regions used were:
MRA-VH (the H chain variable region of an anti-human interleukin-6 receptor antibody, SEQ ID NO: 36);
GC33-VH (the H chain variable region of an anti-GPC3 antibody, SEQ ID NO: 37);
H54-VH (the H chain variable region of an anti-human interleukin-6 receptor antibody, SEQ ID NO: 38) with an isoelectric point lower than that of MRA-VH;
Hu22-VH (the H chain variable region of an anti-GPC3 antibody, SEQ ID NO: 39) with an isoelectric point higher than that of GC33-VH;
H54-VH-Q39K (SEQ ID NO: 40) where Lys is substituted for Gln at position 39 (Kabat numbering) in the sequence of H54-VH; and
Hu22-VH-Q39K (SEQ ID NO: 41) where Lys is substituted for Gln at position 39 in the sequence of Hu22-VH.
The following antibody H chain constant regions were also used:
IgG1-LALA-N297A-CH (SEQ ID NO: 42) where Ala is substituted for Leu at positions 234 and 235 (EU numbering), and Ala is substituted for Asn at position 297 (EU numbering), and the C-terminal Gly and Lys is deleted in the sequence of the H chain constant region of IgG1;
IgG1-LALA-N297A-CHr (SEQ ID NO: 43) where the sequence of IgG1-LALA-N297A-CH has extra two residues of Ser at the N terminus;
IgG1-LALA-N297A-s3-CH (SEQ ID NO: 44) where Glu is substituted for Lys at position 439 (EU numbering) in the sequence of IgG1-LALA-N297A-CH; and
IgG1-LALA-N297A-G3s3-CHr (SEQ ID NO: 45) where Lys is substituted for Asp at position 356 (EU numbering) and Arg is substituted for His at position 435 (EU numbering) in the sequence of IgG1-LALA-N297A-CHr.
Meanwhile, the antibody L chain variable regions used were:
MRA-VL (the L chain variable region of an anti-human interleukin-6 receptor antibody, SEQ ID NO: 46);
GC33-VL (the L chain variable region of an anti-GPC3 antibody, SEQ ID NO: 47);
L28-VL (the L chain variable region of an anti-human interleukin-6 receptor antibody, SEQ ID NO: 48) with an isoelectric point lower than that of MRA-VL;
L28-VL-Q38E (SEQ ID NO: 49) where Glu is substituted for Gln at position 38 (Kabat numbering) in the sequence of L28-VL; and
GC33-VL-Q38E (SEQ ID NO: 50) where Glu is substituted for Gln at position 38 (Kabat numbering) in the sequence of GC33-VL.
The following antibody L chain constant regions were also used.
IgG1-CL (the L chain constant region of IgG1, SEQ ID NO: 51).
IgG1-CLr (SEQ ID NO: 52), which was constructed by substituting Arg and Thr for the C-terminal Ala and Ser, respectively, in the sequence of IgG1-CL.
Gene no1-Mh-H was constructed by linking IgG1-LALA-N297A-CH downstream of MRA-VH. Gene nol-Mh-L was constructed by linking IgG1-CL downstream of MRA-VL. Gene no1-Gh-H was constructed by linking IgG1-LALA-N297A-CH downstream of GC33-VH. Gene nol-Gh-L was constructed by linking IgG1-CL downstream of GC33-VL. Gene no2-Gh-H was constructed by linking IgG1-LALA-N297A-CHr downstream of GC33-VL. Gene no2-Gh-L was constructed by linking IgG1-CLr downstream of GC33-VH. Gene no3-Ml-H was constructed by linking IgG1-LALA-N297A-CH downstream of H54-VH. Gene no3-Ml-L was constructed by linking IgG1-CL downstream of L28-VL. Gene no3-Ghh-L was constructed by linking IgG1-CLr downstream of Hu22-VH.
Gene no5-Ml-H was constructed by linking IgG1-LALA-N297A-s3-CH downstream of H54-VH. Gene no5-Gh-H was constructed by linking IgG1-LALA-N297A-G3s3-CHr downstream of GC33-VL.

Gene no6-Ml-H was constructed by linking IgG1-LALA-N297A-s3-CH downstream of H54-VH-Q39K. Gene no6-Ml-L was constructed by linking IgG1-CL downstream of L28-VL-Q38E. Gene no6-Gh-H was constructed by linking IgG1-LALA-N297A-G3s3-CHr downstream of GC33-VL-Q38E. Gene no6-Ghh-L was constructed by linking IgG1-CLr downstream of Hu22-VH-Q39K.

Respective genes (no1-Mh-H, no1-Mh-L, no1-Gh-H, no1-Gh-L, no2-Gh-H, no2-Gh-L, no3-Ml-H, no3-Ml-L, no3-Ghh-L, no5-Ml-H, no5-Gh-H, no6-Ml-H, no6-Ml-L, no6-Gh-H, and no6-Ghh-L) were inserted into animal cell expression vectors.
The following combinations of expression vectors were introduced into FreeStyle293-F cells to transiently express each designed molecule.

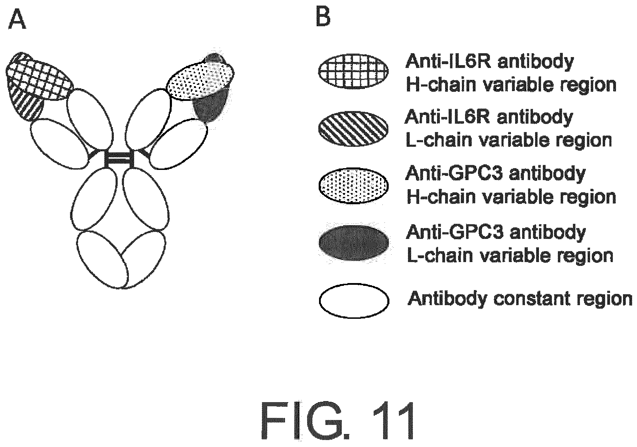

### A. Designed molecule: no1 (Fig. 11)

Description: natural anti-IL-6 receptor/anti-GPC3 bispecific antibody.
Polypeptides encoded by polynucleotides inserted into the expression vector: no1-Mh-H (SEQ ID NO: 53), nol-Mh-L (SEQ ID NO: 54), no1-Gh-H (SEQ ID NO: 55), and nol-Gh-L (SEQ ID NO: 56).

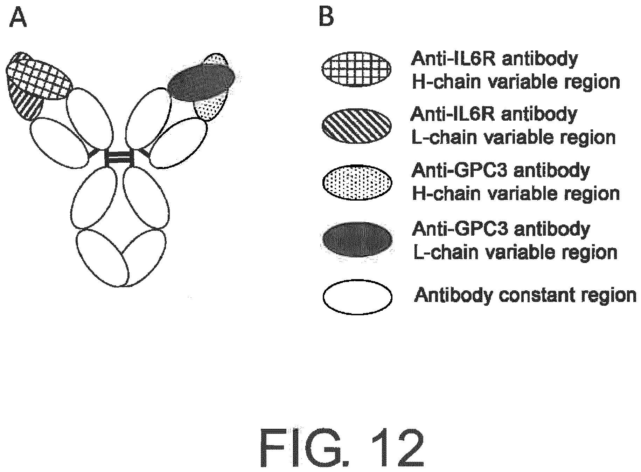

### B. Designed molecule: no2 (Fig. 12)

Description: constructed from no1 by swapping the VH and VL domains of the anti-GPC3 antibody.
Polypeptides encoded by polynucleotides inserted into the expression vector: no1-Mh-H, no1-Mh-L, no2-Gh-H (SEQ ID NO: 57), and no2-Gh-L (SEQ ID NO: 58).

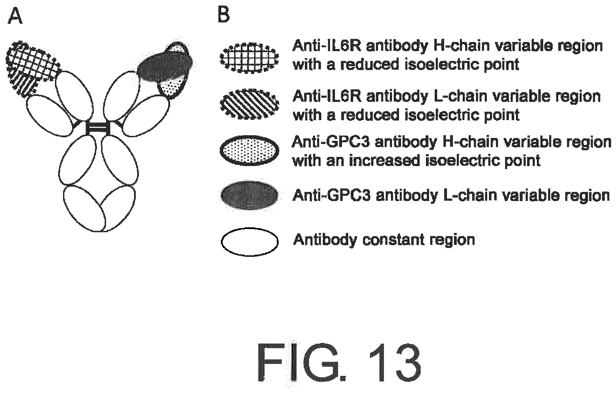

### C. Designed molecule: no3 (Fig. 13)

Description: constructed from no2 by introducing modifications to each chain to alter its isoelectric point.
Polypeptides encoded by polynucleotides inserted into the expression vector: no3-Ml-H (SEQ ID NO: 59), no3-Ml-L (SEQ ID NO: 60), and no2-Gh-H, and no3-Ghh-L (SEQ ID NO: 61).

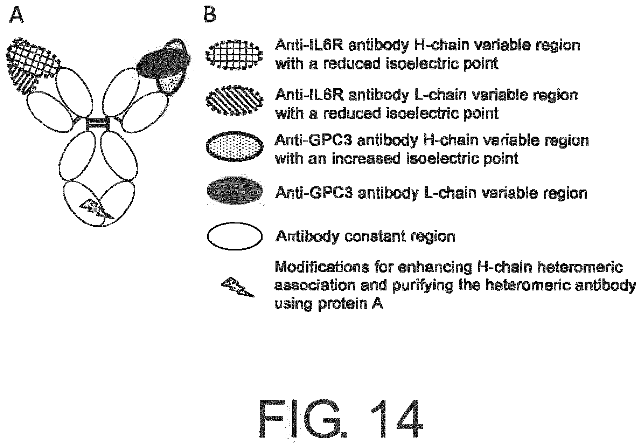

### D. Designed molecule: no5 (Fig. 14)

Description: constructed from no3 by introducing a modification to enhance heteromeric H chain association and a modification that enables protein A-based purification of antibody generated via heteromeric association.
Polypeptides encoded by polynucleotides inserted into the expression vector: no5-Ml-H (SEQ ID NO: 62), no3-Ml-L, no5-Gh-H (SEQ ID NO: 63), and no3-Ghh-L.

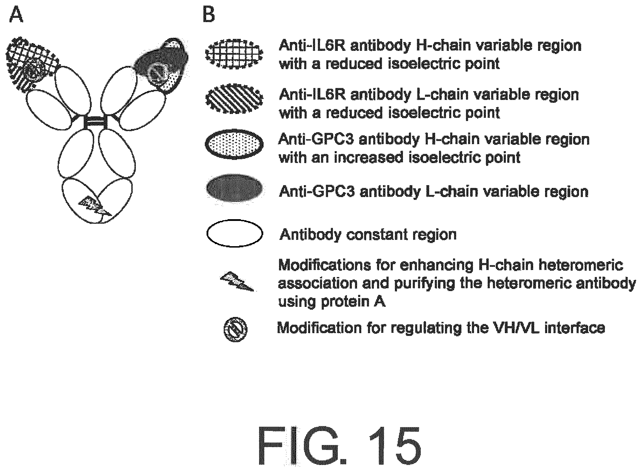

### E. Designed molecule: no6 (Fig. 15)

Description: constructed from no5 by introducing a modification to enhance the association between an H chain of interest and an L chain of interest.
Polypeptides encoded by polynucleotides inserted into the expression vector: no6-Ml-H (SEQ ID NO: 64), no6-Ml-L (SEQ ID NO: 65), no6-Gh-H (SEQ ID NO: 66), and no6-Ghh-L (SEQ ID NO: 67).

Culture supernatants filtered through a filter with a pore size of 0.22 µm were loaded onto rProtein A Sepharose Fast Flow resin (GE Healthcare) equilibrated with the medium. The resin was eluted in a batchwise manner to purify the molecules. Since protein G binds to the Fab domain of an antibody, all antibody species in CM can be purified with protein G regardless of the affinity for protein A.

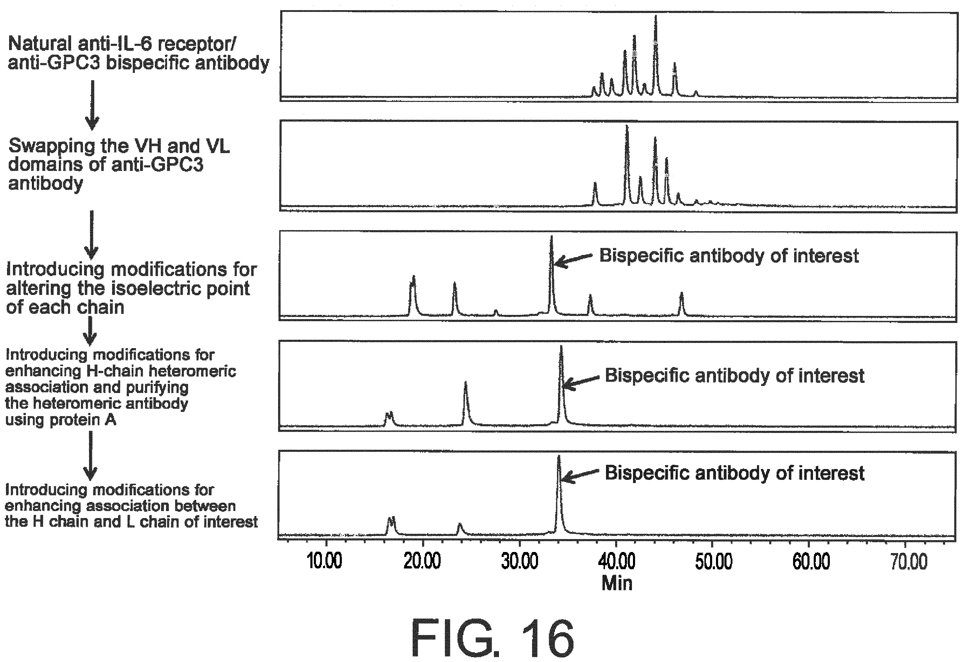

The designed antibodies (no1, no2, no3, no5, and no6) were assessed for their expression by cation exchange chromatography (IEC) using a ProPac WCX-10 column (Dionex), an analytical column. Cation exchange chromatography was performed at a flow rate of 0.5 ml/min with an adequate gradient using mobile phase A (20 mM MES-NaOH, pH 6.1) and mobile phase B (20 mM MES-NaOH, 250 mM NaCl, pH 6.1). The result of IEC assessment of each antibody is shown in Fig. 16. Natural anti-IL-6 receptor/anti-GPC3 bispecific antibody no1 gave a number of peaks in close proximity to each other. It was impossible to determine which peak corresponds to the bispecific antibody of interest. The same applied to no2 which results from swapping the VH domain and VL domain of the anti-GPC3 antibody in no1. The peak for the bispecific antibody of interest could be isolated for the first time in no3 which was modified from no2 by introducing a modification to alter the isoelectric point of each chain of no2. The proportion of the peak corresponding to the bispecific antibody of interest was significantly increased in no5 which was constructed from no3 by introducing a modification to enhance the H-chain heteromeric association and a modification that allows for protein A-based purification of the antibody generated via heteromeric association. The proportion of the peak corresponding to the bispecific antibody of interest was further increased in no6 which was constructed from no5 by introducing a modification that enhances the association between the H chain and L chain of interest.

Then, the present inventors assessed whether the bispecific antibody of interest could be purified from no6 CM to high purity using a purification column. CM samples were filtered through a filter with a pore size of 0.22 µm and loaded onto a HiTrap protein A HP column (GE Healthcare) equilibrated with D-PBS. The column was sequentially subjected to washes 1 and 2 and elution with a pH gradient using elution A and B as shown in Table 18. The pH gradient during elution was achieved with the following linear gradient: elution A/elution B = (100:0) -> (35:65) for 40 minutes.

**[Table 18]**

| **Equilibration** | D-PBS |
|---|---|
| **Wash** 1 | D-PBS |
| **Wash** 2 | 20 mM NaCitrate, pH5.0 |
| **Elution** A | 20 mM NaCitrate, pH5.0 |
| **Elution** B | 20 mM NaCitrate, pH2.7 |

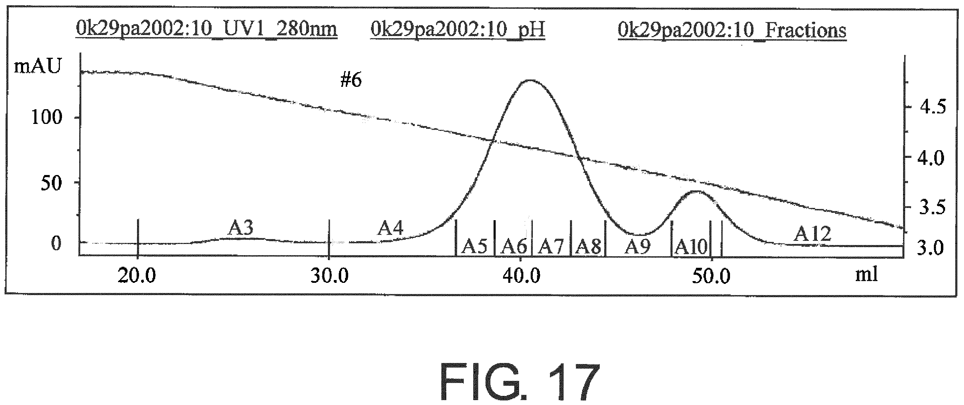

The result of pH gradient elution of No6 is shown in Fig. 17. The homomeric antibody having the H chain of the anti-GPC3 antibody which was incapable of binding to protein A passed through protein A; the first elution peak corresponded to the heteromeric antibody having the H chain of the anti-GPC3 antibody and the H chain of the anti-IL-6 receptor antibody; and the second elution peak corresponded to the homomeric antibody having the H chains of the anti-IL-6 receptor antibody. Thus, the present inventors demonstrated that by substituting Arg for His at position 435 (EU numbering), the heteromeric antibody having the H chain of the anti-GPC3 antibody and the H chain of the anti-IL-6 receptor antibody could be purified by the protein A-based purification step alone.

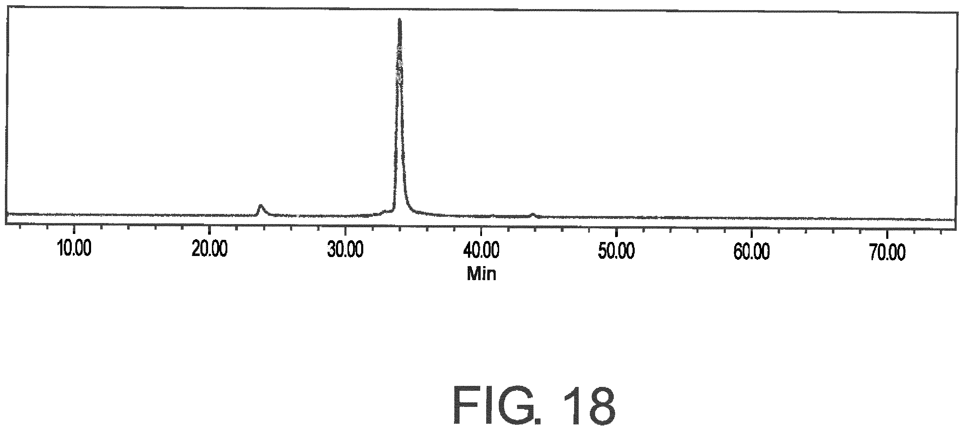

The first elution fraction was loaded onto a HiTrap SP Sepharose HP column (GE Healthcare) equilibrated with 20 mM sodium acetate buffer (pH 5.5). After washing with the same buffer, the column was eluted with a NaCl concentration gradient of 0 to 500 mM. The resulting main peak was analyzed by cation exchange chromatography in the same manner as described above. The result is shown in Fig. 18. The bispecific antibody of interest was demonstrated to be purified to a very high purity.

### Industrial Applicability

The present invention provides efficient methods based on alteration of the protein A-binding ability, for producing or purifying to a high purity polypeptide multimers (multispecific antibodies) having the activity of binding to two or more types of antigens through the protein A-based purification step alone. By using the methods of the present invention, polypeptide multimers of interest can be efficiently produced or purified to high purity without loss of other effects produced by amino acid mutations of interest. In particular, when the methods are combined with a method for controlling the association between two types of protein domains, polypeptide multimers of interest can be more efficiently produced or purified to a higher purity.

## Claims

1. A method for producing a polypeptide multimer that comprises a first polypeptide having an antigen-binding activity and a second polypeptide having an antigen-binding activity or no antigen-binding activity, which comprises the steps of:
(a) expressing a DNA that encodes the first polypeptide and a DNA that encodes the second polypeptide; and
(b) collecting the expression product of step (a) by protein A affinity chromatography, wherein the first polypeptide and the second polypeptide comprise an amino acid sequence of an antibody Fc domain or an amino acid sequence of an antibody heavy-chain constant region;
wherein the antibody Fc domain or antibody heavy-chain constant region is derived from human IgG;
wherein the amino acid residue at position 435, according to EU numbering, in the amino acid sequence of the Fc domain or heavy-chain constant region is histidine or arginine in the first polypeptide and arginine or histidine respectively in the second polypeptide; wherein the amino acid combination at positions 356 and 439 in the amino acid sequence of the Fc region or heavy chain constant region of the first polypeptide have been modified to amino acids with the same electric charge; and
wherein the amino acid combination at positions 356 and 439 in the amino acid sequence of the Fc region or heavy chain constant region of the second polypeptide have been modified to amino acids with an electric charge opposite to that of positions 356 and 439 of the first polypeptide.

2. The method of claim 1,
i) wherein the purity of the collected polypeptide multimer is 95% or more;
and/or
ii) wherein the first polypeptide having an antigen-binding activity and the second polypeptide having an antigen-binding activity comprise an amino acid sequence of an antibody heavy-chain variable region,
particularly wherein at least one amino acid residue has been modified in the amino acid sequences of FR1, CDR2, and FR3 of the antibody heavy-chain variable region;
and/or
iii) wherein the polypeptide multimer comprises one or two third polypeptides having an antigen-binding activity, and step (a) comprises expressing a DNA that encodes the third polypeptide having an antigen-binding activity,
particularly
a) wherein the third polypeptide having an antigen-binding activity comprises an amino acid sequence of an antibody light chain,
and/or
b) wherein the polypeptide multimer additionally comprises a fourth polypeptide having an antigen-binding activity, and step (a) comprises expressing a DNA that encodes the fourth polypeptide having an antigen-binding activity,
especially
b1) wherein at least one of the third and fourth polypeptides having an antigen-binding activity comprises an amino acid sequence of an antibody light chain,
or
b2) wherein the first polypeptide having an antigen-binding activity comprises amino acid sequences of an antibody light-chain variable region and an antibody heavy-chain constant region; the second polypeptide having an antigen-binding activity comprises an amino acid sequence of an antibody heavy chain; the third polypeptide having an antigen-binding activity comprises amino acid sequences of an antibody heavy-chain variable region and an antibody light-chain constant region; and the fourth polypeptide having an antigen-binding activity comprises an amino acid sequence of an antibody light chain;
and/or
iv) wherein the polypeptide multimer is a multispecific antibody,
particularly wherein the multispecific antibody is a bispecific antibody.

3. The method of any one of claims 1 and 2 i) to 2 iv), which comprises the first polypeptide having an antigen-binding activity and the second polypeptide having no antigen-binding activity, and wherein the first polypeptide having an antigen-binding activity comprises an amino acid sequence of an antigen-binding domain of a receptor and an amino acid sequence of an antibody Fc domain, and the second polypeptide having no antigen-binding activity comprises an amino acid sequence of an antibody Fc domain.

4. A method for purifying a polypeptide multimer that comprises a first polypeptide having an antigen-binding activity and a second polypeptide having an antigen-binding activity or no antigen-binding activity, which comprises the steps of:
(a) expressing a DNA that encodes the first polypeptide and a DNA that encodes the second polypeptide; and
(b) collecting the expression product of step (a) by protein A affinity chromatography, wherein the first polypeptide and the second polypeptide comprise an amino acid sequence of an antibody Fc domain or an amino acid sequence of an antibody heavy-chain constant region;
wherein the antibody Fc domain or antibody heavy-chain constant region is derived from human IgG;
wherein the amino acid residue at position 435, according to EU numbering, in the amino acid sequence of the Fc domain or heavy-chain constant region is histidine or arginine in the first polypeptide and arginine or histidine respectively in the second polypeptide; wherein the amino acid combination at positions 356 and 439 in the amino acid sequence of the Fc region or heavy chain constant region of the first polypeptide have been modified to amino acids with the same electric charge; and
wherein the amino acid combination at positions 356 and 439 in the amino acid sequence of the Fc region or heavy chain constant region of the second polypeptide have been modified to amino acids with an electric charge opposite to that of positions 356 and 439 of the first polypeptide.

5. The method of claim 4,
i) wherein the purity of the collected polypeptide multimer is 95% or more;
and/or
ii) wherein the first polypeptide having an antigen-binding activity and the second polypeptide having an antigen-binding activity comprise an amino acid sequence of an antibody heavy-chain variable region,
particularly wherein at least one amino acid residue has been modified in the amino acid sequences of FR1, CDR2, and FR3 of the antibody heavy-chain variable region; and/or
iii) wherein the polypeptide multimer comprises one or two third polypeptides having an antigen-binding activity, and step (a) comprises expressing a DNA that encodes the third polypeptide having an antigen-binding activity,
particularly
a) wherein the third polypeptide having an antigen-binding activity comprises an amino acid sequence of an antibody light chain,
and/or
b) wherein the polypeptide multimer additionally comprises a fourth polypeptide having an antigen-binding activity, and step (a) comprises expressing a DNA that encodes the fourth polypeptide having an antigen-binding activity, especially
b1) wherein at least one of the third and fourth polypeptides having an antigen-binding activity comprises an amino acid sequence of an antibody light chain,
or
b2) wherein the first polypeptide having an antigen-binding activity comprises amino acid sequences of an antibody light-chain variable region and an antibody heavy-chain constant region; the second polypeptide having an antigen-binding activity comprises an amino acid sequence of an antibody heavy chain; the third polypeptide having an antigen-binding activity comprises amino acid sequences of an antibody heavy-chain variable region and an antibody light-chain constant region; and the fourth polypeptide having an antigen-binding activity comprises an amino acid sequence of an antibody light chain;
and/or
iv) wherein the polypeptide multimer is a multispecific antibody,
particularly wherein the multispecific antibody is a bispecific antibody.

## Patentansprüche

1. Verfahren zum Herstellen eines Polypeptid-Multimers, das ein erstes Polypeptid umfasst, das eine Antigen-Bindungsaktivität aufweist, und ein zweites Polypeptid umfasst, das eine Antigen-Bindungsaktivität oder keine Antigen-Bindungsaktivität aufweist, wobei das Verfahren die folgenden Schritte umfasst:
(a) Exprimieren einer DNA, die das erste Polypeptid kodiert, und einer DNA, die das zweite Polypeptid kodiert; und
(b) Sammeln des Expressionsproduktes von Schritt (a) durch Protein-A-Affinitätschromatografie,
wobei das erste Polypeptid und das zweite Polypeptid eine Aminosäuresequenz von einer Fc-Domäne eines Antikörpers oder eine Aminosäuresequenz von einer konstanten Region der/ einer schweren Kette(n) eines Antikörpers umfassen;
wobei die Fc-Domäne eines Antikörpers oder die konstante Region der/ einer schweren Kette(n) eines Antikörpers von humanem IgG abgeleitet ist;
wobei der Aminosäurerest an Position 435 gemäß EU-Nummerierung in der Aminosäuresequenz von der Fc-Domäne oder konstanten Region der/ einer schweren Kette(n) in dem ersten Polypeptid Histidin oder Arginin ist und in dem zweiten Polypeptid Arginin beziehungsweise Histidin ist;
wobei die Aminosäure-Kombination an Positionen 356 und 439 in der Aminosäuresequenz der Fc-Region oder konstanten Region der/ einer schweren Kette(n) des ersten Polypeptids zu Aminosäuren mit der gleichen elektrischen Ladung modifiziert worden ist/ sind; und
wobei die Aminosäure-Kombination an Positionen 356 und 439 in der Aminosäuresequenz der Fc-Region oder konstanten Region der/ einer schweren Kette(n) des zweiten Polypeptids zu Aminosäuren mit einer elektrischen Ladung modifiziert ist/ sind, die entgegengesetzt ist zu der von Positionen 356 und 439 des ersten Polypeptids.

2. Verfahren nach Anspruch 1,
i) wobei die Reinheit des gesammelten Polypeptid-Multimers 95 % oder mehr ist;
und/oder
ii) wobei das erste Polypeptid, das eine Antigen-Bindungsaktivität aufweist, und das zweite Polypeptid, das eine Antigen-Bindungsaktivität aufweist, eine Aminosäuresequenz von einer variablen Region der/ einer schweren Kette(n) des Antikörpers umfassen,
insbesondere wobei mindestens ein Aminosäurerest in den Aminosäuresequenzen von FR1, CDR2 und FR3 der variablen Region der/ einer schweren Kette(n) eines Antikörpers modifiziert worden ist; und/oder
iii) wobei das Polypeptid-Multimer ein oder zwei dritte Polypeptide umfasst, die eine Antigen-Bindungsaktivität aufweisen, und Schritt (a) Exprimieren einer DNA umfasst, die das dritte Polypeptid, das eine Antigen-Bindungsaktivität aufweist, kodiert,
insbesondere
a) wobei das dritte Polypeptid, das eine Antigen-Bindungsaktivität aufweist, eine Aminosäuresequenz von einer leichten Kette eines Antikörpers umfasst,
und/oder
b) wobei das Polypeptid-Multimer zusätzlich ein viertes Polypeptid umfasst, das eine Antigen-Bindungsaktivität aufweist, und Schritt (a) Exprimieren einer DNA, die das vierte Polypeptid, das eine Antigen-Bindungsaktivität aufweist, kodiert, umfasst, insbesondere
b1) wobei mindestens eines von den dritten und vierten Polypeptiden, das/ die eine Antigen-Bindungsaktivität aufweist/ aufweisen, eine Aminosäuresequenz von einer leichten Kette eines Antikörpers umfasst,
oder
b2) wobei das erste Polypeptid, das eine Antigen-Bindungsaktivität aufweist, Aminosäuresequenzen von einer variablen Region der/ einer leichten Kette(n) eines Antikörpers und einer konstanten Region einer schweren Kette eines Antikörpers umfasst; das zweite Polypeptid, das eine Antigen-Bindungsaktivität aufweist, eine Aminosäuresequenz von einer schweren Kette eines Antikörpers umfasst; das dritte Polypeptid, das eine Antigen-Bindungsaktivität aufweist, Aminosäuresequenzen von einer variablen Region der/ einer schweren Kette(n) eines Antikörpers und einer konstanten Region der/ einer leichten Kette(n) eines Antikörpers umfasst; und das vierte Polypeptid, das eine Antigen-Bindungsaktivität aufweist, eine Aminosäuresequenz einer leichten Kette eines Antikörpers umfasst;
und/oder
iv) wobei das Polypeptid-Multimer ein multispezifischer Antikörper ist, insbesondere wobei der multispezifische Antikörper ein bispezifischer Antikörper ist.

3. Verfahren nach einem beliebigen der Ansprüche 1 und 2 i) bis 2 iv), welches das erste Polypeptid, das eine Antigen-Bindungsaktivität aufweist, und das zweite Polypeptid, das keine Antigen-Bindungsaktivität aufweist, umfasst, und wobei das erste Polypeptid, das eine Antigen-Bindungsaktivität aufweist, eine Aminosäuresequenz von einer Antigen-Bindungsdomäne eines Rezeptors und eine Aminosäuresequenz einer Fc-Domäne eines Antikörper umfasst, und das zweite Polypeptid, das keine Antigen-Bindungsaktivität aufweist, eine Aminosäuresequenz von einer Fc-Domäne eines Antikörpers umfasst.

4. Verfahren zum Reinigen eines Polypeptid-Multimers, das ein erstes Polypeptid umfasst, das eine Antigen-Bindungsaktivität aufweist, und ein zweites Polypeptid umfasst, das eine Antigen-Bindungsaktivität oder keine Antigen-Bindungsaktivität aufweist, wobei das Verfahren die folgenden Schritte umfasst:
(a) Exprimieren einer DNA, die das erste Polypeptid kodiert, und einer DNA, die das zweite Polypeptid kodiert; und
(b) Sammeln des Expressionsproduktes von Schritt (a) durch Protein-A-Affinitätschromatografie,
wobei das erste Polypeptid und das zweite Polypeptid eine Aminosäuresequenz von einer Fc-Domäne eines Antikörpers oder eine Aminosäuresequenz von einer konstanten Region der/ einer schweren Kette(n) eines Antikörpers umfassen;
wobei die Fc-Domäne eines Antikörpers oder die konstante Region der/ einer schweren Kette(n) eines Antikörpers von humanem IgG abgeleitet ist; wobei der Aminosäurerest an Position 435 gemäß EU-Nummerierung in der Aminosäuresequenz von der Fc-Domäne oder konstanten Region der/ einer schweren Kette(n) in dem ersten Polypeptid Histidin oder Arginin ist und in dem zweiten Polypeptid Arginin beziehungsweise Histidin ist;
wobei die Aminosäure-Kombination an Positionen 356 und 439 in der Aminosäuresequenz der Fc-Region oder konstanten Region der/ einer schweren Kette(n) des ersten Polypeptids zu Aminosäuren mit der gleichen elektrischen Ladung modifiziert worden ist/ sind; und
wobei die Aminosäure-Kombination an Positionen 356 und 439 in der Aminosäuresequenz der Fc-Region oder konstanten Region der/ einer schweren Kette(n) des zweiten Polypeptids zu Aminosäuren mit einer elektrischen Ladung modifiziert ist/ sind, die entgegengesetzt ist zu der von Positionen 356 und 439 des ersten Polypeptids.

5. Verfahren nach Anspruch 4,
i) wobei die Reinheit des gesammelten Polypeptid-Multimers 95 % oder mehr ist;
und/oder
ii) wobei das erste Polypeptid, das eine Antigen-Bindungsaktivität aufweist, und das zweite Polypeptid, das eine Antigen-Bindungsaktivität aufweist, eine Aminosäuresequenz von einer variablen Region der/ einer schweren Kette(n) des Antikörpers umfassen, insbesondere wobei mindestens ein Aminosäurerest in den Aminosäuresequenzen von FR1, CDR2 und FR3 der variablen Region der/ einer schweren Kette(n) eines Antikörpers modifiziert worden ist; und/oder
iii) wobei das Polypeptid-Multimer ein oder zwei dritte Polypeptide umfasst, die eine Antigen-Bindungsaktivität aufweisen, und Schritt (a) Exprimieren einer DNA umfasst, die das dritte Polypeptid, das eine Antigen-Bindungsaktivität aufweist, kodiert,
insbesondere
a) wobei das dritte Polypeptid, das eine Antigen-Bindungsaktivität aufweist, eine Aminosäuresequenz von einer leichten Kette eines Antikörpers umfasst,
und/oder
b) wobei das Polypeptid-Multimer zusätzlich ein viertes Polypeptid umfasst, das eine Antigen-Bindungsaktivität aufweist, und Schritt (a) Exprimieren einer DNA, die das vierte Polypeptid, das eine Antigen-Bindungsaktivität aufweist, kodiert, umfasst,
insbesondere
b1) wobei mindestens eines von den dritten und vierten Polypeptiden, das/ die eine Antigen-Bindungsaktivität aufweist/ aufweisen, eine Aminosäuresequenz von einer leichten Kette eines Antikörpers umfasst,
oder
b2) wobei das erste Polypeptid, das eine Antigen-Bindungsaktivität aufweist, Aminosäuresequenzen von einer variablen Region der/ einer leichten Kette(n) eines Antikörpers und einer konstanten Region einer schweren Kette eines Antikörpers umfasst; das zweite Polypeptid, das eine Antigen-Bindungsaktivität aufweist, eine Aminosäuresequenz von einer schweren Kette eines Antikörpers umfasst; das dritte Polypeptid, das eine Antigen-Bindungsaktivität aufweist, Aminosäuresequenzen von einer variablen Region der/ einer schweren Kette(n) eines Antikörpers und einer konstanten Region der/ einer leichten Kette(n) eines Antikörpers umfasst; und das vierte Polypeptid, das eine Antigen-Bindungsaktivität aufweist, eine Aminosäuresequenz einer leichten Kette eines Antikörpers umfasst;
und/oder
iv) wobei das Polypeptid-Multimer ein multispezifischer Antikörper ist,
insbesondere wobei der multispezifische Antikörper ein bispezifischer Antikörper ist.

## Revendications

1. Procédé pour produire un multimère polypeptidique qui comprend un premier polypeptide ayant une activité de liaison à un antigène et un deuxième polypeptide ayant une activité de liaison à un antigène ou n'ayant pas d'activité de liaison à un antigène, qui comprend les étapes consistant à :
(a) exprimer un ADN qui code pour le premier polypeptide et un ADN qui code pour le deuxième polypeptide ; et
(b) collecter le produit d'expression de l'étape (a) par chromatographie d'affinité sur protéine A,
dans lequel le premier polypeptide et le deuxième polypeptide comprennent une séquence d'acides aminés d'un domaine Fc d'anticorps ou une séquence d'acides aminés d'une région constante de chaîne lourde d'anticorps ;
dans lequel le domaine Fc d'anticorps ou la région constante de chaîne lourde d'anticorps dérive d'IgG humaine ;
dans lequel le résidu d'acide aminé à la position 435, conformément à la numérotation EU, dans la séquence d'acides aminés du domaine Fc ou de la région constante de chaîne lourde, est l'histidine ou l'arginine dans le premier polypeptide et l'arginine ou l'histidine respectivement dans le deuxième polypeptide ;
dans lequel la combinaison d'acides aminés aux positions 356 et 439 dans la séquence d'acides aminés de la région Fc ou de la région constante de chaîne lourde du premier polypeptide a été modifiée en des acides aminés ayant la même charge électrique ; et
dans lequel la combinaison d'acides aminés aux positions 356 et 439 dans la séquence d'acides aminés de la région Fc ou de la région constante de chaîne lourde du deuxième polypeptide a été modifiée en des acides aminés ayant une charge électrique opposée à celle des positions 356 et 439 du premier polypeptide.

2. Procédé selon la revendication 1,
i) dans lequel la pureté du multimère polypeptidique collecté est de 95 % ou plus ; et/ou
ii) dans lequel le premier polypeptide ayant une activité de liaison à un antigène et le deuxième polypeptide ayant une activité de liaison à un antigène comprennent une séquence d'acides aminés d'une région variable de chaîne lourde d'anticorps,
en particulier dans lequel au moins un résidu d'acide aminé a été modifié dans les séquences d'acides aminés de FR1, CDR2 et FR3 de la région variable de chaîne lourde d'anticorps ; et/ou
iii) dans lequel le multimère polypeptidique comprend un ou deux troisièmes polypeptides ayant une activité de liaison à un antigène, et l'étape (a) comprend l'expression d'un ADN qui code pour le troisième polypeptide ayant une activité de liaison à un antigène, en particulier
a) dans lequel le troisième polypeptide ayant une activité de liaison à un antigène comprend une séquence d'acides aminés d'une chaîne légère d'anticorps, et/ou
b) dans lequel le multimère polypeptidique comprend de plus un quatrième polypeptide ayant une activité de liaison à un antigène, et l'étape (a) comprend l'expression d'un ADN qui code pour le quatrième polypeptide ayant une activité de liaison à un antigène, en particulier
b1) dans lequel au moins l'un des troisième et quatrième polypeptides ayant une activité de liaison à un antigène comprend une séquence d'acides aminés d'une chaîne légère d'anticorps, ou
b2) dans lequel le premier polypeptide ayant une activité de liaison à un antigène comprend des séquences d'acides aminés d'une région variable de chaîne légère d'anticorps et d'une région constante de chaîne lourde d'anticorps ; le deuxième polypeptide ayant une activité de liaison à un antigène comprend une séquence d'acides aminés d'une chaîne lourde d'anticorps ; le troisième polypeptide ayant une activité de liaison à un antigène comprend des séquences d'acides aminés d'une région variable de chaîne lourde d'anticorps et d'une région constante de chaîne légère d'anticorps ; et le quatrième polypeptide ayant une activité de liaison à un antigène comprend une séquence d'acides aminés d'une chaîne légère d'anticorps ; et/ou
iv) dans lequel le multimère polypeptidique est un anticorps multispécifique,
en particulier dans lequel l'anticorps multispécifique est un anticorps bispécifique.

3. Procédé selon l'une quelconque des revendications 1 et 2 i) à 2 iv), qui comprend le premier polypeptide ayant une activité de liaison à un antigène et le deuxième polypeptide n'ayant pas d'activité de liaison à un antigène, dans lequel le premier polypeptide ayant une activité de liaison à un antigène comprend une séquence d'acides aminés d'un domaine de liaison à un antigène d'un récepteur et une séquence d'acides aminés d'un domaine Fc d'anticorps, et le deuxième polypeptide n'ayant pas d'activité de liaison à un antigène comprend une séquence d'acides aminés d'un domaine Fc d'anticorps.

4. Procédé pour purifier un multimère polypeptidique qui comprend un premier polypeptide ayant une activité de liaison à un antigène et un deuxième polypeptide ayant une activité de liaison à un antigène ou n'ayant pas d'activité de liaison à un antigène, qui comprend les étapes consistant à :
(a) exprimer un ADN qui code pour le premier polypeptide et un ADN qui code pour le deuxième polypeptide ; et
(b) collecter le produit d'expression de l'étape (a) par chromatographie d'affinité sur protéine A,
dans lequel le premier polypeptide et le deuxième polypeptide comprennent une séquence d'acides aminés d'un domaine Fc d'anticorps ou une séquence d'acides aminés d'une région constante de chaîne lourde d'anticorps ;
dans lequel le domaine Fc d'anticorps ou la région constante de chaîne lourde d'anticorps dérive d'IgG humaine ;
dans lequel le résidu d'acide aminé à la position 435, conformément à la numérotation EU, dans la séquence d'acides aminés du domaine Fc ou de la région constante de chaîne lourde, est l'histidine ou l'arginine dans le premier polypeptide et l'arginine ou l'histidine respectivement dans le deuxième polypeptide ;
dans lequel la combinaison d'acides aminés aux positions 356 et 439 dans la séquence d'acides aminés de la région Fc ou de la région constante de chaîne lourde du premier polypeptide a été modifiée en des acides aminés ayant la même charge électrique ; et
dans lequel la combinaison d'acides aminés aux positions 356 et 439 dans la séquence d'acides aminés de la région Fc ou de la région constante de chaîne lourde du deuxième polypeptide a été modifiée en des acides aminés ayant une charge électrique opposée à celle des positions 356 et 439 du premier polypeptide.

5. Procédé selon la revendication 4,
i) dans lequel la pureté du multimère polypeptidique collecté est de 95 % ou plus ; et/ou
ii) dans lequel le premier polypeptide ayant une activité de liaison à un antigène et le deuxième polypeptide ayant une activité de liaison à un antigène comprennent une séquence d'acides aminés d'une région variable de chaîne lourde d'anticorps,
en particulier dans lequel au moins un résidu d'acide aminé a été modifié dans les séquences d'acides aminés de FR1, CDR2 et FR3 de la région variable de chaîne lourde d'anticorps ; et/ou
iii) dans lequel le multimère polypeptidique comprend un ou deux troisièmes polypeptides ayant une activité de liaison à un antigène, et l'étape (a) comprend l'expression d'un ADN qui code pour le troisième polypeptide ayant une activité de liaison à un antigène, en particulier
a) dans lequel le troisième polypeptide ayant une activité de liaison à un antigène comprend une séquence d'acides aminés d'une chaîne légère d'anticorps, et/ou
b) dans lequel le multimère polypeptidique comprend de plus un quatrième polypeptide ayant une activité de liaison à un antigène, et l'étape (a) comprend l'expression d'un ADN qui code pour le quatrième polypeptide ayant une activité de liaison à un antigène, en particulier
b1) dans lequel au moins l'un des troisième et quatrième polypeptides ayant une activité de liaison à un antigène comprend une séquence d'acides aminés d'une chaîne légère d'anticorps, ou
b2) dans lequel le premier polypeptide ayant une activité de liaison à un antigène comprend des séquences d'acides aminés d'une région variable de chaîne légère d'anticorps et d'une région constante de chaîne lourde d'anticorps ; le deuxième polypeptide ayant une activité de liaison à un antigène comprend une séquence d'acides aminés d'une chaîne lourde d'anticorps ; le troisième polypeptide ayant une activité de liaison à un antigène comprend des séquences d'acides aminés d'une région variable de chaîne lourde d'anticorps et d'une région constante de chaîne légère d'anticorps ; et le quatrième polypeptide ayant une activité de liaison à un antigène comprend une séquence d'acides aminés d'une chaîne légère d'anticorps ; et/ou
iv) dans lequel le multimère polypeptidique est un anticorps multispécifique,
en particulier dans lequel l'anticorps multispécifique est un anticorps bispécifique.
